(19) Europäisches Patentamt
European Patent Office
Office européen des brevets

(11) Veröffentlichungsnummer: **0 255 020**
**B1**

(12) # EUROPÄISCHE PATENTSCHRIFT

(45) Veröffentlichungstag der Patentschrift: **19.09.90**

(51) Int. Cl.$^5$: **C 09 B 62/002,**
C 07 D 249/18, D 06 P 1/38

(21) Anmeldenummer: **87110515.1**

(22) Anmeldetag: **21.07.87**

(54) **Wasserlösliche Triphendioxazin-Verbindungen, Verfahren zu ihrer Herstellung und ihre Verwendung als Farbstoffe.**

(30) Priorität: **26.07.86 DE 3625346**

(43) Veröffentlichungstag der Anmeldung:
**03.02.88 Patentblatt 88/05**

(45) Bekanntmachung des Hinweises auf die Patenterteilung:
**19.09.90 Patentblatt 90/38**

(84) Benannte Vertragsstaaten:
**BE CH DE ES FR GB IT LI**

(56) Entgegenhaltungen:
**EP-A-0 222 098**
**FR-A-2 351 205**

(73) Patentinhaber: **HOECHST AKTIENGESELLSCHAFT**
**Postfach 80 03 20**
**D-6230 Frankfurt am Main 80 (DE)**

(72) Erfinder: **Schwaiger, Günther, Dr.**
**Johannesallee 41**
**D-6230 Frankfurt am Main 80 (DE)**
Erfinder: **Springer, Hartmut, Dr.**
**Am Erdbeerstein 27**
**D-6240 Königstein/Taunus (DE)**
Erfinder: **Helmling, Walter, Dr.**
**Fichtestrasse 29**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 255 020 B1

Courier Press, Leamington Spa, England.

**Beschreibung**

Die vorliegende Erfindung liegt auf dem Gebiet der faserreaktiven Farbstoffe.

Aus den europäischen Patentanmeldungs-Veröffentlichungen Nrs. 0 141 996 A und O 168 751 A sowie der US—PS 4 577 015 sind bereits faserreaktive Triphendioxazin-Farbstoffe bekannt, deren faserreaktive Gruppen jedoch nicht an einen heterocyclischen Rest gebunden sind.

Des weiteren werden in der nicht vorveröffentlichten (Art. 54(3) EPÜ) Patentanmeldungs-Veröffentlichung Nr. O 222 098 A2 faserreaktive Triphendioxazinfarbstoffe beschrieben, welche u.a. über einen Benzotriazol-Rest als Brückenglied zum Triphendioxazingerüst eine faserreaktive Gruppe der Vinylsulfonreihe gebunden enthalten.

Es wurden nunmehr neue wasserlösliche Triphendioxazin-Verbindungen entsprechend der allgemeinen Formel (1)

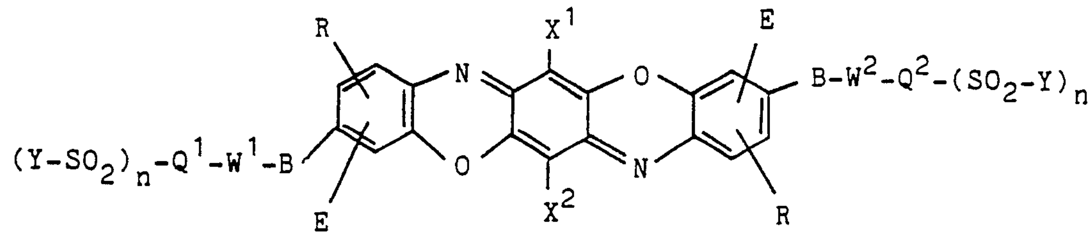

(1)

gefunden, die wertvolle faserreaktive Farbstoffeigenschaften besitzen.

In dieser Formel (1) bedeuten:

n ist die Zahl Null oder 1, bevorzugt 1, wobei im Falle n = 0 die Gruppe $—SO_2—Y$ ein Wasserstoffatom darstellt und n nur dann Null sein darf, wenn E eine Gruppe $—SO_2—Y$ mit Y gleich β-Sulfatoethyl ist;

Y ist die Vinylgruppe oder eine Ethylgruppe, die in β-Stellung einen durch ein Alkali eliminierbaren Substituenten enthält;

$Q^1$ ist ein Benzotriazolrest der nachstehend angegebenen allgemeinen Formel (2a) und

$Q^2$ ist ein Benzotriazolrest der nachstehend angegebenen allgemeinen Formel (2b)

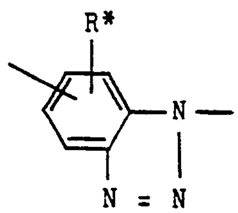

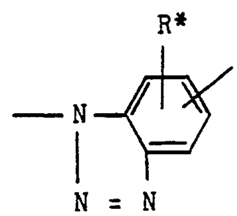

(2a) (2b)

in welchen

R* ein Wasserstoffatom, eine Alkylgruppe von 1 bis 4 C-Atomen, wie die Methyl- oder Ethylgruppe, eine Alkoxygruppe von 1 bis 4 C-Atomen, wie die Methoxy- oder Ethoxygruppe, ein Halogenatom, wie Chloratom, oder eine Carboxy- oder Sulfogruppe, bevorzugt jedoch ein Wasserstoffatom, bedeutet und die freie Bindung am Benzolrest die Bindung zur Gruppierung $(Y—SO_2)_n$-kennzeichnet,

B ist ein Sauerstoff- oder Schwefelatom oder eine Aminogruppe der Formel —NH—, bevorzugt die Aminogruppe —NH—, oder ist eine Gruppe der Formel —N(R')—, in welcher

R' eine Alkylgruppe von 1 bis 6 C-Atomen, bevorzugt von 1 bis 4 C-Atomen, wie insbesondere die Methyl- oder Ethylgruppe, ist, die substituiert sein kann;

$W^1$ ist ein bivalenter, gegebenenfalls substituierter aliphatischer, gegebenenfalls durch Alkyl substituierter $(C_5—C_{10})$-cycloaliphatischer, gegebenenfalls durch Alkyl substituierter aliphatisch-$(C_5—C_8)$-cycloaliphatischer, gegebenenfalls substituierter araliphatischer oder gegebenenfalls substituierter aromatisch-carbocyclischer Rest, wobei die aliphatischen Reste in $W^1$ durch Heterogruppen, bevorzugt 1 oder 2 Heterogruppen, unterbrochen sein können, die aus den Gruppen —O—, —S—, $—SO_2—$, —CO—, 1,4-Piperidino, —NH— und $—N(R°)—$, worin R° eine Alkylgruppe von 1 bis 6 C-Atomen, bevorzugt von 1 bis 4 C-Atomen, wie insbesondere die Methyl- oder Ethylgruppe, die substituiert sein kann, oder eine Alkanoylgruppe von 2 bis 5 C-Atomen, wie Acetylgruppe, ist, ausgewählt sind, und/oder wobei aliphatische und Arylreste durch eine solche Heterogruppe miteinander verbunden sein können;

$W^2$ hat eine für $W^1$ angegebene Bedeutung und ist mit $W^1$ gleich oder von $W^1$ verschieden;

R ist ein Wasserstoffatom oder ein Alkyl von 1 bis 6 C-Atomen, bevorzugt von 1 bis 4 C-Atomen, wie Methyl und Ethyl, ein Alkoxy von 1 bis 5 C-Atomen, bevorzugt von 1 bis 4 C-Atomen, wie Methoxy und

Ethoxy, ein Halogen, wie Fluor und Brom und insbesondere Chlor, Carboxy oder Sulfo, bevorzugt ein Wasserstoffatom;

E ist ein Wasserstoffatom oder eine Sulfo- oder Carboxygruppe oder eine Gruppe der allgemeinen Formel $-SO_2-Y$ mit Y der oben angegebenen Bedeutung oder eine gegebenenfalls substituierte Sulfonamidgruppe, insbesondere jedoch eine Sulfogruppe;

$X^1$ ist ein Wasserstoffatom oder ein Halogenatom, wie Fluor und insbesondere ein Chlor- oder Bromatom, eine Cycloalkylgruppe von 5 bis 8 C-Atomen, wie die Cyclohexylgruppe, eine Aralkyloxygruppe, eine Alkoxygruppe von 1 bis 4 C-Atomen, wie die Methoxygruppe, eine Aryloxygruppe, eine Alkylgruppe von 1 bis 4 C-Atomen, wie die Methylgruppe, eine Arylgruppe, eine Aralkylgruppe, eine Cyangruppe, eine Carboxygruppe, eine Carbalkoxygruppe von 2 bis 5 C-Atomen, wie die Carbomethoxy- oder Carbethoxygruppe, eine Arylaminogruppe, eine Carbamoylgruppe, eine N-Alkyl-carbamoyl-Gruppe oder N,N-Dialkylcarbamoyl-Gruppe mit Alkylresten von jeweils 1 bis 4 C-Atomen, eine N-Aroyl-carbamoyl-Gruppe, einer Alkanoylaminogruppe von 2 bis 5 C-Atomen, wie die Acetylaminogruppe, oder eine Aroyl aminogruppe, wie die Benzoylaminogruppe, wobei die Arylreste in diesen genannten Substituenten bevorzugt Phenylreste sind, die noch durch 1 oder 2 Substituenten aus der Gruppe Halogen, wie Chlor, Nitro, Alkyl von 1 bis 4 C-Atomen, Alkoxy von 1 bis 4 C-Atomen, Carboxy und Sulfo substituiert sein können und wobei $X^1$ bevorzugt ein Wasserstoffatom, eine Alkanyolaminogruppe von 2 bis 5 C-Atomen, eine Phenoxygruppe, die substituiert sein kann, eine Alkoxygruppe von 1 bis 4 C-Atomen, eine Alkylgruppe von 1 bis 4 C-Atomen und insbesondere bevorzugt ein Chloratom oder Bromatom ist;

$X^2$ ist mit $X^1$ gleich oder von $X^1$ verschieden und hat eine der für $X^1$ angegebenen Bedeutungen; die Gruppe E steht bevorzugt in ortho-Stellung zur Gruppe $-B-W^1-Q^1-(SO_2-Y)_n$ bzw. $-B-W^2-Q^2-(SO_2-Y)_n$ gebunden; von den Carboxy-, Sulfo- und Sulfatogruppen, die im Molekül (1) enthalten sein können, enthält das Molekül (1) zwingend mindestens eine, bevorzugt mindestens zwei davon.

Die einzelnen, auch zweifach erscheinenden Formelglieder, können zueinander gleiche oder voneinander verschiedene Bedeutungen besitzen.

Arylreste in den obengenannten oder nachstehend genannten Gruppen sind insbesondere die Phenyl- und Naphthylreste; sie können substituiert sein, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und ($C_5-C_8$)-Cycloalkyl-Reste sind.

Arylreste in den araliphatischen Resten sind insbesondere Phenylen- und Naphthylenreste; sie können substituiert sein, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und ($C_5-C_8$)-Cycloalkyl-Reste sind.

Aromatisch-carbocyclische Reste sind beispielsweise Phenylen- und Naphthylen- bzw. Phenyl- und Naphthylreste, die substituiert sein können, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und ($C_5-C_8$)-Cycloalkyl-Reste sind. Hiervon bevorzugt sind insbesondere solche Phenylen- oder Phenylreste, die durch Substituenten aus der Gruppe Alkyl von 1 bis 4 C-Atomen, Alkoxy von 1 bis 4 C-Atomen und Sulfo und/oder durch eine gegebenenfalls mono- oder disubstituierte Aminogruppe substituiert sein können, bspw. durch Alkyl von 1 bis 4 C-Atomen, Phenyl und Benzyl.

Aliphatische Reste sind beispielsweise Alkylgruppen oder Alkylengruppen von jeweils 1 bis 6 C-Atomen, bevorzugt 1 bis 4 C-Atomen, die substituiert sein können. Substituierte Alkyl- und Alkylengruppen sind beispielsweise solche, die durch 1 oder 2 Substituenten aus der Gruppe Chlor, Alkoxy von 1 bis 4 C-Atomen, Benzoylamino, Sulfobenzoylamino, Alkanoylamino von 2 bis 5 C-Atomen, Hydroxy, Sulfato, Phosphato, Phosphono, Acetyloxy, Sulfo, Carboxy oder gegebenenfalls substituiertes Aryl substituiert sein können. Bevorzugte Substituenten sind hiervon die Carboxy- und Sulfogruppen sowie Sulfatogruppen.

Die Formelglieder $W^1$ und $W^2$ sind beispielsweise Alkylengruppen von 1 bis 6 C-Atomen, insbesondere von 2 bis 4 C-Atomen, wobie die Alkylenkette durch 1 oder 2 Heterogruppen, die bevorzugt aus den Gruppen —O—, —NH— und —N(R')— mit R' der obigen Bedeutung ausgewählt sind, unterbrochen sein kann, oder ein Alkylen-phenylen-, ein Phenylen-alkylen-, ein Phenylen-alkylen-phenylen- oder Alkylen-phenylen-alkylen-Rest, wobei in diesen araliphatischen Resten die Alkylenreste solche von 1 bis 6, bevorzugt 1 bis 4 C-Atomen sind und gegebenenfalls durch die angegebenen Substituenten substituiert und/oder durch eine oder zwei der genannten Heterogruppen unterbrochen sein können und die Benzolkerne jeweils noch durch 1 oder 2 Substituenten substituiert sein können, die aus der Gruppe der Substituenten von Sulfo, Carboxy, Sulfamoyl, Carbamoyl, Methyl, Ethyl, Methoxy, Ethoxy, Nitro, Chlor, Amino und durch gegebenenfalls substituierte aliphatische und/oder gegebenenfalls substituierte Arylreste substituiertes Amino ausgewählt sind, wobei im Falle, daß eine Alkylengruppe durch Heterogruppen unterbrochen ist, deren Alkylenanteile darin bevorzugt solche von 2 oder 3 C-Atomen sind, und wobei die aliphatischen und Arylreste noch durch ein Sauerstoffatom oder eine Gruppe —NH— verbunden sein können. Die Formelglieder $W^1$ und $W^2$ sind weiterhin beispielsweise ein Phenylenrest, insbesondere ein meta- oder para-Phenylenrest, der noch durch 1 oder 2 Substituenten aus der Gruppe Sulfo, Carboxy, Sulfamoyl, Carbamoyl, Methyl, Ethyl, Methoxy, Ethoxy, Chlor, Amino und durch gegebenenfalls substituierte aliphatische und/oder gegebenenfalls substituierte Arylreste substituiertes Amino, bevorzugt jedoch durch Sulfo, substituiert sein kann, oder ein gegebenenfalls durch Sulfo substituierter Naphthylenrest. Bevorzugt ist $W^1$ bzw. $W^2$ ein Alkylenrest von 2 bis 4 C-Atomen, der durch 1 oder Substituenten, bevorzugt einen Substituenten, aus der Gruppe Sulfo, Sulfato, Carboxy, Phenyl und Sulfophenyl substituiert sein kann, wobei die Alkylenreste auch durch ein Sauerstoffatom oder eine Gruppe —NH— verbunden sein können, oder ein Alkylenphenylen-Rest mit solchen Alkylenresten.

Die faserreaktiven Gruppen der Formel —$SO_2$—Y sind an die aromatischen C-Atome der Benzolreste des Benzotriazols bzw. Phenoxazines gebunden; bevorzugt steht die Gruppe —$SO_2$—Y in den Benzo-1,2,3-triazol-Resten $Q^1$ bzw. $Q^2$ in deren 5-Stellung.

Reste $W^2$ sind beispielsweise der meta- oder para-Phenylenrest, der 1,2-Ethylen-, 1,3-Propylen-, 1,4-Butylen-, 1,5-Pentylen-, 1,6-Hexylen, 2-Methyl-1,3-propylen-, 2-Sulfophenyl-1,3-propylen- und 2-Sulfato-1,3-propylen-Rest, desweiteren beispielsweise ein sulfosubstituierter 1,4-Phenylenrest, ein 1,4- und 1,3-Cyclohexylenrest, ein bivalenter Rest der nachstehend angegebenen Formeln (a) bis (z), hiervon bevorzugt der 1,2-Ethylen-, 1,3-Propylen- und 1,4-Butylen-Rest, ein Rest der Formeln (a), ein 2-Sulfophenyl-1,3-propylen- und ein 2-Sulfato-1,3-propylen-Rest und ein sulfosubstituierter 1,4-Phenylenrest:

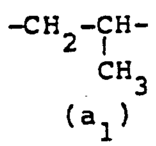
($a_1$)

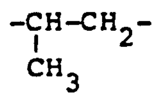
($a_2$)

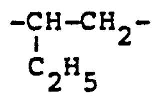
($b_1$)

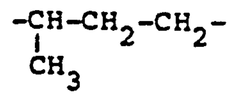
($b_2$)

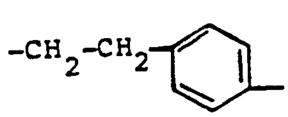
(c)

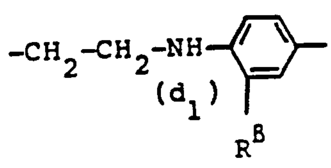
($d_1$)

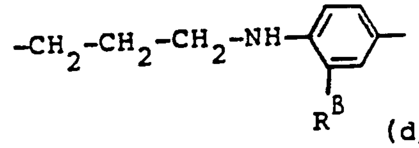
($d_2$)

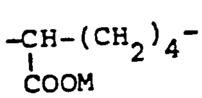
(e)

$-CH_2-CH_2-O-CH_2-CH_2-$

(f)

$-CH_2-CH_2-S-CH_2-CH_2-$

($g_1$)

$-CH_2-CH_2-SO_2-CH_2-CH_2-$

($g_2$)

$-(CH_2)_3-O-CH_2-CH_2-O-(CH_2)_3-$

(h)

$-CH_2-CH_2-NH-CH_2-CH_2-$

(j)

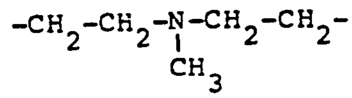
(k)

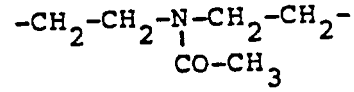
(m)

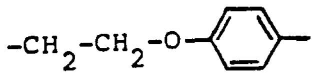

(n)

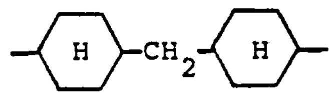

(p)

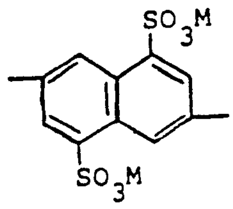

(q)

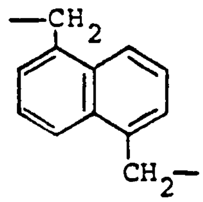

(r)

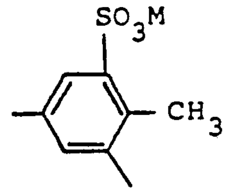

(s)

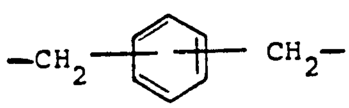

(t)

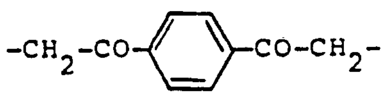

(u)

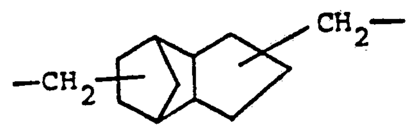

(v)

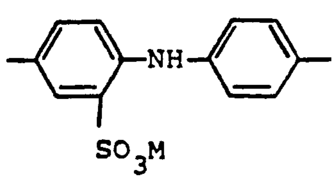

(w)

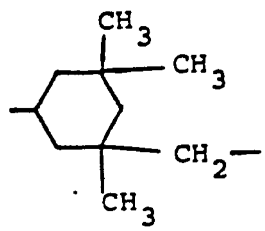

(x)

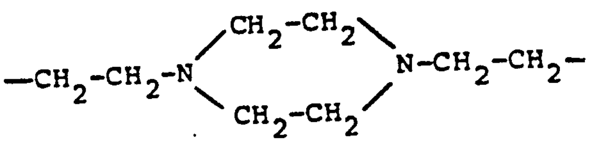

(y)

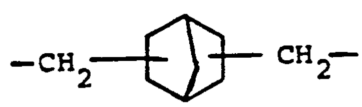

(z)

in welchen $R^{\beta}$ ein Wasserstoffatom oder eine Sulfogruppe ist und M ein Wasserstoffatom oder ein Alkalimetall, wie Natrium, Kalium und Lithium, oder das Äquivalent eines Erdalkalimetalls, wie beispielsweise des Calciums, insbesondere jedoch ein Alkalimetall ist.

Reste $W^1$ sind beispielsweise die eben für $W^2$ genannten, jedoch "spiegelbildlich" angeordneten Gruppen.

Sofern der Formelrest E eine substituierte Sulfonamidgruppe ist, ist deren N-Atom durch gegebenenfalls substituierte Alkylgruppen von 1 bis 6 C-Atomen (einschließlich gegebenenfalls substituiertes Aralkyl) und gegebenenfalls substituierte Arylreste mono- oder disubstituiert, wobei die Substituenten bevorzugt wasserlöslichmachende Gruppen, wie die Sulfo-, Carboxy-, Sulfato-, Phosphato oder Phosphono-Gruppe sind. Bevorzugt ist die gegebenenfalls substituierte Sulfonamidgruppe eine Gruppe der allgemeinen Formel $—SO_2—NR^1R^2$, in welcher $R^1$ ein Wasserstoffatom oder eine Alkylengruppe von 2 bis 6 C-Atomen, bevorzugt von 2 bis 4 C-Atomen, ist, die durch eine Sulfo- oder Sulfatogruppe substituiert ist, wobei die Alkylengruppe noch durch 1 oder 2 Heterogruppen, die aus den Gruppen der Formeln —O—, —S—, —NH— und —N(R')— mit R' der oben angegebenen Bedeutung ausgewählt sind, unterbrochen sein kann, oder ist ein Alkylenrest von 2 bis 6 C-Atomen, bevorzugt 2 bis 4 C-Atomen, der durch eine Carboxy- oder Phosphatogruppe substituiert ist, oder ist ein Alkylenrest von 1 bis 3 C-Atomen, der durch eine Phosphonogruppe substituiert ist, oder ist ein Naphthyl- oder Phenylrest, die beide durch Substituenten aus der Gruppe Methyl, Methoxy, Ethoxy, Chlor, Carboxy und Sulfo substituiert sein können, wobei die Substituenten bevorzugt 1 bis 3 Sulfogruppen sind und hiervon der Monosulfo- und Disulfophenol-Rest bevorzugt ist, und worin $R^2$ für ein Wasserstoffatom steht oder eine Alkylgruppe von 1 bis 6 C-Atomen, bevorzugt 2 bis 4 C-Atomen, bedeutet, die substituiert sein kann, bevorzugt durch 1 oder 2, bevorzugt 1, Substituenten aus der Gruppe Hydroxy, Sulfato, Carboxy, Sulfo und Methoxy. Substituierte Sulfonamidgruppen sind auch beispielsweise Gruppen einer allgemeinen Formel $—SO_2—NH—SO_2—R^3$, in welcher $R^3$ eine gegebenenfalls substituierte Alkylgruppe von 1 bis 6 C-Atomen, bevorzugt 1 bis 4 C-Atomen, insbesondere Methylgruppe, bedeutet oder einen gegebenenfalls substituierten Arylrest darstellt,

wobei der Arylrest insbesondere bevorzugt ein Phenylrest ist, der durch 1 oder 2 Sulfogruppen substituiert sein kann.

Bevorzugt ist die Gruppe E eine Sulfogruppe, eine Carboxygruppe, eine N,N-Di-(β-sulfatoethyl)-sulfamoyl-Gruppe, eine N-(β-Sulfatoethyl)-sulfamoyl-Gruppe, eine N-(β-Sulfoethyl)-sulfamoyl-Gruppe, eine N-Methyl-N-(β-Sulfoethyl)-sulfamoyl-Gruppe, oder eine β-Sulfatoethylsulfonyl-Gruppe, wobei die β-Sulfatoethylsulfonyl-Gruppe zwingend ist, falls in Formel (1) der Formelindex n für Null steht, insbesondere bevorzugt eine Sulfogruppe.

Substituenten, die gemäß dem Formelglied Y in β-Stellung der Ethylgruppe gebunden sind und durch eine Alkali unter Bildung der Vinylgruppe eliminiert werden können, sind beispielsweise Alkanoyloxygruppen von 2 bis 5 C-Atomen, wie die Acetyloxygruppe, Aroyloxygruppen, wie die Benzoyloxy-, Sulfobenzoyloxy- oder Carboxybenzoyloxy-Gruppe, Dialkylaminogruppen mit Alkylresten von 1 bis 4 C-Atomen, wie insbesondere die Dimethylamino- und Diethylaminogruppe, Trialkylammoniumgruppen mit Alkylresten von 1 bis 4 C-Atomen, wie die Trimethylammoniumgruppe, das Chloratom, das Bromatom, Alkylsulfonyloxygruppen mit Alkylresten von 1 bis 4 C-Atomen, eine Phosphatogruppe, eine Thiosulfatogruppe oder eine Sulfatogruppe. Bevorzugt sind von den Gruppen entsprechend dem Formelglied Y die β-Chlorethyl-, β-Phosphatoethyl-, β-Acetyloxyethyl- und β-Thiosulfatoethyl-Gruppe und insbesondere die Vinylgruppe und ganz besonders bevorzugt die β-Sulfatoethyl-Gruppe.

Die beiden Formelreste Y in der allgemeinen Formel (1) können zueinander gleiche oder voneinander verschiedene Bedeutungen haben; bevorzugt besitzen sie die gleiche Bedeutung. Ebenfalls können die Formelreste $(Y—SO_2)_n—Q^1—W^1—B—$ und $—B—W^2—Q^2—(SO_2—Y)_n$ zueinander gleiche oder voneinander verschiedene Bedeutungen besitzen; bevorzugt haben sie die gleiche Bedeutung. Ebenfalls können die Gruppen E zueinander gleiche oder voneinander verschiedene Bedeutungen haben; bevorzugt haben sie die gleiche Bedeutung.

Sulfogruppen sind Gruppen entsprechend der allgemeinen Formel $—SO_3M$, Carboxygruppen sind Gruppen entsprechend der allgemeinen Formel —COOM, Sulfatogruppen sind Gruppen entsprechend der allgemeinen Formel $—OSO_3M$, Phosponogruppen sind Gruppen der allgemeinen Formel $—PO_3M_2$, Thiosulfatogruppen sind Gruppen entsprechend der allgemeinen Formel $—S—SO_3M$ und Phosphatogruppen sind Gruppen entsprechend der allgemeinen Formel $—OPO_3M_2$, wobei M die obengenannte Bedeutung hat.

Besonders hervorzuheben sind von den erfindungsgemäßen Triphendioxazin-Verbindungen diejenigen, die der allgemeinen Formel (1a)

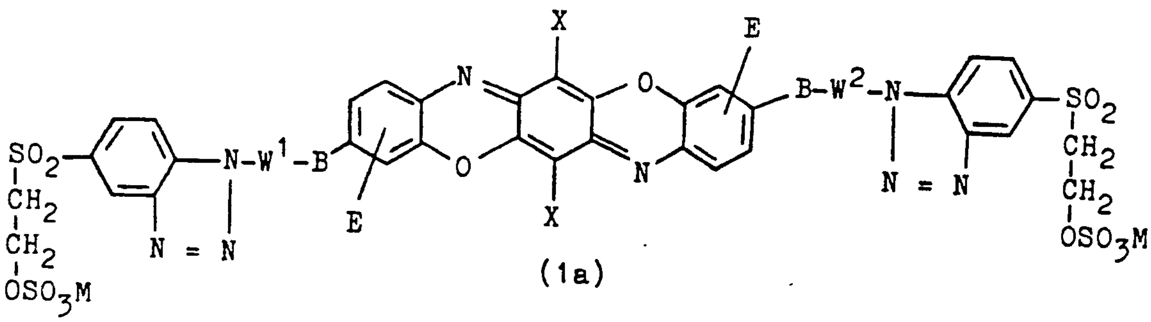

(1a)

entsprechen, in welcher

X für ein Bromatom oder bevorzugt ein Chloratom steht,

M ein Wasserstoffatom oder bevorzugt ein Alkalimetallatom, wie insbesondere Natrium, ist,

B für die Gruppe —NH— steht,

$W^2$ eine 1,2-Ethylen-, 1,3-Propylen-, 1,4-Butylen-, 1,6-Hexylen-, 1,4-Cyclohexylen- oder eine 1,4-Phenylen-Gruppe oder ein sulfosubstituierter 1,4-Phenylenrest oder ein Rest der oben angegebenen Formeln (a), (c), $(d_1)$, $(d_2)$ oder ein Rest der nachstehenden Formel $(z_1)$ oder $(z_2)$

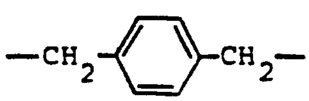

$(z_1)$

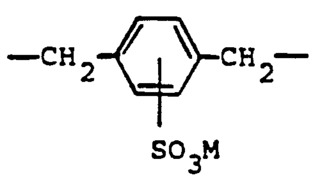

$(z_2)$

mit M der obengenannten Bedeutung ist, hiervon bevorzugt die 1,2-Ethylen-, 1,3-Propylen- und 1,4-Butylengruppe sowie die Gruppe der Formeln (a), und

$W^1$ einen solchen für $W^2$ erwähnten, jedoch "spiegelbildlich" angeordneten Rest bedeutet.

Bevorzugt sind in Formel (1a) beide Reste E und ebenso die Reste $W^1$ und $W^2$ jeweils zueinander identisch.

Weitere erfindungsgemäße Triphendioxazin-Verbindungen, die hervorgehoben werden können, sind solche entsprechend einer allgemeinen Formel (1b)

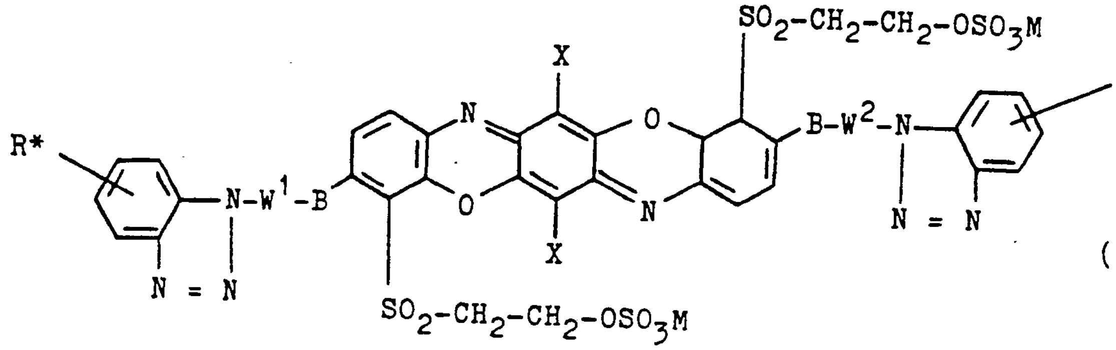

(1b)

in welcher

X für ein Bromatom oder bevorzugt ein Chloratom steht,

M ein Wasserstoffatom oder bevorzugt ein Alkalimetallatom, wie insbesondere Natrium, ist,

B für die Gruppe —NH— steht,

$W^2$ der 1,2-Ethylen- oder 1,3-Propylen- oder der 1,4-Cyclohexylen-Rest oder ein Rest der oben angegebenen Formeln (a), (c), ($d_1$), ($d_2$) oder ($z_2$), hiervon bevorzugt der 1,2-Ethylen- oder 1,3-Propylenrest oder ein Rest der Formeln (a), ist und

$W^1$ einen solchen für $W^2$ eben erwähnten, jedoch "spiegelbildlich" hierzu angeordneten Rest bedeutet sowie

R* jeweils eine Sulfogruppe ist, die bevorzugt in 5-Stellung des Benzo-1,2,3-triazols steht.

Interessante Verbindungen sind ebenfalls Verbindungen der allgemeinen Formel (1c)

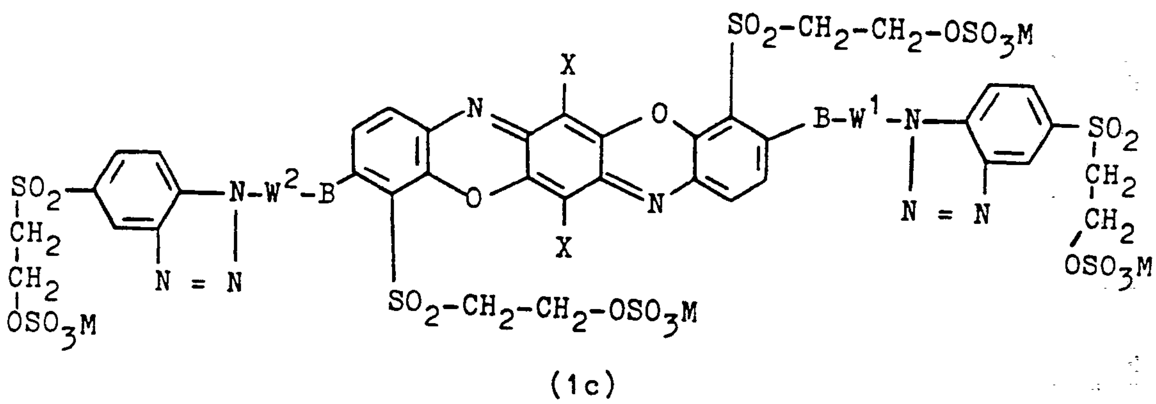

(1c)

in welcher

X für ein Bromatom oder bevorzugt ein Chloratom steht,

M ein Wasserstoffatom oder bevorzugt ein Alkalimetallatom, wie insbesondere Natrium, ist,

B für die Gruppe —NH— steht und

$W^1$ und $W^2$ beide eine der oben angegebenen sulfogruppenhaltigen oder sulfatogruppenhaltigen Reste bedeutet.

In den Formeln (1b) und (1c) haben die beiden R* bzw. die beiden Gruppen $W^1$ und $W^2$ bevorzugt eine gleiche Bedeutung.

Die neuen Dioxazinverbindungen können sowohl in saurer Form als auch in Form ihrer Salze vorliegen. Bevorzugt sind sie in Form der Salze, insbesondere der Alkali- und Erdalkalimetallsalze, und finden auch bevorzugt in Form dieser Salze Verwendung zum Färben (hier und im folgenden im allgemeinen Sinne und einschließlich des Bedruckens verstanden) von hydroxy- und/oder carbonamidgruppenhaltigen Materialien, insbesondere Fasermaterialien.

Die vorliegende Erfindung betrifft weiterhin Verfahren zur Herstellung der obengenannten und definierten Verbindungen der allgemeinen Formel (1). Diese sind dadurch gekennzeichnet, daß man eine Verbindung der allgemeinen Formel (3)

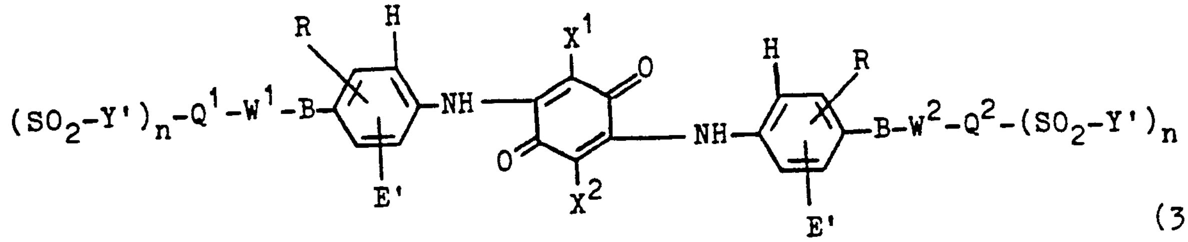

(3)

(in welcher Y′ die Vinylgruppe, die β-Hydroxyethyl-Gruppe oder eine Ethylgruppe, die in β-Stellung einen durch ein Alkali eliminierbaren Substituenten, wie einen der für Y genannten, enthält, bevorzugt die β-Hydroxyethyl-Gruppe ist, und n, R, B, $Q^1$, $Q^2$, $W^1$, $W^2$, $X^1$ und $X^2$ die obengenannten Bedeutungen haben und E′ eine der für E genannten Bedeutungen hat oder eine β-Hydroxyethylsulfonyl-Gruppe ist, und wobei substituierte Alkylgruppen in diesen Resten auch hydroxysubstituierte Alkylgruppen sein können und die Gruppen E′ bevorzugt in ortho-Stellung zur Gruppe $(Y'—SO_2)_n—Q^1—W^1—B$ bzw. $—B—W^2—Q^2—(SO_2—Y')_n$ gebunden sind) in saurem Medium und vorzugsweise in Gegenwart eines Oxidationsmittels zum Triphendioxazin cyclisiert. Die Umsetzung erfolgt in an und für sich bekannter Verfahrensweise, so beispielsweise in Schwefelsäure oder Schwefeltrioxid enthaltender Schwefelsäure als Reaktionsmedium, wobei als Oxidationsmittel Schwefeltrioxid, Ammonium- oder Alkalipersulfate, Jod oder anorgansiche Jodverbindungen in Gegenwart von Oleum, Natriumperborat, vorzugsweise jedoch Natrium- oder Kaliumperoxodisulfat (entsprechend den Formeln $Na_2S_2O_8$ bzw. $K_2S_2O_8$), verwendet werden. Solche Verfahrensweisen sind bspw. aus der britischen Patentschrift Nr. 1 589 915 und der europäischen Patentanmeldungs-Veröffentlichung Nr. O 141 359A bekannt.

Vorzugsweise führt man die Umsetzung in konzentrierter Schwefelsäure, wie 96- bis bevorzugt 100%iger Schwefelsäure und insbesondere in Schwefeltrioxid enthaltender Schwefelsäure (Oleum), wie bis zu 50 gew.-%igem Oleum, durch. Die Reaktionstemperatur wird zwischen 0 und 80°C gewählt. Das als Reaktionsmedium und Agenz verwendete Oleum besitzt in der Regel einen Gehalt von 5 bis 40 Gew.-%, bevorzugt 10 bis 20 Gew.-% an Schwefeltrioxid. Bei Zusatzvon Peroxodisulfat als Oxidationsmittel führt man die Cyclisierung zwischen 0 und 40°C, bevorzugt zwischen 15 und 25°C durch. Bei Verwendung von Oleum/Peroxodisulfat soll die Reaktionstemperatur 30°C nicht überschreiten. Bevorzugt ist 10 bis 20%iges Oleum unter Verwendung einer zur Verbindung (3) äquivalenten Menge Peroxodisulfat.

Bei Jod als Oxidationsmittel wird dieses in katalytischen Mengen in 10 bis 50%igem Oleum eingestzt; hier liegt die Reaktionstemperatur in der Regel zwischen 0 und 40°C.

Gegebenenfalls vor oder gleichzeitig mit der Cyclisierung oder erst nach der Cyclisierungsreaktion können gegebenenfalls vorhandene Hydroxyalkylgruppen, wie beispielsweise die β-Hydroxyethyl-Gruppe des Formelrestes Y′, mittels eines Sulfatisierungs- oder Phosphatierungsmittels, wie 96—100%iger Schwefelsäure oder Schwefeltrioxid enthaltender Schwefelsäure bzw. Polyphosphorsäure, in die entsprechenden β-Sulfatoalkyl- bzw. β-Phosphatoalkyl-Gruppen verestert werden. Wird also der Ringschluß in Schwefelsäure oder Oleum als Reaktionsmedium ausgeführt, werden Hydroxygruppen, die an einem Alkylrest des Moleküls gebunden sind, wie beispielsweise die oben bereits erwähnten β-Hydroxyethyl-Gruppen des Formelrestes Y′ oder Hydroxyalkyl-Gruppen der Formelreste $W^1$ bzw. $W^2$ und E′, in die entsprechenen Sulfatoalkylgruppen übergeführt.

Bei Temperaturen oberhalb von etwa 35°C, wie bei Temperaturen zwischen 40 und 60°C, lassen sich erfindungsgemäß auch Sulfogruppen in die aromatischen Ringe des Triphendioxazins (die entsprechenden Arylreste von $W^1$, $W^2$, E′, $X^1$ und $X^2$ eingeschlossen) einführen.

Verbindungen der Formel (1) mit Y gleich einer β-Sulfatoethyl-Gruppe können anschließend gemäß bekannten Verfahrensweisen in andere erfindungsgemäße Verbindungen der Formel (1) übergeführt werden, in welcher Y für die Vinylgruppe oder eine Ethylgruppe mit einem anderen in β-Stellung befindlichen alkalisch eliminierbaren Substituenten stehen.

Die Verbindungen der allgemeinen Formel (3) können analog bekannten Verfahrensweisen durch Umsetzung einer Verbindung der allgemeinen Formel (4)

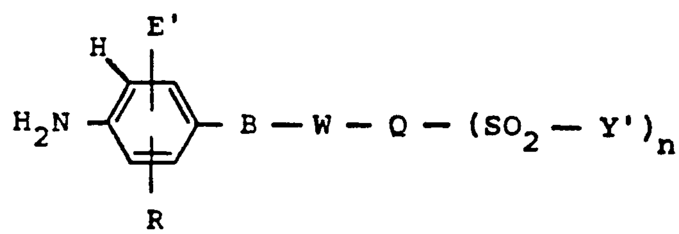

(4)

(worin Q die Bedeutung $Q^1$ bzw. $Q^2$ und W die Bedeutung von $W^1$ bzw. $W^2$ hat und Y′ die obengenannte Bedeutung besitzt und bevorzugt die β-Hydroxyethyl-Gruppe ist und n, R, B und E′ die obengenannten

Bedeutungen haben, wobei substituierte Alkylgruppen in diesen Resten auch hydroxysubstituierte Alkylgruppen sein können und die Gruppen E' bevorzugt in ortho-Stellung zur Gruppe B gebunden sind) mit einer 1,4-Benzochinon-Verbindung der allgemeinen Formel (5)

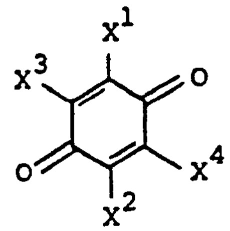

(5)

in welcher $X^1$ und $X^2$ die obengenannten Bedeutungen haben und $X^3$ und $X^4$ zueinander gleich oder voneinander verschieden sind und jedes für ein Wasserstoffatom, eine Alkoxygruppe von 1 bis 4 C-Atomen, wie insbesondere die Methoxygruppe, oder für eine Phenoxygruppe steht oder bevorzugt ein Halogenatom, wie Fluoratom, besonders Bromatom und insbesondere Chloratom, ist, wobei $X^3$ und $X^4$ auch eine zu $X^1$ und $X^2$ gleiche Bedeutung haben können, hergestellt werden.

Die Umsetzung einer Verbindung der allgemeinen Formel (4) oder zweier verschiedener Aminoverbindungen der allgemeinen Formel (4), jeweils in zusammen 2-fach äquivalenter Menge, mit einem Äquivalent einer Verbindung der allgemeinen Formel (5) zur Verbindung der allgemeinen Formel (3) erfolgt analog bekannten Verfahrensweisen, die beispielsweise in K. Venkataraman, "The Chemistry of Synthetic Dyes", Band V, S. 419—427 (1971), und in Ullmanns Encyklopädie der technischen Chemie, 4. Aufl., Band 8, S. 240 + 241 (1974) sowie in der britischen Patentanmeldungs-Veröffentlichung Nr. 2 019 872, in der deutschen Offenlegungsschrift Nr. 28 23 828 und in der europäischen Patentanmeldungs-Veröffentlichung Nr. 0 141 966A beschrieben sind. Beispielsweise kann die Umsetzung in wäßrigem Medium oder in wäßrigorganischem Medium oder in rein organischem Medium erfolgen, wobei die organischen Lösemittel polare aprotische und protische Lösungsmittel darstellen, wie beispielsweise niedere Alkanole, wie Methanol und Ethanol, und halogenierte Benzole, wie o-Dichlorbenzol. Bevorzugt wird das Chinon der Formel (5) jedoch in einem mehr oder weniger stärkeren Überschuß eingesetzt, der in der Regel 2—20% beträgt, aber auch, abhängig vom gewählten Chinon, bis zu 100% oder mehr betragen kann. Die Umsetzung der Amine (4) mit den Chinonen (5) kann bei einer Temperatur zwischen 20 und 100°C, vorzugsweise zwischen 50 und 70°C, in Gegenwart eines säurebindenden Mittels, wie beispielsweise eines Alkali- oder Erdalkalicarbonats oder -acetats, so beispielsweise Natriumacetat, Natriumcarbonat oder Natriumbicarbonat, oder eines Alkali- oder Erdalkalihydroxids, wie Natriumhydroxid, oder eines Oxids eines Erdalkalimetalls, wie beispielsweise Magnesiumoxid, durchgeführt werden. Sofern in einem wäßrigen oder wäßrig-organischen Medium gearbeitet wird, wird ein pH-Bereich zwischen 4 und 7, vorzugsweise zwischen 5,5 und 6,5, eingestellt.

Die Anilin-Ausgangsverbindungen der allgemeinen Formel (4) sind bisher noch nicht bekannt. Die Erfindung betrifft somit auch diese Verbindungen, Verfahren zu deren Herstellung und ihre Verwendung zur Synthese von Farbstoffen. Sie lassen sich analog bekannten Verfahrensweisen der Umsetzung von Nitrochlorbenzolen mit Aminen herstellen, so beispielsweise durch Umsetzung eines Chlornitrobenzols der allgemeinen Formel (6)

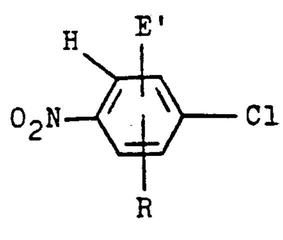

(6)

in welcher R und E' die obengenannten Bedeutungen besitzen, mit einem Amin der allgemeinen Formel (7)

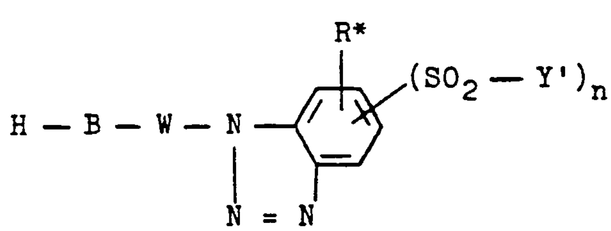

(7)

in welcher B, R*, W, Y' und n die obengenannten Bedeutungen haben, in Wasser oder einem organischen Medium, wie einem Alkanol, beispielsweise Methanol, Dioxan und Toluol, oder in einem Gemisch von Wasser und von wassermischbaren organischen Lösemitteln, unter Zusatz eines basischen, säurebindenden Mittels, wie beispielsweise Kaliumcarbonat, Magnesiumoxid, Natriumcarbonat, Natriumhydroxid, Triethylamin oder Triethanolamin, bei einer Temperatur zwischen 20 und 140°C, vorzugsweise zwischen 70 und 120°C, und bei Normaldruck oder bei Druck von bis zu 50 bar, vorzugsweise bis zu 10 bar. In wäßrigem Medium wird ein pH-Wert zwischen 6 und 12, bevorzugt zwischen 8 und 10, eingehalten.

Solche Verfahrensweisen sind beispielsweise aus den Verfahren zur Herstellung substituierter Phenyl-(β-hydroxyethyl)-sulfone bekannt (s. bspw. deutsche Offenlegungsschrift Nr. 3 502 991).

Die auf diese Weise erhältlichen und ebenfalls neuen und erfindungsgemäßen Nitro-anilin-Verbindungen der allgemeinen Formel (8)

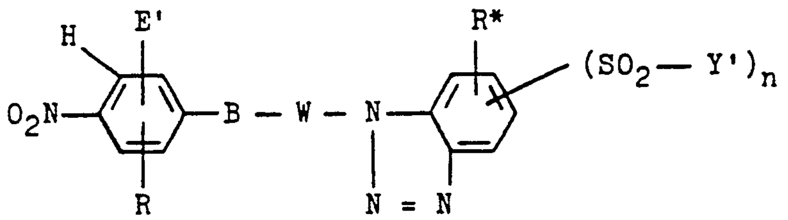

(8)

in welcher R, R*, E', B, W, Y' und n die obengenannten Bedeutungen haben, können sodann nach an und für sich üblichen Methoden der Reduktion von aromatischen Nitrogruppen mittels Wasserstoff und einem metallischen Katalysator, wie einem Palladium-, Platin- oder Raney-Nickelkatalysator, unter Druck im Autoklaven oder mittels der Reduktion nach Béchamp bei Verwendung von Eisenspänen zur Verbindung entsprechend der allgemeinen Formel (4), in welcher Y' bevorzugt für die β-Hydroxy-ethyl-Gruppe steht, reduziert werden. Aus den β-Hydroxy-ethylsulfonyl-Verbindungen der allgemeinen Formel (4) lassen sich die entsprechenden faserreaktiven Ausgangsverbindungen (4), in denen Y' für die Vinylgruppe oder eine Ethylgruppe, die in β-Stellung einen durch Alkali eliminierbaren Substituenten enthält, steht, in allgemein bekannter Verfahrensweise der Überführung der β-Hydroxyethylsulfonyl-Gruppe in solche Gruppen herstellen. Bevorzugt wird die β-Hydroxy-ethylsulfonyl-Gruppe in die β-Sulfatoethylsulfonyl-Gruppe übergeführt.

Chlornitrobenzolverbindungen der allgemeinen Formel (6), die als Ausgangsverbindungen dienen können, sind beispielsweise 2-Sulfo-4-nitro-chlorobenzol, 2-Carboxy-4-nitro-chlorbenzol, 2-(β-Hydroxyethylsulfonyl)-4-nitro-chlorbenzol, 2-[N,N-Di(β-hydroxyethyl)-sulfamoyl]-4-nitro-chlorbenzol, 2-[N-(β-Hydroxyethyl)-sulfamoyl]-4-nitro-chlorbenzol, 2-[N-(β-Sulfoethyl)-sulfamoyl]-4-nitro-chlorbenzol, 2-[N-Methyl-N-(β-sulfoethyl)-sulfamoyl]-4-nitro-chlorbenzol, 2-[N-β-(4'-B'-Hydroxyethylsulfonyl-2'-sulfophenyl)-ethyl-sulfamoyl]-4-nitro-chlorbenzol, 2-(N-Phenylethyl)-sulfamoyl-4-nitro-chlorbenzol, 2-Phenylsulfamoyl-4-nitro-chlorbenzol, 2-[N-(3'-β-Hydroxyethylsulfonyl-4'-β'-hydroxyethylamino-phenyl)-sulfamoyl]-4-nitro-chlorbenzol, 2-[N-(3'-β-Hydroxyethylsulfonyl-4-methoxy-phenyl)-sulfamoyl]-4-nitro-chlorbenzol, 2-[N-β-(β'-Hydroxyethylsulfonyl)-ethyl-sulfamoyl]-4-nitro-chlorbenzol, 2-[N-(Phenylsulfonyl)-sulfamoyl]-4-nitro-chlorbenzol und 2-[N-(Methylsulfonyl)-sulfamoyl]-4-nitro-chlorbenzol.

Ausgangsverbindungen der oben angegebenen und definierten allgemeinen Formel (7) mit n = 1 sind in der Literatur noch nicht bekannt; sie lassen sich jedoch analog den in der Literatur beschriebenen Verfahrensweisen herstellen, beispielsweise, indem man das aus dem Beispiel 5 der deutschen Patentschrift 859 462 bekannte 4-(β-Hydroxyethylsulfonyl)-2-nitro-chlorbenzol mit einem Amin der allgemeinen Formel A—B—W—$NH_2$, in welcher B und W die obengenannten Bedeutungen haben und A ein Wasserstoffatom oder einen Acylrest, wie einen Alkanoylrest von 2 bis 5 C-Atomen, beispielsweise den Acetylrest, oder den Benzoylrest, bedeutet, unter Abspaltung von Chlorwasserstoff umsetzt und sodann bei der so erhaltenen Verbindung die Nitrogruppe reduziert und in der hieraus erhaltenen Anilin-Verbindung der allgemeinen Formel

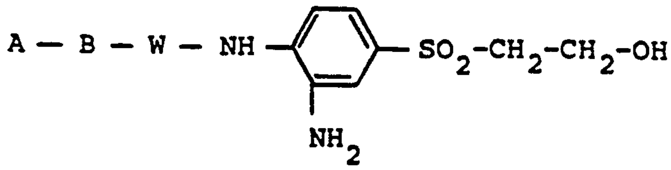

in welcher A, B und W die obengenannten Bedeutungen haben, deren Aminogruppe in an und für sich üblicher Weise diazotiert, wobei gleichzeitig Ringschluß zum Triazol erfolgt. Sofern die so gebildete Benzotriazolverbindung noch den Acylrest A enthält, kann dieser in üblicher Weise anschlißend hydrolytisch abgespalten werden.

So kann analog hierzu die Synthese der Ausgangsverbindungen der allgemeinen Formel (7) in der

Weise erfolgen, daß man eine Verbindung der allgemeinen Formel (9)

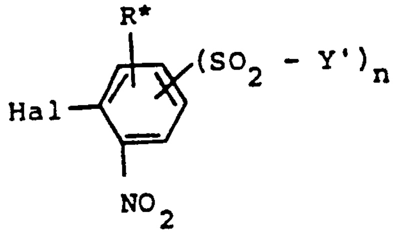

(9)

in welcher R*, Y' und n die obengenannten Bedeutungen haben und Hal für ein Fluor- oder Bromatom steht, mit einer Verbindung der allgemeinen Formel A—B—W—$NH_2$, in welcher A, B und W die obengenannten Bedeutungen haben und B bevorzugt die Gruppe NH ist, in einem für diese Reaktanten geeigneten Lösemittel in Gegenwart eines säurebindenden Mittels bei einer Temperatur zwischen 30 und 120°C, vorzugsweise zwischen 70 und 90°C, umsetzt.

Ausgangsverbindungen entsprechend der Formel A—B—W—$NH_2$ sind beispielsweise 1,2-Diamino-ethan, 1,3-Diamino-propan, 1,4-Diamino-butan, 1,5-Diamino-pentan, 1,6-Diamino-hexan, 1,2-Diamino-propan, 1,2-Diamino-butan, 1,3-Diamino-butan, die N-Acyl-, wie die N-Acetyl- und N-Benzoyl-Verbindungen, des 1-Amino-3-methylamino-propans, das 1,3-Diamino-2-methyl-propan, das 1,3-Diamino-2-hydroxy-propan, das 1,5-Diamino-2-carboxy-pentan, das 1,3-Diamino-2-phenyl-propan oder dessen im Benzolrest sulfosubstituiertes Derivat, des weiteren Verbindungen entsprechend einer allgemeinen Formel (a*), (b*), (c*) und (d*)

$$H_2N-(CH_2)_2-G-(CH_2)_2-NH_2 \quad (a^*)$$

$$H_2N-(CH_3)_2-G-(CH_3)_2-NH_2 \quad (b^*)$$

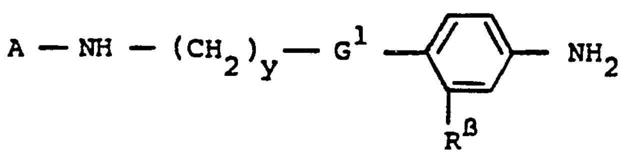

(c*)

$$H_2N-(CH_2-CH_2-NH)_r-CH_2-CH_2-NH_2 \quad (d^*)$$

in welchen

G ein Sauerstoffatom, ein Schwefelatom, eine Sulfonylgruppe oder eine Gruppe der Formel —NH—, —$N(CH_3)$— oder —$N(COCH_3)$— bedeutet,

$R^\beta$ für ein Wasserstoffatom oder eine Sulfogruppe steht,

A die obengenannte Bedeutung besitzt,

r die Zahl 2, 3 oder 4 ist,

$G^1$ für die Gruppe —NH— oder für ein Sauerstoffatom steht und

y die Zahl 2 oder 3 bedeutet,

des weiteren das, 1,3- oder 1,4-Cyclohexylen-diamin, das Bis-(4-amino-cyclohex-1-yl)-methan, das 1,8-Di-(aminomethyl)-naphthalin, das 1,4-Di-(aminomethyl)-benzol, das 1,3-Di-(aminomethyl)-benzol, das N,N'-Bis-(β-aminoethyl)-1,4-piperidin, das 1,4- oder 1,3-Phenylendiamin, das 4-Aminobenzylamin, das 4-Aminophenylethylamin sowie die entsprechenden N-Monoacyl-Derivate solcher Verbindungen, hiervon bevorzugt 1,2-Diamino-ethan, 1,3-Diamino-propan, 1,4-Diaminobutan und 1,2-Diaminopropan.

Geeignete Lösungsmittel für die Umsetzung der Halogennitrobenzole und der Aminoverbindungen sind beispielsweise Wasser, Alkanole von 1 bis 4 C-Atomen, Dioxan, Toluol, die Xylole, Chlorbenzol, o-Dichlorbenzol, m-Dichlorbenzol, Di-methylformamid und N-Methyl-pyrrolidon. Als Lösemittel kann auch das Amin selbst im Überschuß verwendet werden.

Die so synthetisierte Nitroverbindung der allgemeinen Formel (10)

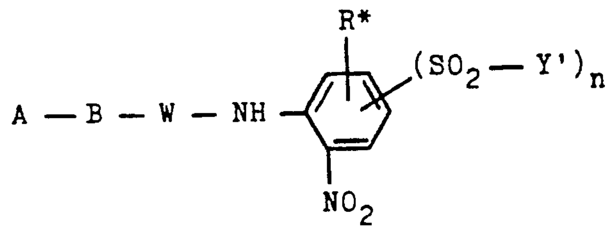

(10)

in welcher A, B, R*, W, Y' und n die obengenannten Bedeutungen haben, kann in an und für sich üblicher Weise, beispielsweise durch Kristallisation aus dieser Reaktionsmischung bzw. durch Abdestillieren des Lösemittels oder überschgüssigen Amins oder durch Ansuren und Filtration, isoliert werden.

Die anschließende Reduktion der Nitrogruppe in der Nitroverbindung (10) zur Aminogruppe kann in an und für sich bekannter Weise, so durch katalytische Hydrierung mit Wasserstoff an Palladium, Platin oder Raney-Nickel bie einer Temperatur zwischen 50 und 100°C und bei erhöhtem Druck oder durch Reduktion nach Béchamp mit Eisen in saurem Medium, beispielsweise mit Eisen in Ethanol/Eisessig, durchgeführt werden. Die Reduktion kann in einem hierfür geeigneten Lösemittel, wie Wasser, Methanol oder Ethanol oder einer Mischung derselben, erfolgen.

Die im Hydrieransatz enthaltene Aminoverbindung kann — nach vorheriger Abtrennung von Katalysatoren oder metallischen Reductionsmitteln — direkt, ohne Zwischenisolierung, unter gleichzeitigem Ringschluß zum Triazol diazotiert werden. Die Diazotierung erfolgt in an und für sich üblicher Verfahrensweise, so beispielsweise mit Natriumnitrit in salzsaurem Medium bei einer Temperatur zwischen −5°C und +15°C. Eine in der erhaltenen Benztriazolverbindung eventuell vorhandene Acylaminogruppe kann in üblicher Verfahrensweise, so beispielsweise in wäßrigem Medium bei einem pH-Wert von größer als 12 und einer Temperatur von 90 bis 100°C, zur Aminogruppe und somit zur Verbindung der allgemeinen Formel (7) hydrolysiert werden.

Ausgangsverbindungen der oben angegebenen und definierten allgemeinen Formel (7), die den faserreaktiven Rest der Vinylsulfon-Reihe enthalten bzw. deren β-Hydroxyethylsulfonyl-Derivat darstellen, sind beispielsweise β-[5-(β-Hydroxyethylsulfonyl)-benzotriazol-1-yl]-ethylamin, γ-[5-β-Hydroxyethylsulfonyl)-benzotriazol-1-yl]-n-propylamin, die ω-[5-(β-Hydroxyethylsulfonyl)-benzotriazol-1-yl]-$C_4$—$C_6$-n-alkylamine, β-[5-(β-Hydroxyethylsulfonyl)-benzotriazol-1'-yl]-ethanol, die ω-[5'-(β-Hydroxyethylsulfonyl)-benzotriazol-1'-yl]-$C_3$—$C_6$-n-alkanole, β-[5-(β-Hydroxyethylsulfonyl)-benzotriazol-1'-yl]-n-propylamin, 4-[5'-(β-Hydroxyethylsulfonyl)-benzotriazol-1'-yl]-cyclohexylamin, β-{4-[5-(β-Hydroxyethylsulfonyl)-benzotriazol-1'-yl]-phenethylamino}-ethylamin, β-{4-[5'-(β-Hydroxyethylsulfonyl)-benzotriazol-1'-yl]-phenylamino}-ethanol, 4-[5'-(β-Hydroxyethylsulfonyl)-benzotriazol-1'-yl]-phenethylamin und die anderen aus den Beispielen zahlreich ersichtlichen Benzotriazolverbindungen bzw. deren β-Hydroxyethylsulfonyl-Abkömmlinge.

Die als Ausgangsverbindungen dienenden Benzochinone der allgemeinen Formel (5) sind in der Literatur zahlreich bekannt. Verbindungen dieser Art sind beispielsweise 1,4-Benzochinon, 2-Methyl-1,4-benzochinon, 2-Ethyl-1,4-benzochinon, 2-n-Propyl-1,4-benzochinon, 2-Isopropyl-1,4-benzochinon, 2-(β-Ethoxyethyl)-1,4-benzochinon, 2-Phenyl-1,4-benzochinon, 2-(4'-Methylphenyl)-1,4-benzochinon, 2-(4'-Methoxyphenyl)-1,4-benzochinon, 2-(3'-Chlorophenyl)-1,4-benzochinon, 2-(4'-Nitrophenyl)-1,4-benzochinon, 2,5-Dimethyl-1,4-benzochinon, 2-Methyl-5-ethyl-1,4-benzochinon, 2-Methyl-3-chloro-1,4-benzochinon, 2-Methyl-6-chloro-1,4-benzochinon, 2-Methyl-3,5-dichloro-1,4-benzochinon, 2-Methyl-3,5,6-tribromo-1,4-benzochinon, 2-(4'-Methylphenoxy)-3,6-dibromo-1,4-benzochinon, 2-(3'-Methylphenoxy)-3,6-dibromo-1,4-benzochinon, 2-Methyl-3,5,6-trichloro-1,4-benzochinon, 2-Methyl-3-chloro-5-bromo-1,4-benzochinon, 2-Methyl-3,6-dichloro-1,4-benzochinon, 2-Methyl-3,6-dichloro-5-bromo-1,4-benzochinon, 2-Phenyl-3,6-dichloro-1,4-benzochinon, 2-(4'-Methoxyphenyl)-3,6-dichloro-1,4-benzochinon, 2-(4'-Chlorophenyl)-3,6-dichloro-1,4-benzochinon, 2-(4'-Nitrophenyl)-3,6-dichloro-1,4-benzochinon, 2-(4'-Nitrophenyl)-3,5,6-trichloro-1,4-benzochinon, 2,5-Dimethyl-3,6-dibromo-1,4-benzochinon, 2,5-Di-methyl-3-chloro-1,4-benzochinon, 2-Methyl-5-n-propyl-6-bromo-1,4-benzochinon, 2-Methyl-5-isopropyl-3-chloro-1,4-benzochinon, 2-Methyl-5-isopropyl-6-bromo-1,4-benzochinon, 2-(2'-Chlorophenyl)-3,5,7-tribromo-1,4-benzochinon, 2-Methyl-3-methoxy-1,4-benzochinon, 2,3,5,6-Tetramethoxy-1,4-benzochinon, 2,3,5,6-Tetraphenoxy-1,4-benzochinon, 2,3,5,6-Tetra-(4'-methylphenoxy)-1,4-benzochinon, 2,3,5,6-Tetra-(4'-methoxyphenoxy)-1,4-benzochinon, 2,3,5,6-Tetra-(4'-chlorophenoxy)-1,4-benzochinon, 2,3,5,6-Tetra-4-(3'-methyl-4'-chlorophenoxy)-1,4-benzochinon, 2-Ethyl-3,6-dimethoxy-1,4-benzochinon, 2-Chloro-3,6-dimethoxy-1,4-benzochinon, 2,3,5-Trimethoxy-1,4-benzochinon, 2,5-Dimethyl-3,6-dimethoxy-1,4-benzochinon, 2,5-Dimethyl-3,6-dimethoxy-1,4-benzochinon, 2-Methyl-3,6-dimethoxy-1,4-benzochinon, 2-Methyl-5,6-dimethoxy-1,4-benzochinon, 2-Ethyl-3,6-dimethoxy-1,4-benzochinon, 2-Chloro-3-n-propyl-5-methoxy-1,4-benzochinon und 2-Chloro-3,5-dimethoxy-1,4-benzochinon, 2,3,5,6-Tetrafluor-1,4-benzochinon sowie bevorzgt 2,3,5,6-Tetrabrom-1,4-benzochinon und insbesondere 2,3,5,6-Tetrachlor-1,4-benzochinon.

Die Abscheidung und Isolierung der erfindungsgemäß hergestellten Verbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Puffersubstanz zugefügt werden kann.

Die erfindungsgemäßen Verbindungen der allgemeinen Formel (1) haben faserreaktive Eigenschaften und besitzen wertvolle Farbstoffeigenschaften. Sie können deshalb zum Färben (einschließich Bedrucken) von natürlichen, regenerierten oder synthetischen hydroxygruppenhaltigen und/oder carbonamidgruppenhaltigen Materialien, beispielsweise in Form von Flächengebilden, wie Papier und Leder, oder in der Masse, von Polyamid oder Polyurethan, insbesondere aber von solchen Materialien in Faserform, wie Cellulosefasermaterialien, Seide, Wolle und synthetische Polyamid- und Polyurethanfasern, verwendet werden. Auch können die bei der Synthese der erfindungsgemäßen Verbindungen anfallenden Lösungen, gegebenenfalls nach Zusatz einer Puffersubstanz, gegebenenfalls auch nach Konzentrierung, direkt als Flüssigpräparation der färberischen Verwendung zugeführt werden.

Die erfindungsgemäßen Verbindungen der Formel (1) lassen sich, gemäß der erfindungsgemäßen Anwendung, auf den genannten Substraten, insbesondere auf den genannten Fasermaterialien, nach den für wasserlösliche, insbesondere faserreaktive, Farbstoffe bekannten Anwendungstechniken applizieren und fixieren, so beispielsweise, indem man die Dioxazinberbindung der allgemeinen Formel (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solcher Färbeund Fixierweisen sind in der Literatur zahlreich beschrieben.

Gegenstand der vorliegenden Erfindung ist deshalb auch die Verwendung der erfindungsgemäßen Verbindungen der allgemeinen Formel (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen der Applikation und Fixierung von faserreaktiven Farbstoffen vorgehen.

Die mit erfindungsgemäßen Verbindungen der allgemeinen Formel (1) hergestellten Färbungen und Drucke zeichnen sich durch reine, vorwiegend blaue Farbstöne aus. Insbesondere die Färbungen und Drucke auf Cellulosefasermaterialien besitzen sehr hohe Farbstärken und ebenso sehr gute Lichtechtheiten, einschließlich guter Naßlicht- und Schweißlichtechtheiten, ebenso gute Hypochloritbleich- und Chlorbadewasserechtheiten, weiterhin, vorzügliche Naßechtheiten, wie beispielsweise gute bis sehr gute Waschechtheiten bei 60 bis 95°C, auch in Gegenwart von Perboraten, saure und alkalische Walk-, Überfärbe- und Schweißechtheiten, Alkali-, Säure-, Wasser- und Seewasserechtheiten, desweiteren eine gute Plissierechtheit, Bügelechtheit und Reibechtheit. Ebenso besitzen sie eine gute Naßliegeechtheit und eine sehr gute Säurelagerbeständigkeit ("acid fading") beim Lagern von feuchten, noch Essigsäure enthaltendem, gefärbtem Material. Desweiteren sind die Färbungen gegen die üblichen Kunstharzappreturen stabil. Ein Teil der erfindungsgemäßen Verbindungen (Farbstoffe) sind in der Reinheit des Farbtones und in wichtigen Echtheitseigenschaften mit faserreaktiven Anthrachinonfarbstoffen vergleichbar.

Die nachstehenden Beispiele dienen zur Erläuterung der Erfindung. Die Teile sind Gewichtsteile, die Prozentangaben stellen Gewichtspozente dar, sofern nicht anders vermerkt. Gewichtsteile beziehen sich zu Volumenteilen wie Kilogramm zu Liter.

## Beispiel 1

a) 441 Teile der Verbindung der Formel

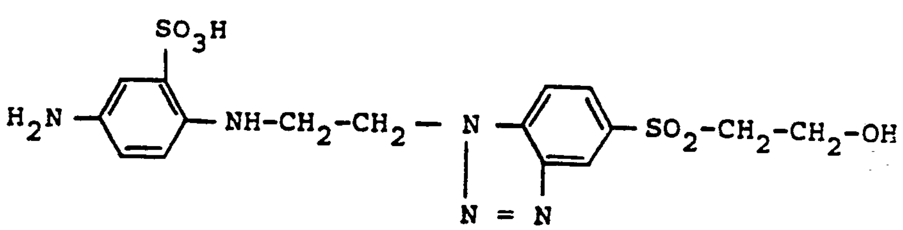

werden in 2000 Teilen Wasser bei pH 6 und 60°C gelöst. 124 teile Chloranil werden eingetragen, wobei mit etwa 90 Teilen Natriumbicarbonat ein pH-Wert von 6,5 und eine Reaktionstemperatur von etwa 65°C gehalten wird. Der Ansatz wird noch sechs Stunden nachgerührt, das Reaktionsprodukt sodann bei etwa 65°C geklärt, mit wenig Natriumchlorid ausgefällt, abgesaugt, mit 1000 Teilen einer 10%igen wäßrigen Natriumchloridlösung gewaschen und unter reduziertem Druck bei 70°C getrocknet.

b) 105 teile des unter a) erhaltenen Produktes werden bei einer Temperatur zwischen 20 und 25°C in 750 Teile 7%iges Oleum eingetragen. Der Reaktionsansatz wird anschließend noch etwa drei Stunden bei dieser Temperatur nachgerührt; sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, filtriert die ausgefallene erfindungsgemäße verbindung ab, löst sie wieder in etwa 1000 Teilen Wasser, stellt mit Natriumcarbonat einen pH-Wert von 5 ein und salzt sie, gegebenenfalls nach vorheriger üblicher Klärung der Lösung, mit Natriumchlorid aus.

Die erfindungsgemäße Triphendioxazin-Verbindung kann auch in Form ihres Natriumsalzes durch Eindampfen oder Sprühtrocknen de geklärten Syntheselösung erhalten werden. Sie besitzt, in Form der freien Säure geschrieben, die mutmaßliche Formel

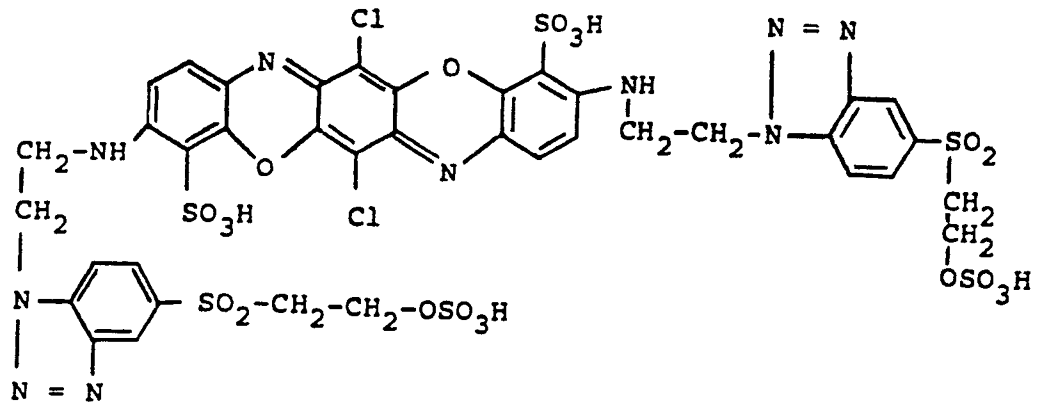

(die jeweilige Sulfogruppe kann auch in der anderen ortho-Stellung zur Benztriazolyl-ethylamino-Gruppe gebunden sein, befindet sich jedoch mit größerer Wahrscheinlichkeit in der in der obigen Formel angegebenen Stellung).

Diese erfindungsgemäße Verbindung besitzt gute faserreaktive Farbstoffeigenschaften. Sie färbt die in der Beschreibung genannten Materialien, insbesondere Cellulosefasermaterialien, wie Baumwolle, nach den in der Technick üblichen und bekannten Verfahrensweisen der Applikation und Fixierung von faserreaktiven Farbstoffen in farbstarken, reinen rotstichig blauen Tönen (entsprechend der Farbkennzahl 13 von Colour Index Hue Indication Chart) mit guten Echtheiten, wie insbesondere guter Lichtechtheit der trockenen oder feuchten, wie mit Trinkwasser befeuchteten Färbung, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit. In wäßriger Lösung zeigt sie im sichtbaren Bereich eine Absorptionsmaximum bei 612 nm.

c) Das unter a) eingesetzte 4-[β-(5′-β-Hydroxyethylsulfonylbenzotriazol-1′-yl)-ethylamino]-3-sulfo-anilin kann beispielsweise wie folgt hergestellt werden:

259 Teile des Natriumsalzes der 5-Nitro-2-chlor-benzol-sulfonsäure werden zu einer auf 40°C vorgeheizten Mischung von 270 Teilen 5-(β-Hydroxyethylsulfonyl)-1-(β-aminoethyl)-benzotriazol in 1000 Teilen Wasser und 500 Teilen Triethanolamin gegeben. Innerhalb von zwei Stunden wird die Mischung auf 100 bis 110°C aufgeheizt, wobei das Wasser teilweise abdestilliert. Man rührt weitere 10 Stunden bei 115 bis 120°C zur quantitativen Umsetzung nach und gibt sodann bei 100°C 3000 teile Wasser hinzu und klärt die Lösung bei 80 bis 90°C. Aus dem wäßrigen Medium kristallisiert beim Abkühlen das Natriumsalz des 4-[β-(5′-β-Hydroxyethylsulfonylbenzotriazol-1′-yl)-ethylamino]-3-sulfo-nitrobenzol in hoher Ausbeute und Reinheit aus.

Die Nitroverbindung zeigt folgende Daten in der $^1H$-NMR-Analyse (in $D_6$-DMSO mit TMS als innerem Standard): δ = 3,5 ppm (m, 2H); 3.64 ppm (t, 2H); 3,95 ppm (m, 2H); 4.82 ppm (t, OH); 5,0 ppm (t, 2H); 6.69 ppm (d, 1H); 7,57 ppm (t, NH); 7.95 ppm (m, 2H); 8.16 ppm (m, 1H); 8,3 ppm (d, 1H); 8.57 ppm (d, 1H).

d) Die Nitroverbindung von c) wird durch katalytische Hydrierung zur Anilinverbindung reduziert, indem man 236 Teile der Nitroverbindung in 1000 Teilen Wasser löst und in Gegenwart eines Pd/C-Katalysators in einem Austoklaven bei einer Temperatur bis 100°C und einem Wasserstoffdruck von 50 bar hydriert. Der Katalysator wird anschließend abfiltriert, das Filtrat abgekühlt und angesäuert. Aus ihm kristallisiert die Anilinverbindung in guter Ausbeute und hoher Reinheit aus. Die $^1H$-NMR-Analyse zeigt folgende Daten: δ = 3,4—3,9 ppm (m, 6H); 4,96 ppm (m, 2H, OH, NH); 6,68 ppm (d, 1H); 7,1 ppm (dd, 1H); 7.52 ppm (d, 1H); 7,95 ppm (dd, 1H); 8.15 ppm (d, 1H); 8.58 ppm (m, 1H); 9,7 ppm (breites $NH_3^+$).

Die Hydrierlösung kann auch direkt zur Umsetzung nach a verwendet werden.

e) Die unter c) eingesetzte Benzotriazolyl-ethylamino-Verbindung kann beispielsweise wie folgt hergestellt werden:

260 Teile β-[4-(β-Hydroxyethylsulfonyl)-2-amino-phenylamino]-ethylamin werden in etwa 1800 Teilen einer salzsauren wäßrigen Lösung bei 0 bis 5°C mittels einer wäßrigen Natriumnitritlösung in üblicher Weise diazotiert. Der Ringschluß erfolgt sofort und quantitativ. Die Lösung kann direkt in c) eingesetzt werden.

Eine isolierte Probe zeigt folgende $^1H$-NMR-Analyse (in $D_6$-DMSO mit TMS als inneren Standard): δ = 3,06 ppm (t, 2H); 3,54 ppm (m, 2H); 3,7 ppm (m, 2H); 4,72 ppm (t, 2H); 8,0 ppm (dd, 1H); 8,16 ppm (dd, 1H); 8,6 ppm (m, 1H); mobile Protonen (OH, $NH_2$).

f) Die unter e) eingesetzte 4-Hydroxyethylsulfonyl-2-amino-phenylaminoethylamin-Verbindung kann beispielsweise wie folgt erhalten werden:

530 Teile 4-(β-Hydroxyethylsulfonyl)-2-nitro-chlorbenzol werden bei 70 bis 80°C langsam in 620 Teile

Ethylendiamin eingetragen. Nach quantitativer Umsetzung wird das erhaltene Produkt auf Wasser ausgerührt und abgesaugt. Man erhält die Nitroverbindung in guter Ausbeute und hoher Reinheit mit einem Fp. von 146/147°C. Sie zeigt in der $^1$H-NMR-Analyse (in $D_6$-DMSO mit TMS als inneren Standard) folgende Daten: δ = 2,83 ppm (t, 2H); 3,44 ppm (m, 2H); 3,7 ppm (m, 2H); 7,27 ppm (d, 1H); 7,9 ppm (dd, 1H); 8.49 ppm (d, 1H); bewegliche Protonen bei 1,7 ppm ($NH_2$), 4,8 ppm (OH) und 8,8 ppm (NH).

g) Die Nitroverbindung von f) wird zur Anilinverbindung reduziert, indem man 290 Teile der Nitroverbindung in 1200 Teilen Wasser in Gegenwart eines Pd/C-Katalysators in einem Autoklaven bei einer Temperatur bis 100°C und einem Wasserstoffdruck von 50 bar hydriert. Der Katalysator wird anschließend abfiltriert, das Filtrat kann direkt in e) weiterverarbeitet werden.

Eine isolierte Probe (Fp. 169—172°C) zeigt folgende Daten in der $^1$H-NMR-Analyse in ($D_6$-DMSO mit TMS als inneren Standard): δ = 2,84 ppm (t, 2H); 3,2 ppm (m, 4H); 3,6 ppm (m, 2H); 6,53 ppm (dd, 1H); 6.96 ppm (d, 1H); 7,0 ppm (dd, 1H); mobile Protonen bei 4—5 ppm ($NH_2$, OH) uind 5,45 ppm (NH).

Beispiel 2

a) 455 Teile der Verbindung der Formel

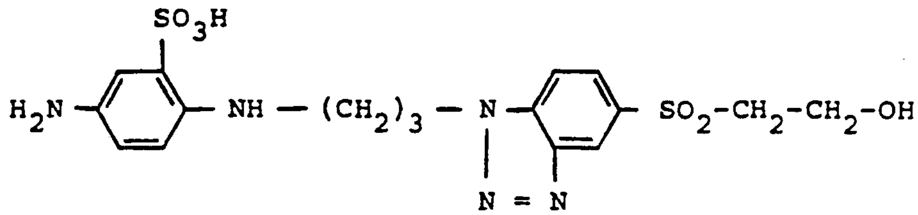

werden in 2000 Teilen Wasser bei pH 6 und 60°C gelöst. 124 Teile Chloranil werden eingetragen, wobei mit etwa 90 Teilen Natriumbicarbonat ein pH-Wert von 6,5 und eine Reaktionstemperatur von etwa 65°C gehalten wird. Der Ansatz wird noch sechs Stunden nachgerührt, das Reaktionsprodukt sodann bei etwa 25°C geklärt, mit wenig Natriumchlorid ausgefällt, abgesaugt, mit 1000 Teilen einer 10%igen wäßrigen Natriumchloridlösung gewaschen und unter reduziertem Druck bei 70°C getrocknet.

b) 108 Teile des unter a) erhaltenen Produktes werden bei einer Temperatur zwischen 20 und 25°C in 750 Teile 13%iges Oleum eingetragen. Der Reaktionsansatz wird anschließend noch etwa drei Stunden bei dieser Temperatur nachgerührt; sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, filtriert die ausgefallene erfindungsgemäße verbindung ab, löst sie wieder in etwa 1000 Teilen Wasser, stellt mit Natriumcarbonat einen pH-Wert von 5 ein und salzt sie, gegebenenfalls nach vorheriger üblicher Klärung der Lösung, mit Natriumchlorid aus.

Die erfindungsgemäße Triphendioxazin-Verbindung kann auch in Form ihres Natriumsalzes durch Eindampfen oder Sprühtrocknen de geklärten Syntheselösung erhalten werden. Sie besitzt, in Form der freien Säure geschrieben, die mutmaßliche Formel

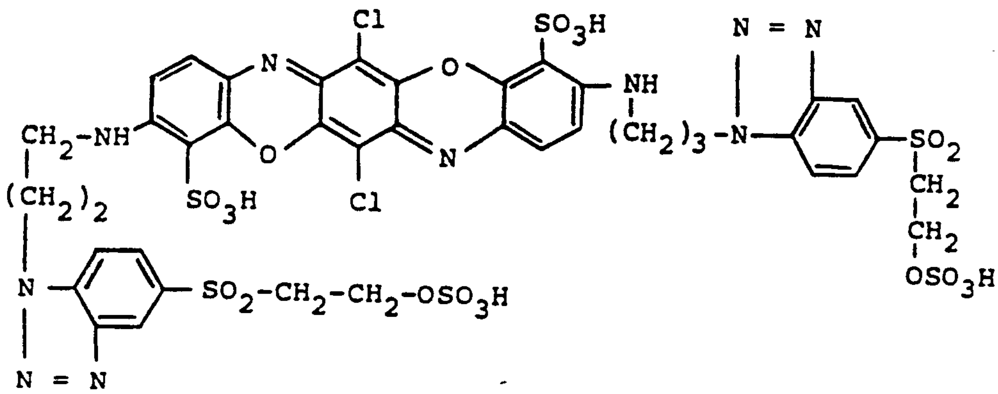

(die jeweilige Sulfogruppe kann auch in der anderen ortho-Stellung zur Benztriazolyl-ethylamino-Gruppe gebunden sein, befindet sich jedoch mit größerer Wahrscheinlichkeit in der in der obigen Formel angegebenen Stellung).

Diese erfindungsgemäße Verbindung besitzt gute faserreaktive Farbstoffeigenschaften. Sie färbt die in der Beschreibung genannten Materialien, insbesondere Cellulosefasermaterialien, wie Baumwolle, nach den in der Technick üblichen und bekannten Verfahrensweisen der Applikation und Fixierung von faserreaktiven Farbstoffen in farbstarken, reinen rotstichig blauen Tönen (entsprechend der Farbkennzahl 13 von Colour Index Hue Indication Chart) mit guten Echtheiten, wie insbesondere guter Lichtechtheit der

trockenen oder feuchten, wie mit Trinkwasser befeuchteten Färbung, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit. In wäßriger Lösung zeigt sie im sichtbaren Bereich ein Absorptionsmaximum bei 618 nm.

c) Das unter a) eingesetzte 4-[γ-(5'-β-Hydroxyethylsulfonylbenzotriazol-1'-yl)-propylamino]-3-sulfo-anilin kann beispielsweise wie folgt hergestellt werden:

259 Teile des Natriumsalzes der 5-Nitro-2-chlor-benzolsulfonsäure werden zu einer auf 40°C vorgeheizten Mischung von 285 Teilen 5-(β-Hydroxyethylsulfonyl)-1-(γ-aminopropyl)-benzotriazol in 1000 Teilen Wasser und 500 Teilen Triethanolamin gegeben. Innerhalb von zwei Stunden wird die Mischung auf 100 bis 110°C aufgeheizt, wobei das Wasser teilweise abdestilliert. Man rührt weitere 10 Stunden bei 115 bis 120°C zur quantitativen Umsetzung nach und gibt sodann bei 100°C 3000 teile Wasser hinzu und klärt die Lösung bei 80 bis 90°C. Aus dem wäßrigen Medium kristallisiert beim Abkühlen das Natriumsalz des 4-[γ-(5'-β-Hydroxyethylsulfonylbenzotriazol-1'-yl)-propylamino]-3-sulfo-nitrobenzol in hoher Ausbeute und Reinheit aus.

Die Nitroverbindung zeigt folgende Daten in der $^1$H-NMR-Analyse (in $D_6$-DMSO mit TMS als innerem Standard): δ = 2,25 ppm (m, 2H); 3.32 ppm (m, 2H); 3,54 ppm (m, 2H); 3,66 ppm (m, 2H); 4,9 ppm (m, OH, 2H); 6,69 ppm (d, 1H); 7,47 ppm (t, NH); 8,0 ppm (m, 2H); 8,20 ppm (m, 1H); 8,37 ppm (d, 1H); 8,62 ppm (d, 1H).

d) Die Nitroverbindung von c) kann analog Beispiel 1d) katalytisch hydriert werden. Sie zeigt folgende $^1$H-NMR-Daten: δ = 2,2 ppm (m, 2H); 3,14 ppm (t, 2H); 3,56 ppm (t, 2H); 3,7 ppm (t, 2H); 4,9 ppm (t, 2H); 6,6 ppm (d, 1H); 7,05 ppm (dd, 1H); 7,54 ppm (d, 1H); 7,95 (d, 1H); 8,17 ppm (d, 1H); 8.59 ppm (d, 1H); 9,5 ppm (breites $NH_3^+$); mobile Protonen (OH, NH), bei ca. 5 ppm.

Die in c) für die Verbindung von c) eingesetzten Ausgangsverbindungen können analog den Beispielen 1e) bis 1g) hergestellt werden.

Beispiele 3 bis 81

In den nachfolgenden Tabellenbeispielen sind weitere erfindungsgemäße Triphendioxazin-verbindungen entsprechend der allgemeinen Formel (1A)

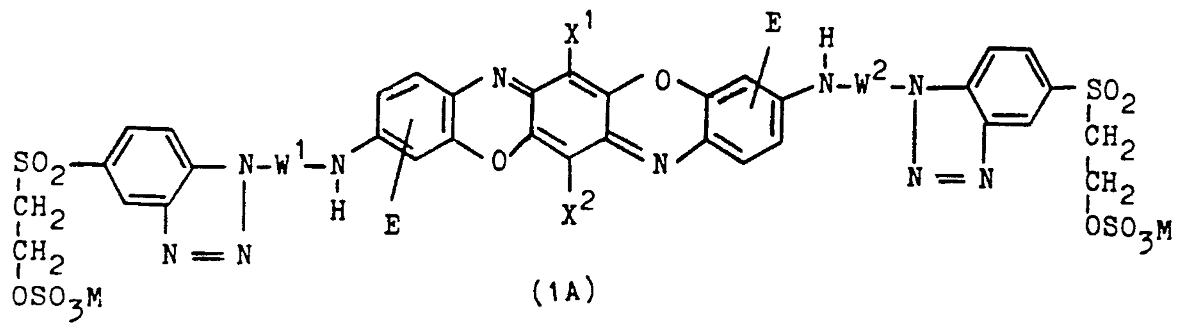

(1A)

(worin M die in der Beschreibung angegebenen Bedeutung hat) mit Hilfe ihrer Fomelreste beschrieben (hierin stellt der Rest $W^2$ jeweils die "spiegelbildlich" strukturierte Gruppe des aufgezeigten Restes $W^1$ dar). Sie lassen sich in erfindungsgemäßer Weise, beispielsweise analog den obigen Ausführungsbeispielen durch Umsetzung einer aus dem jeweiligen Tabellenbeispiel ersichtlichen 1,4-Benzochinon-Verbindung entsprechend der allgemeinen Formel (5) mit einer Verbindung der allgemeinen Formel (4A)

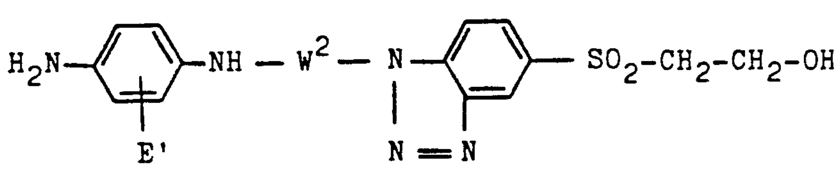

(4A)

(wobei E' das Hydroxy-Derivat von E ist, falls E eine Sulfatogruppe enthält) und nachträglicher Sulfatierung und Cyclisierung, herstellen. Sie besitzen ebenfalls sehr gute faserreaktive Farbstoffeigenschaften und liefern auf den in der Beschreibung genannten Materialien, wie insbesondere Cellulosefasermaterialien, farbstarke, echte Färbungen mit dem in dem jeweiligen Tabellenbeispiel auf Baumwolle angegebenen Farbton (mit der in Klammern angegebenen Farbkennzahl nach Colour Index Hue Indication Chart). Die in eckigen Klammern angegebenen Werte sind die Absorptionsmaxima (in nm) dieser Farbstoffe im sichtbaren Bereich, gemessen in wäßriger Lösung.

| Bsp. | Verbindung entsprechend Formel (1A) | | | | Verbindung (5) = ... 1,4-benzochinon | Farbton |
|---|---|---|---|---|---|---|
| | Rest E | Rest $W^2$ | $X^1$ ist ... | $X^2$ ist ... | | |
| 3 | Sulfo | 1,2-Ethylen | Brom | Brom | 2,3,5,6-Tetra-bromo-... | rotstichig blau (13) /¯612_7 |
| 4 | Sulfo | dito | Methyl | Methyl | 2,5-Dimethyl-3,6-dichloro-... | dito |
| 5 | Sulfo | dito | Methyl | Chlor | 2-Methyl-3,5,6-trichloro-... | dito |
| 6 | Sulfo | dito | Methoxy | Methoxy | 2,3,5,6-Tetra-methoxy-... | dito |
| 7 | Sulfo | 1,3-Propylen | Chlor | Chlor | 2,3,5,6-Tetra-chloro-... | dito /¯618_7 |
| 8 | Sulfo | dito | Brom | Brom | 2,3,5,6-Tetra-bromo-... | dito /¯618_7 |
| 9 | Sulfo | dito | Methyl | Methyl | 2,5-Dimethyl-3,6-dichloro-... | dito |
| 10 | Sulfo | dito | Methyl | Chlor | 2-Methyl-3,5,6-trichloro-... | dito |
| 11 | Sulfo | $-CH(CH_3)-CH_2-$ | Chlor | Chlor | 2,3,5,6-Tetra-chloro-... | dito /¯614_7 |
| 12 | Sulfo | dito | Brom | Brom | 2,3,5,6-Tetra-bromo-... | dito /¯614_7 |
| 13 | Sulfo | 1,4-Butylen | Chlor | Chlor | 2,3,5,6-Tetra-chloro-... | dito |
| 14 | Sulfo | 1,6-Hexylen | Chlor | Chlor | dito | dito |
| 15 | Sulfo | 2-Sulfato-1,3-propylen | Chlor | Chlor | dito | dito |

| Bsp. | Verbindung entsprechend Formel (1A) | | | | Verbindung (5) = ... 1,4-benzo-chinon | Farbton |
|---|---|---|---|---|---|---|
| | Rest E | Rest $W^2$ | $X^1$ ist ... | $X^2$ ist ... | | |
| 16 | Sulfo | $-CH_2-CH_2-$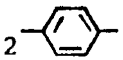 | Chlor | Chlor | 2,3,5,6-Tetra-chloro-... | rotstichig blau (13) |
| 17 | Sulfo | $-CH_2-CH_2-NH-$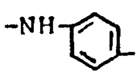 | Chlor | Chlor | dito | dito |
| 18 | Sulfo | $-(CH_2)_3-NH-$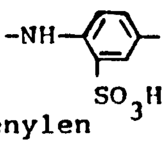 | Chlor | Chlor | dito | dito |
| 19 | Sulfo | 1,4-Phenylen | Chlor | Chlor | dito | grünstichig blau (15) |
| 20 | Sulfo | 1,5-Pentylen | Chlor | Chlor | dito | rotstichig blau (13) |
| 21 | Sulfo | $-CH(C_2H_5)-CH_2-$ | Chlor | Chlor | dito | dito |
| 22 | Sulfo | $-CH(CH_3)-CH_2-CH_2-$ | Chlor | Chlor | dito | dito |
| 23 | Sulfo | 2-Methyl-1,3-propylen | Chlor | Chlor | dito | dito |
| 24 | Sulfo | 2-Methoxy-1,3-propylen | Chlor | Chlor | dito | dito |
| 25 | Sulfo | $-(CH_2)_3-O-(CH_2)_2-O-(CH_2)_3-$ | Chlor | Chlor | dito | dito |
| 26 | Sulfo | $-(CH_2)_2-SO_2-(CH_2)_2-$ | Chlor | Chlor | dito | dito |

| Bsp. | Verbindung entsprechend Formel (1A) Rest E | Rest $W^2$ | $X^1$ ist ... | $X^2$ ist ... | Verbindung (5) = ... 1,4-benzochinon | Farbton |
|---|---|---|---|---|---|---|
| 27 | Sulfo | $-CH_2-$ 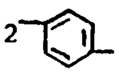 | Chlor | Chlor | 2,3,5,6-Tetrachloro-... | rotstichig blau (13) |
| 28 | Sulfo | $-(CH_2)_2-N(CH_3)-(CH_2)_2-$ | Chlor | Chlor | dito | dito |
| 29 | Sulfo | $-CH_2-CH_2-O-$ 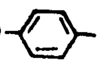 | Chlor | Chlor | dito | dito |
| 30 | Sulfo | $-CH(COOH)-(CH_2)_4-$ | Chlor | Chlor | dito | dito |
| 31 | Sulfo | $-(CH_2)_3-NH-(CH_2)_3-$ | Chlor | Chlor | dito | dito |
| 32 | Sulfo | 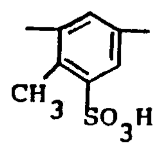 | Chlor | Chlor | dito | grünstichig blau (15) |
| 33 | Carboxy | 1,2-Ethylen | Chlor | Chlor | dito | rotstichig blau (13) |
| 34 | Carboxy | 1,2-Ethylen | Brom | Brom | 2,3,5,6-Tetrabromo-... | dito |
| 35 | Carboxy | dito | Methyl | Chlor | 2-Methyl-3,5,6-trichloro-... | dito |
| 36 | Carboxy | dito | Methoxy | Methoxy | 2,3,5,6-Tetramethoxy-... | dito |

| Bsp. | Verbindung entsprechend Formel (1A)<br>Rest E | Rest $W^2$ | $X^1$ ist ... | $X^2$ ist ... | Verbindung (5) = ... 1,4-benzo-chinon | Farbton |
|---|---|---|---|---|---|---|
| 37 | Carboxy | 1,3-Propylen | Chlor | Chlor | 2,3,5,6-Tetra-chloro-... | rotstichig blau (13) |
| 38 | Carboxy | dito | Brom | Brom | 2,3,5,6-Tetra-bromo-... | dito |
| 39 | Carboxy | dito | Methyl | Methyl | 2,5-Dimethyl-3,6-dichloro-... | dito |
| 40 | Carboxy | dito | Methyl | Chlor | 2-Methyl-3,5,6-trichloro-... | dito |
| 41 | Carboxy | $-CH(CH_3)-CH_2-$ | Chlor | Chlor | 2,3,5,6-Tetra-chloro-... | dito |
| 42 | Carboxy | dito | Brom | Brom | 2,3,5,6-Tetra-bromo-... | dito |
| 43 | Carboxy | 2-(4'-Sulfo-phenyl)-1,3-propylen | Chlor | Chlor | 2,3,5,6-Tetra-chloro-... | dito |
| 44 | Carboxy | $-CH_2-CH_2-O-CH_2-CH_2-$ | Chlor | Chlor | dito | dito |
| 45 | Carboxy | 2-Sulfato-1,3-propylen | Chlor | Chlor | dito | dito |
| 46 | Carboxy | $-CH_2-CH_2-$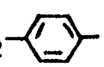 | Chlor | Chlor | dito | dito |
| 47 | Carboxy | $-(CH_2)_2-NH-$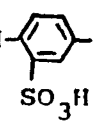 | Chlor | Chlor | dito | dito |

| Bsp. | Rest E | Rest $W^2$ | $X^1$ ist ... | $X^2$ ist ... | Verbindung (5) = ... 1,4-benzo-chinon | Farbton |
|---|---|---|---|---|---|---|
| | Verbindung entsprechend Formel (1A) | | | | | |
| 48 | Carboxy | $-(CH_2)_3-NH-$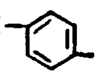 | Chlor | Chlor | 2,3,5,6-Tetra-chloro-... | rotstichig blau (13) |
| 49 | Carboxy | Sulfophen-1,4-ylen | Chlor | Chlor | dito | grünstichig blau (14) |
| 50 | N,N-Di-(ß-sulfato-ethyl)-sulfamoyl | 1,2-Ethylen | Brom | Brom | 2,3,5,6-Tetra-bromo-... | rotstichig blau (13) |
| 51 | dito | dito | Chlor | Chlor | 2,3,5,6-Tetra-chloro-... | dito |
| 52 | dito | dito | Methyl | Chlor | 2-Methyl-3,5,6-trichloro-... | dito |
| 53 | dito | dito | Methoxy | Methoxy | 2,3,5,6-Tetra-methoxy-... | dito |
| 54 | dito | 1,3-Propylen | Chlor | Chlor | 2,3,5,6-Tetra-chloro-... | dito |
| 55 | dito | dito | Brom | Brom | 2,3,5,6-Tetra-bromo-... | dito |
| 56 | dito | dito | Methyl | Methyl | 2,5-Dimethyl-3,6-dichloro-... | dito |
| 57 | dito | dito | Methyl | Chlor | 2-Methyl-3,5,6-trichloro-... | dito |

| Bsp. | Verbindung entsprechend Formel (1A) Rest E | Rest $W^2$ | $X^1$ ist ... | $X^2$ ist ... | Verbindung (5) = ... 1,4-benzo-chinon | Farbton |
|---|---|---|---|---|---|---|
| 58 | dito | $-CH(CH_3)-CH_2-$ | Chlor | Chlor | 2,3,5,6-Tetra-chloro-... | dito |
| 59 | dito | dito | Brom | Brom | 2,3,5,6-Tetra-bromo-... | dito |
| 60 | dito | 1,3-Cyclohexylen | Chlor | Chlor | 2,3,5,6-Tetra-chloro-... | dito |
| 61 | dito | Bis-(1,4-cyclo-hexylen)-methan | Chlor | Chlor | dito | dito |
| 62 | dito | 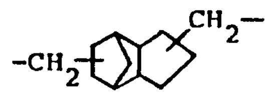 | Chlor | Chlor | dito | dito |
| 63 | dito | 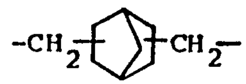 | Chlor | Chlor | dito | dito |
| 64 | dito | 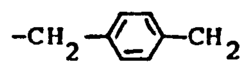 | Chlor | Chlor | dito | dito |
| 65 | dito | 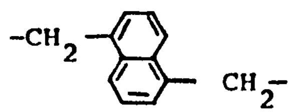 | Chlor | Chlor | dito | dito |
| 66 | dito | 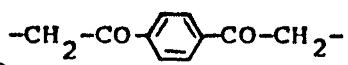 | Chlor | Chlor | dito | dito |
| 67 | N-(ß-Sul-fatoethyl)-sulfamoyl | 1,2-Ethylen | Chlor | Chlor | dito | dito |

| Bsp. | Verbindung entsprechend Formel (1A) Rest E | Rest $W^2$ | $X^1$ ist ... | $X^2$ ist ... | Verbindung (5) = ...1,4-benzo-chinon | Farbton |
|---|---|---|---|---|---|---|
| 68 | dito | 1,3-Propylen | Chlor | Chlor | dito | dito |
| 69 | dito | $-(CH_2)_2-N(COCH_3)-(CH_2)_2-$ | Chlor | Chlor | dito | dito |
| 70 | dito | 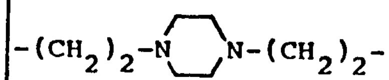 | Chlor | Chlor | dito | dito |
| 71 | N-(ß-Sulfoethyl)-sulfamoyl | 1,2-Ethylen | Chlor | Chlor | dito | dito |
| 72 | N-Methyl-N-(ß-sulfoethyl)-sulfamoyl | 1,3-Propylen | Chlor | Chlor | dito | dito |
| 73 | dito | 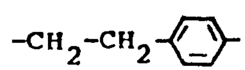 | Chlor | Chlor | dito | dito |
| 74 | dito | 1,5-Disulfo-naphth-3,7-ylen | Chlor | Chlor | dito | dito |
| 75 | dito | 1,2-Ethylen | Chlor | Chlor | dito | dito |
| 76 | N-[(Sulfophenyl)-sulfonyl]-sulfamoyl | dito | Chlor | Chlor | dito | dito |

| Bsp. | Verbindung entsprechend Formel (1A) | | | | Verbindung (5) = ... 1,4-benzo-chinon | Farbton |
|---|---|---|---|---|---|---|
| | Rest E | Rest $W^2$ | $X^1$ ist ... | $X^2$ ist ... | | |
| 77 | N-[(β-Sulfatoethyl)-sulfonyl-methyl]-sulfamoyl | 2-(4'-Sulfo-phenyl)-1,3-propylen | Chlor | Chlor | dito | dito |
| 78 | N-β-(4-Sulfophenyl)-ethyl-sulf-amoyl | 2-Sulfato-1,3-propylen | Chlor | Chlor | dito | dito |
| 79 | N-[4-Methoxy-3-(β-sulfato-ethylsulfonyl-phenyl]-sulfamoyl | dito | Chlor | Chlor | dito | dito |
| 80 | N-β-[2-Sulfo-4-(β-sulfatoethyl-sulfonyl)-phenyl]-ethyl-sulfamoyl | 1,2-Ethylen | Chlor | Chlor | dito | dito |
| 81 | Carboxy | dito | Methyl | Methyl | 2,5-Dimethyl-3,6-dichloro-... | dito |

Beispiel 82

a) 441 Teile der Verbindung der Formel

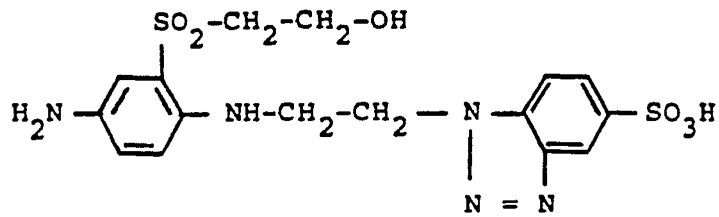

werden in 2000 Teilen Wasser bei pH 6 und 65°C gelöst. 124 Teile Chloranil werden eingetragen, wobei mit etwa 90 Teilen Natriumbicarbonat ein pH-Wert von 6 bis 6,5 und eine Reaktionstemperatur von etwa 65°C gehalten wird. Der Ansatz wird noch zehn Stunden nachgerührt, das Reaktionsprodukt sodann bei etwa 30°C geklärt, mit wenig Natriumchlorid ausgefällt, abgesaugt, mit 1000 Teilen einer 10%igen wäßrigen Natriumchloridlösung gewaschen und unter reduziertem Druck bei 70°C getrocknet.

b) 105 Teile des unter a) erhaltenen Produktes werden bei einer Temperatur zwischen 20 und 25°C in 750 Teile 13%iges Oleum eingetragen. Der Reaktionsansatz wird anschließend noch etwa zehn Stunden bei dieser Temperatur nachgerührt; sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, filtriert die ausgefallene erfindungsgemäße Verbindung ab, löst sie wieder in etwa 1000 Teilen Wasser, stellt mit Natriumcarbonat einen pH-Wert von 5 ein und salzt sie, gegebenenfalls nach vorheriger üblicher Klärung der Lösung, mit Natriumchlorid aus.

Die erfindungsgemäße Triphendioxazin-Verbindung kann auch in Form ihres Natriumsalzes durch Eindampfen oder Sprühtrocknen de geklärten Syntheselösung erhalten werden. Sie besitzt, in Form der freien Säure geschrieben, die mutmaßliche Formel

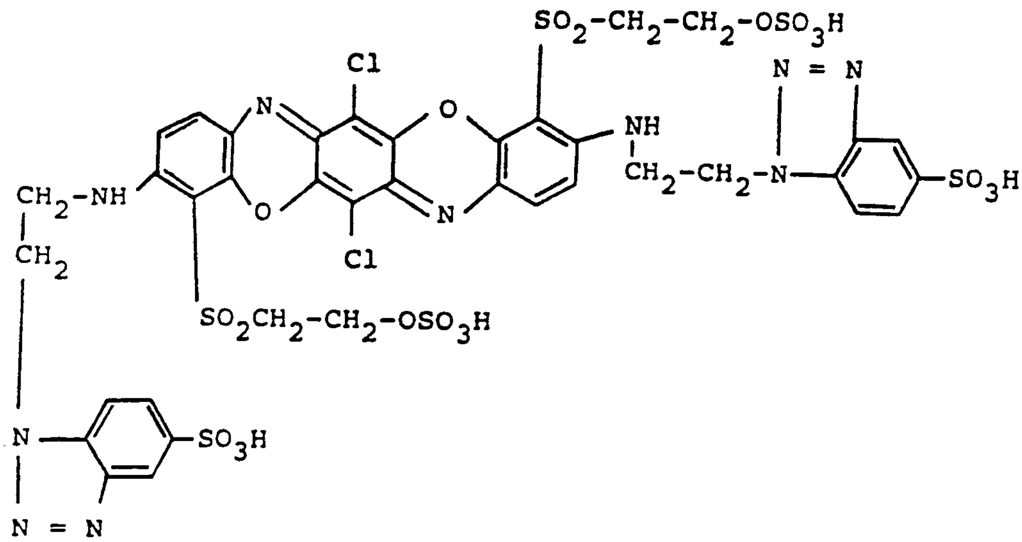

(die jeweilige Sulfatoethylsulfonylgruppe kann auch in der anderen ortho-Stellung zur Sulfobenztriazolylethylamino-Gruppe gebunden sein, befindet sich jedoch mit größerer Wahrscheinlichkeit in der in der obigen Formel angegebenen Stellung). Diese erfindungsgemäße Verbindung besitzt gute faserreaktive Farbstoffeigenschaften. Sie färbt die in der Beschreibung genannten Materialien, insbesondere Cellulosefasermaterialien, wie Baumwolle, nach den in der Technick üblichen und bekannten Verfahrensweisen der Applikation und Fixierung von faserreaktiven Farbstoffen in farbstarken, reinen rotstichig blauen Tönen (entsprechend der Farbkennzahl 13 von Colour Index Hue Indication Chart) mit guten Echtheiten, wie insbesondere guter Lichtechtheit der trockenen oder feuchten, wie mit Trinkwasser befeuchteten Färbung, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit. In wäßriger Lösung zeigt sie im sichtbaren Bereich ein Absorptionsmaximum bei 604 nm.

c) Das unter a) eingesetzte 4-[β-(5'-β-Sulfobenzotriazol-1'-yl)-ethylamino]-3-(β-hydroxyethylsulfonyl)-anilin kann beispielsweise wie folgt hergestellt werden:

266 Teile 4-Nitro-2-(β-hydroxyethylsulfonyl)-chlorbenzol werden zu einer 30°C vorgeheizten Mischung

von 243 Teilen 5-Sulfo-1-(β-aminoethyl)-benzotriazol in 1000 Teilen Wasser und 600 Teilen Triethanolamin gegeben. Innerhalb von zwei Stunden wird die Mischung auf 100 bis 110°C aufgeheizt, wobei das Wasser teilweise abdestilliert. Man rührt weitere 10 Stunden bei 115 bis 120°C zur quantitativen Umsetzung nach und gibt sodann bei 100°C 3000 Teile Wasser hinzu und klärt die Lösung bei 80 bis 90°C. Aus dem wäßrigen Medium kristallisiert beim Abkühlen das Natriumsalz des 4-[β-(5'-β-Sulfobenzotriazol-1'-yl)-ethylamino]-3-(β-hydroxyethylsulfonyl)nitrobenzol in hoher Ausbeute und Teinheit aus.

d) Die Nitroverbindung von c) wird durch katalytische Hydrierung zur Anilinverbindung reduziert, indem man 236 Teile der Nitroverbindung in 1000 Teilen Wasser löst (man kann sie statt dessen auch in Form ihrer obigen Losung einsetzen) und in Gegenwart eines Pd/C-Katalysators in einem Autoklaven bei einer Temperatur bis 100°C und einem Wasserstoffdruck von 50 bar hydriert. Der Katalysator wird anschließend abfiltriert, das Filtrat abgekühlt und angesäuert. Aus ihm kristallisiert die Anilinverbindung in guter Ausbeute und hoher Reinheit aus.

Die Hydrierlösung kann auch direkt zur Umsetzung nach a) verwendet werden.

e) Das unter c) eingesetzte 1-(β-Aminoethyl)-5-sulfo-benzo-1,2,3-triazol kann nach literaturebekannten Verfahrensweisen, so auch analog den obigen Ausführungsbeispielen 1 und 2, hergestellt werden, indem man 4-Sulfo-2-nitro-chlorbenzol mit Ethylendiamin umsetzt, das erhaltene 4-Sulfo-2-nitro-1-N-(β-aminoethyl)-anilin reduziert und anschließend diazotiert, wobei der gewünschte Ringschluß zum Benzotriazol erfolgt.

## Beispiele 83 bis 92

In den nachfolgenden Tabellenbeispielen sind weitere erfindungsgemäße Triphendioxazin-verbindungen entsprechend der allgemeinen Formel (1B)

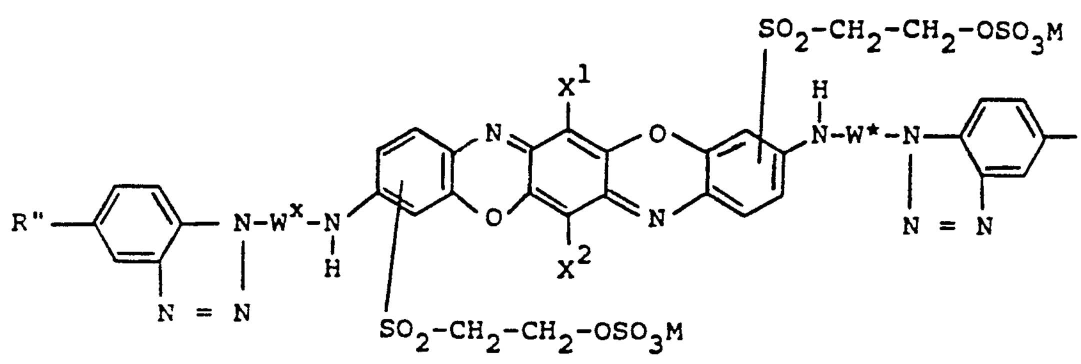

(1B)

(worin M die in der Beschreibung angegebene Bedeutung hat) mit Hilfe ihrer Fomelreste beschrieben (hierin stellt der Rest W* jeweils die "spiegelbildlich" strukturierte Gruppe des aufgezeigten Restes $W^x$ dar). Sie lassen sich in erfindungsgemäßer Weise, beispielsweise analog den obigen Ausführungsbeispielen durch Umsetzung einer aus dem jeweiligen Tabellenbeispiel ersichtlichen 1,4-Benzochinon-Verbindung entsprechend der allgemeinen Formel (5) mit einer Verbindung der allgemeinen Formel (4B)

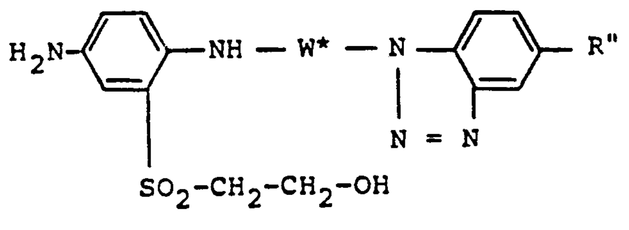

(4B)

(wobei in Formel (4B) R'' das Hydroxy-Derivat von R'' in Formel (1B) ist, falls dort R'' eine Sulfatogruppe enthält) und nachträglicher Sulfatierung und Cyclisierung, herstellen. Diese erfindungsgemäßen Triphendioxazin-Verbindungen besitzen ebenfalls sehr gute faserreaktive Farbstoffeigenschaften und liefern auf den in der Beschreibung genannten Materialien, wie insbesondere Cellulosefasermaterialien, farbstarke, echte Färbungen mit dem in dem jeweiligen Tabellenbeispiel auf Baumwolle angegebenen Farbton (mit der in Klammern angegebenen Farbkennzahl nach Colour Index Hue Indication Chart).

Für die in den Tabellenbeispielen 83 bis 86 beschriebenen Triphendioxazinverbindungen wurden folgende Absorptionsmaxima ($\lambda_{max}$) im sichtbaren Bereich in wäßriger Lösung ermittelt:

Beispiel 83: 604 nm.
Beispiel 84: 612 nm.
Beispiel 85: 612 nm.
Beispiel 86: 606 nm.

| Bsp. | Verbindung entsprechend Formel (1B) Gruppe R" | Rest W* | $X^1$ ist ... | $X^2$ ist ... | Verbindung (5) = ... 1,4-benzo-chinon | Farbton |
|---|---|---|---|---|---|---|
| 83 | Sulfo | 1,2-Ethylen | Brom | Brom | 2,3,5,6-Tetra-bromo-... | rotstichig blau (13) |
| 84 | Sulfo | 1,3-Propylen | Chlor | Chlor | 2,3,5,6-Tetra-chloro-... | dito |
| 85 | Sulfo | dito | Brom | Brom | 2,3,5,6-Tetra-bromo-... | dito |
| 86 | Sulfo | $-CH(CH_3)-CH_2-$ | Chlor | Chlor | 2,3,5,6-Tetra-chloro-... | dito |
| 87 | Sulfo | 2-Sulfato-1,3-propylen | Chlor | Chlor | dito | dito |
| 88 | Sulfo | $-CH_2-$ 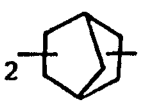 $-CH_2-$ | Chlor | Chlor | dito | dito |
| 89 | Sulfo | 1,4-(Bis-methylen)-phenylen | Chlor | Chlor | dito | dito |
| 90 | Wasserstoff | 1,5-Disulfo-naphth-3,7-ylen | Chlor | Chlor | dito | grünstichig blau (15) |
| 91 | ß-Sulfato-ethyl-sulfonyl | 2-Sulfato-1,3-propylen | Chlor | Chlor | dito | rotstichig blau (13) |
| 92 | dito | Sulfophen-1,4-ylen | Chlor | Chlor | dito | grünstichig blau (15) |

**Patentansprüche für die Vertragsstaaten: BE CH DE FR GB IT LI**

1. Wasserlösliche Triphendioxazin-Verbindung entsprechend der allgemeinen Formel (1)

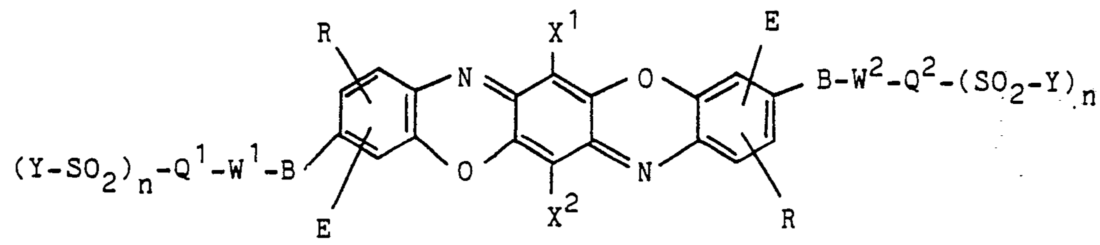

(1)

in welcher bedeuten:

n ist die Zahl Null oder 1, wobei im Falle n = 0 die Gruppe $-SO_2-Y$ ein Wasserstoffatom darstellt und n nur dann Null sein darf, wenn E eine Gruppe $-SO_2-Y$ mit Y gleich β-Sulfatoethyl ist;

Y ist die Vinylgruppe oder eine Ethylgruppe, die in β-Stellung einen durch ein Alkali eliminierbaren Substituenten enthält;

$Q^1$ ist ein Benzotriazolrest der nachstehend angegebenen allgemeinen Formel (2a) und

$Q^2$ ist ein Benzotriazolrest der nachstehend angegebenen allgemeinen Formel (2b)

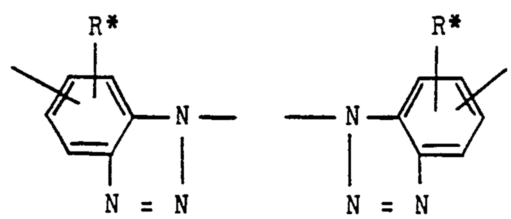

(2a) (2b)

in welchen

R* ein Wasserstoffatom, eine Alkylgruppe von 1 bis 4 C-Atomen, eine Alkoxygruppe von 1 bis 4 C-Atomen, ein Halogenatom oder eine Carboxy- oder Sulfogruppe bedeutet und die freie Bindung am Benzolrest die Bindung zur Gruppierung $(Y-SO_2)_n-$ kennzeichnet,

B ist ein Sauerstoff- oder Schwefelatom oder eine Aminogruppe der Formel $-N(R')-$, in welcher R' eine Alkylgruppe von 1 bis 6 C-Atomen ist, die substituiert sein kann, oder ist bevorzugt die Gruppe $-NH-$;

$W^1$ ist ein bivalenter, gegebenenfalls substituierter aliphatischer, gegebenenfalls durch Alkyl substituierter $(C_5-C_{10})$-cycloaliphatischer, gegebenenfalls durch Alkyl substituierter aliphatisch-$(C_5-C_8)$-cycloaliphatischer, gegebenenfalls substituierter araliphatischer oder gegebenenfalls substituierter aromatisch-carbocyclischer Rest, wobei die aliphatischen Reste in $W^1$ durch Heterogruppen unterbrochen sein können, die aus den Gruppen $-O-$, $-S-$, $-SO_2-$, $-CO-$, 1,4-Piperidino, $-NH-$ und $-N(R^o)-$, worin $R^o$ eine Alkylgruppe von 1 bis 6 C-Atomen, die substituiert sein kann, oder eine Alkanoylgruppe von 2 bis 5 C-Atomen ist, ausgewählt sind und/oder wobei aliphatische und Arylreste durch eine solche Heterogruppe miteinander verbunden sein können;

$W^2$ hat eine für $W^1$ angegebene Bedeutung und ist mit $W^1$ gleich oder von $W^1$ verschieden;

R ist ein Wasserstoffatom oder ein Alkyl von 1 bis 6 C-Atomen, ein Alkoxy von 1 bis 5 C-Atomen, ein Halogenatom oder eine Carboxy- oder Sulfogruppe, bevorzugt ein Wasserstoffatom;

E ist ein Wasserstoffatom oder eine Sulfo- oder Carboxygruppe oder eine Gruppe der allgemeinen Formel $-SO_2-Y$ mit Y der oben angegebenen Bedeutung oder eine gegebenenfalls substituierte Sulfonamidgruppe;

$X^1$ ist ein Wasserstoffatom oder ein Halogenatom; eine Cycloalkylgruppe von 5 bis 8 C-Atomen, eine Aralkyloxygruppe, eine Alkoxygruppe von 1 bis 4 C-Atomen, eine Aryloxygruppe, eine Alkylgruppe von 1 bis 4 C-Atomen, eine Arylgruppe, eine Aralkylgruppe, eine Cyangruppe, eine Carboxygruppe, eine Carbalkoxygruppe von 2 bis 5 C-Atomen, eine Arylaminogruppe, eine Carbamoylgruppe, eine N-Alkyl-carbamoyl-Gruppe oder N,N-Dialkyl-carbamoyl-Gruppe mit Alkylresten von jeweils 1 bis 4 C-Atomen, eine N-Aryl-carbamoyl-Gruppe, eine Alkanoylaminogruppe von 2 bis 5 C-Atomen oder eine Aroylaminogruppe, wobei die Arylreste in diesen genannten Substituenten bevorzugt Phenylreste sind, die noch durch 1 oder 2 Substituenten aus der Gruppe Halogen, Nitro, Alkyl von 1 bis 4 C-Atomen, Alkoxy von 1 bis 4 C-Atomen, Carboxy und Sulfo substituiert sein können, wobei $X^1$ bevorzugt ein Brom- oder Chloratom ist;

$X^2$ ist mit $X^1$ gleich oder von $X^1$ verschieden und hat eine der für $X^1$ angegebenen Bedeutungen;

die Gruppe E steht bevorzugt in ortho-Stellung zur Gruppe $-B-W^1-Q^1-(SO_2-Y)_n$ bzw. $-B-W^2-Q^2-(SO_2-Y)_n$ gebunden;

von den Carboxy-, Sulfo- und Sulfatogruppen, die im Molekül (1) enthalten sein können, enthält das Molekül (1) zwingend mindestens eine.

2. Verbindung nach Anspruch 1, dadurch gekennzeichnet, daß E eine Sulfogruppe ist.

3. Verbindung nach Anspruch 1 oder 2, dadurch gekennzeichnet, daß $W^1$ bzw. $W^2$ eine 1,3-Phenylen-, 1,4-Phenylen- oder β-(p-Phenylen)-ethylen-Gruppe oder eine 2-(4'-Sulfophenyl)-1,3-propylen- oder eine 2-Sulfato-1,3-propylen-Gruppe oder eine Gruppe der Formel $-CH_2-CH_2-O-CH_2-CH_2-$, $-CH_2-CH_2-NH-CH_2-CH_2-$ oder $-CH_2-CH_2-N(CH_3)-CH_2-CH_2-$ ist.

4. Verbindung nach Anspruch 1 oder 2, dadurch gekennzeichnet, daß $W^1$ eine Gruppe der Formel

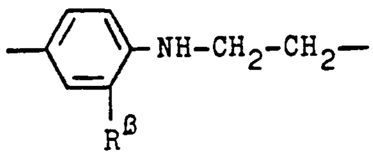

oder

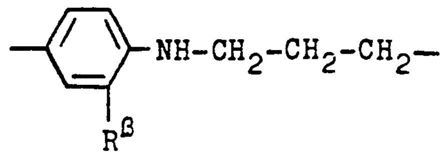

und $W^2$ eine Gruppe der Formel

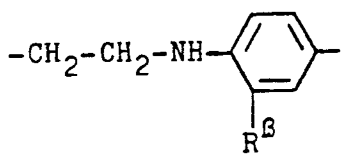

oder

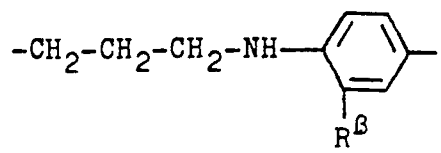

in welchen $R^β$ ein Wasserstoffatom oder eine Sulfogruppe ist, bedeuten.

5. Verbindung nach Anspruch 1 oder 2, dadurch gekennzeichnet, daß $W^1$ bzw. $W^2$ eine 1,2-Ethylen-, 1,3-Propylen-, 1,4-Butylen- oder eine Isopropylen-Gruppe ist.

6. Verbindung nach mindestens einem der Ansprüche 1, 2, 4, 5, 6 und 7, dadurch gekennzeichnet, daß E eine β-Sulfatoethylsulfonyl-, N,N-Di-(β-Sulfatoethyl)sulfamoyl-, N-(β-Sulfatoethyl)-sulfamoyl-, N-(β-Sulfoethyl)-sulfamoyl- oder N-Methyl-N-(β-sulfoethyl)-sulfamoyl-Gruppe ist.

7. Verbindung nach mindestens einem der Ansprüche 1 bis 6, dadurch gekennzeichnet, daß $X^1$ und $X^2$ beide für ein Chloratom stehen.

8. Verbindung nach Anspruch 1 entsprechend der allgemeinen Formel (1a)

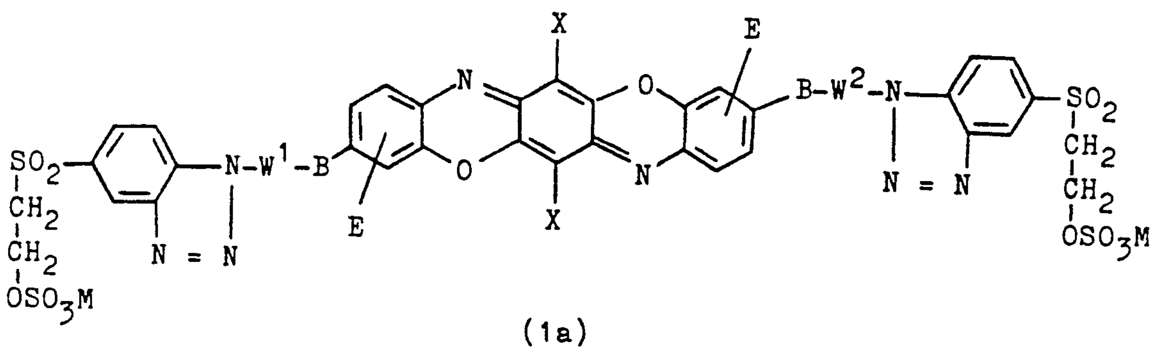

(1a)

in welcher

X für ein Brom- oder ein Chloratom steht,

M ein Wasserstoffatom oder ein Alkalimetall ist,

E eine Sulfo- oder Carboxygruppe bedeutet und

$W^1$ und $W^2$ beide eine 1,2-Ethylen- oder 1,3-Propylen-Gruppe bedeuten.

9. Verbindung nach Anspruch 8, dadurch gekennzeichnet, daß E eine Sulfogruppe und X ein Chloratom ist.

10. Verbindung nach Anspruch 1 entsprechend der allgemeinen Formel (1b)

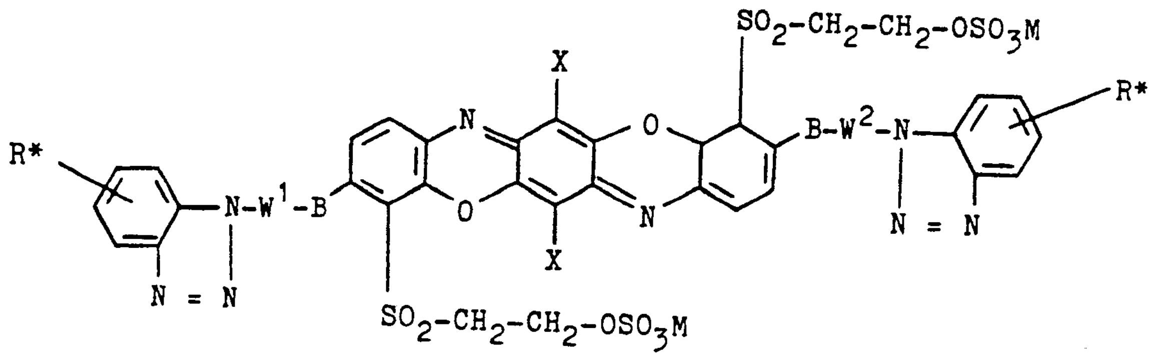

(1b)

in welcher

X für ein Bromatom oder ein Chloratom steht,

M ein Wasserstoffatom oder ein Alkalimetall ist,

B die Gruppe —NH— bedeutet,

$W^1$ und $W^2$ beide für die 1,2-Ethylen- oder 1,3-Propylen- oder eine Isopropylen-Gruppe stehen oder $W^1$ eine Gruppe der Formel

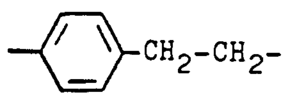

oder

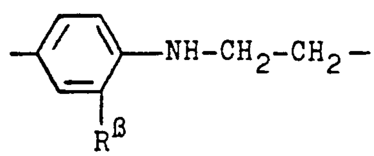

oder

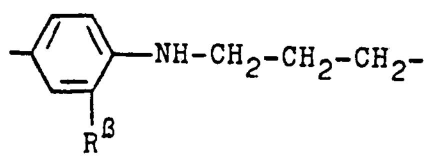

und $W^2$ eine Gruppe der Formel

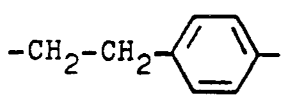

oder

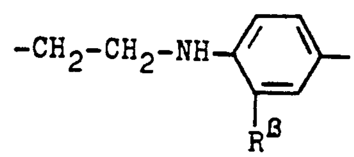

oder

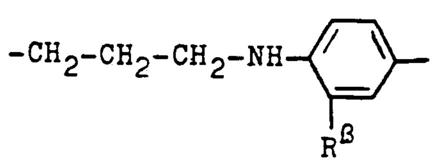

in welchen $R^\beta$ für ein Wasserstoffatom oder eine Sulfogruppe steht, und

R* jeweils eine Sulfogruppe bedeutet.

11. Verbindung nach Anspruch 1 entsprechend der allgemeinen Formel (1c)

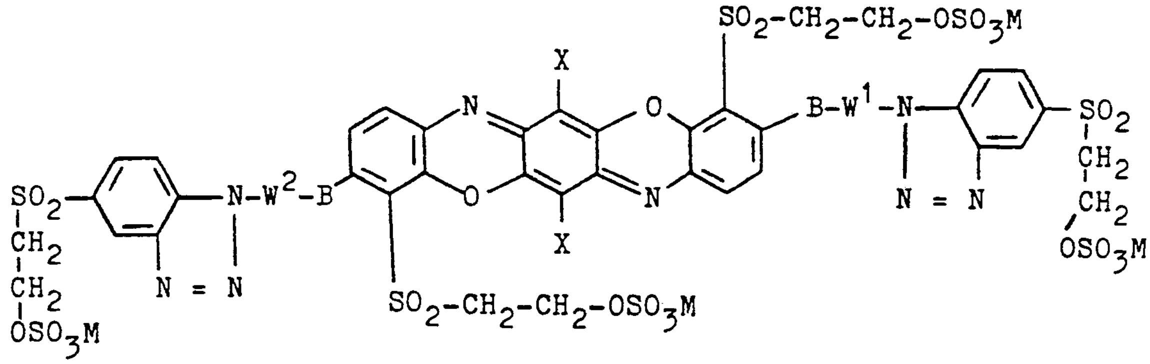

(1c)

in welcher
X für ein Brom- oder ein Chloratom steht,
M ein Wasserstoffatom oder ein Alkalimetall ist,
B die Gruppe —NH— bedeutet und
$W^1$ und $W^2$ beide einen bivalenten, sulfogruppenhaltigen oder sulfatogruppenhaltigen Rest bedeuten.

12. Verbindung nach Anspruch 10 oder 11 oder beiden, dadurch gekennzeichnet, daß beide X für ein Chloratom stehen.

13. Verfahren zur Herstellung der in Anspruch 1 genannten und definierten Verbindungen, dadurch gekennzeichnet, daß man eine Verbindung der allgemeinen Formel (3)

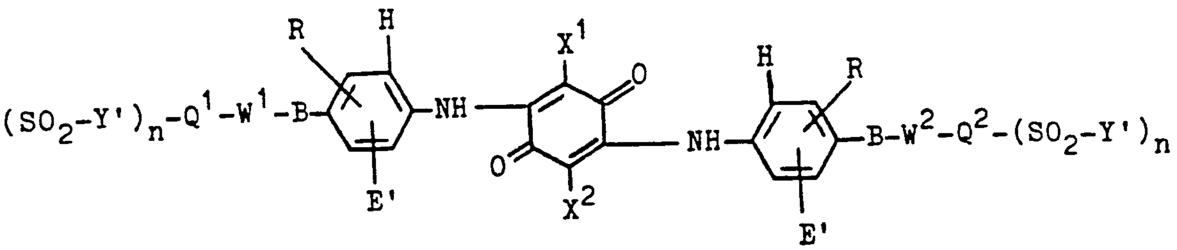

(3)

(in welcher Y' die Vinylgruppe, die β-Hydroxyethyl-Gruppe oder eine Ethylgruppe, die in β-Stellung einen durch ein Alkali eliminierbaren Substituenten enthält, und n, R, B, $Q^1$, $Q^2$, $W^1$, $W^2$, $X^1$ und $X^2$ die in Anspruch 1 genannten Bedeutungen haben und E' eine der für E in Anspruch 1 genannten Bedeutungen hat oder eine β-Hydroxyethylsulfonylgruppe ist und wobei substituierte Alkylgruppen in diesen Resten auch hydroxysubstituierte Alkylgruppen sein können, die Gruppen E' bevorzugt in ortho-Stellung zur Gruppe $—B—W^2—Q^2—(SO_2—Y')_n$ bzw. $(SO_2—Y')_n—Q^1—W^1—$B-gebunden sind und die Benzolkerne in einer der ortho-Stellungen zur angegebenen Aminogruppe —NH— nicht substituiert sein dürfen) in saurem Medium und vorzugsweise in Gegenwart eines Oxidationsmittels zum Triphendioxazin cyclisiert, wobei man gegebenenfalls vor oder gleichzeitig mit der Cyclisierung oder erst nach der Cyclisierungsreaktion gegebenenfalls vorhandene Hydroxyalkylgruppen, mittels eines Sulfatisierungs- oder Phosphatierungsmittels, in die entsprechenden β-Sulfatoalkyl- bzw. β-Phosphatoalkyl-Gruppen verestert und gegebenenfalls gleichzeitig Sulfogruppen in Arylreste einführt.

14. Verwendung einer in Anspruch 1 genannten und definierten Verbindung der allgemeinen Formel (1) zum Färben (einschließlich Bedrucken) von hydroxy- und/oder carbonamidgruppenhaltigem Material, insbesondere Fasermaterial.

15. Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder in das Material einbringt und ihn mittels Wärme und/oder mittels eines säurebindenden Mittels fixiert, dadurch gekennzeichnet, daß man als Farbstoff eine Verbindung der in Anspruch 1 genannten und definierten allgemeinen Formel (1) einsetzt.

16. Verbindung der allgemeinen Formel (4)

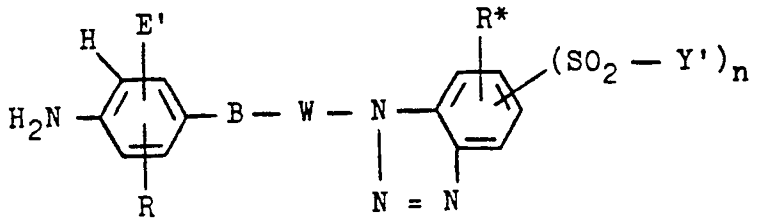

(4)

in welcher Y' die Vinylgruppe, die β-Hydroxyethyl-Gruppe oder eine Ethylgruppe, die in β-Stellung einen durch ein Alkali eliminierbaren Substituenten enthält, bedeutet, R, B, R* und n die in Anspruch 1 genannten Bedeutungen haben, W ein bivalenter, gegebenenfalls substituierter aliphatischer, gegebenenfalls durch Alkyl substituierter ($C_5$—$C_{10}$)-cycloaliphatischer, gegebenenfalls durch Alkyl substituierter aliphatisch-($C_5$—$C_8$)-cycloaliphatischer, gegebenenfalls substituierter araliphatischer oder gegebenenfalls substituierter aromatisch-carbocyclischer Rest ist, wobei die aliphatischen Reste in W durch Heterogruppen, bevorzugt 1 oder 2 Heterogruppen, unterbrochen sein können, die aus den Gruppen —O—, —S—, —$SO_2$—, —CO—, 1,4-Piperidino, —NH— und —N(R°)—, worin R° eine Alkylgruppe von 1 bis 6 C-Atomen, die substituiert sein kann, oder eine Alkanoylgruppe von 2 bis 5 C-Atomen ist, ausgewählt sind, und/oder wobei aliphatische und Arylreste durch eine solche Heterogruppe miteinander verbunden sein können, sowie E' ein Wasserstoffatom, eine Sulfo- oder Carboxygruppe oder eine Gruppe der oben definierten Formel —$SO_2$—Y' oder eine gegebenenfalls substituierte Sulfonamidgruppe ist, wobei die Gruppe E' bevorzugt in ortho-Stellung zum Rest B gebunden steht.

17. Verbindung der allgemeinen Formel (8)

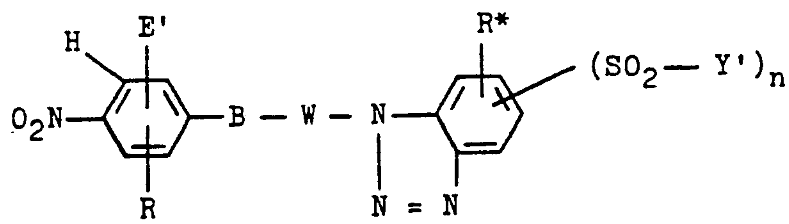

(8)

in welcher Y' die Vinylgruppe, die β-Hydroxyethyl-Gruppe oder eine Ethylgruppe, die in β-Stellung einen durch ein Alkali eliminierten Substituenten enthält, bedeutet, R, B, R* und n die in Anspruch 1 genannten Bedeutungen haben, W ein bivalenter, gegebenenfalls substituierter aliphatischer, gegebenenfalls durch Alkyl substituierter ($C_5$—$C_{10}$)-cycloaliphatischer, gegebenenfalls durch Alkyl substituierter aliphatisch—($C_5$—$C_8$)-cycloaliphatischer, gegebenenfalls substituierter araliphatischer, gegebenenfalls substituierter caromatisch-carbocyclischer Rest ist, wobei die aliphatischen Reste in W durch Heterogruppen, bevorzugt 1 oder 2 Heterogruppen, unterbrochen sein können, die aus den Gruppen —O—, —S—, —$SO_2$—, —CO—, 1,4-Piperidino, —NH— und —N(R°)—, worin R° eine Alkylgruppe von 1 bis 6 C-Atomen, die substituiert sein kann, oder eine Alkanoylgruppe von 2 bis 5 C-Atomen ist, ausgewählt sind, und/oder wobei aliphatische und Arylreste durch eine solche Heterogruppe miteinander verbunden sein können, sowie E' ein Wasserstoffatom, eine Sulfo- oder Carboxygruppe oder eine Gruppe der oben definierten Formel —$SO_2$—Y' oder eine gegebenenfalls substituierte Sulfonamidgruppe ist, wobei die Gruppe E' bevorzugt in ortho-Stellung zum Rest B gebunden steht.

18. Verfahren zur Herstellung einer in Anspruch 16 angegebenen und definierten Verbindung der allgemeinen Formel (4) oder einer in Anspruch 17 angegebenen und definierten Verbindung der allgemeinen Formel (8), dadurch gekennzeichnet, daß man eine Verbindung der allgemeinen Formel (6)

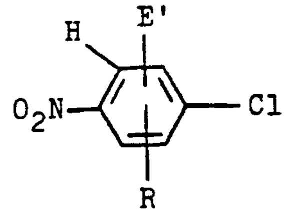

(6)

in welcher R und E' die in Anspruch 16 angegebenen Bedeutungen haben, mit einer Aminoverbindung der allgemeinen Formel (7)

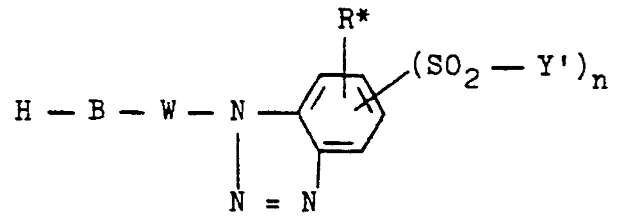

(7)

in welcher B, R*, W, Y' und n die in Anspruch 16 genannten Bedeutungen haben, unter Zusatz eines basischen, säurebindenden Mittels bei einer Temperatur zwischen 70 und 120°C umsetzt und gegebenenfalls in der so erhaltenen Nitroverbindung der allgemeinen Formel (8) die Nitrogruppe zur Aminogruppe reduziert.

19. Verwendung einer Verbindung der allgemeinen Formel (4) von Anspruch 16 oder einer Verbindung der allgemeinen Formel (8) von Anspruch 17 zur Synthese von Farbstoffen, insbesondere von Triphendioxazin-Verbindungen der allgemeinen Formel (1) gemäß Anspruch 1.

**Patentansprüche für den Vertragsstaat: ES**

1. Verfahren zur Herstellung einer wasserlöslichen Triphendioxazin-Verbindung entsprechend der allgemeinen Formel (1)

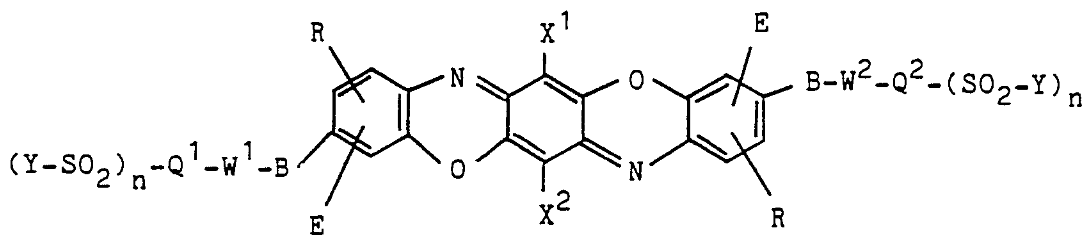

(1)

in welcher bedeuten:

n ist die Zahl Null oder 1, wobei im Falle n = 0 die Gruppe $—SO_2—Y$ ein Wasserstoffatom darstellt und n nur dann Null sein darf, wenn E eine Gruppe $—SO_2—Y$ mit Y gleich β-Sulfatoethyl ist;

Y ist die Vinylgruppe oder eine Ethylgruppe, die in β-Stellung einen durch ein Alkali eliminierbaren Substituenten enthält; und ist bevorzugt die β-Sulfatoethyl-Gruppe;

$Q^1$ ist ein Benzotriazolrest der nachstehend angegebenen allgemeinen Formel (2a) und

$Q^2$ ist ein Benzotriazolrest der nachstehend angegebenen allgemeinen Formel (2b)

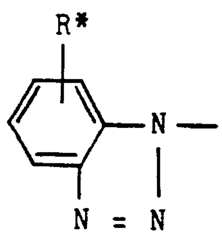

(2a)

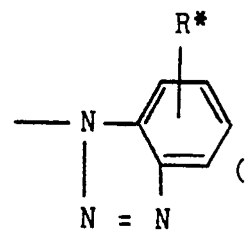

(2b)

in welchen

R* ein Wasserstoffatom, eine Alkylgruppe von 1 bis 4 C-Atomen, eine Alkoxygruppe von 1 bis 4 C-Atomen, ein Halogenatom oder eine Carboxy- oder Sulfogruppe bedeutet und die freie Bindung am Benzolrest die Bindung zur Gruppierung $(Y—SO_2)_n—$ kennzeichnet,

B ist ein Sauerstoff- oder Schwefelatom oder eine Aminogruppe der Formel —N(R')—, in welcher R' eine Alkylgruppe von 1 bis 6 C-Atomen ist, die substituiert sein kann, oder ist bevorzugt die Gruppe —NH—;

$W^1$ ist ein bivalenter, gegebenenfalls substituierter aliphatischer, gegebenenfalls durch Alkyl substituierter $(C_5—C_{10})$-cycloaliphatischer, gegebenenfalls durch Alkyl substituierter aliphatisch-$(C_5—C_8)$-cycloaliphatischer, gegebenenfalls substituierter araliphatischer oder gegebenenenfalls substituierter aromatisch-carbocyclischer Rest, wobei die aliphatischen Reste in $W^1$ durch Heterogruppen unterbrochen sein können, die aus den Gruppen —O—, —S—, $—SO_2—$, —CO—, 1,4-Piperidino, —NH— und —N(R°)—,

worin $R^o$ eine Alkylgruppe von 1 bis 6 C-Atomen, die substituiert sein kann, oder eine Alkanoylgruppe von 2 bis 5 C-Atomen ist, ausgewählt sind und/oder wobei aliphatische und Arylreste durch eine solche Heterogruppe miteinander verbunden sein können;

$W^2$ hat eine für $W^1$ angegebene Bedeutung und ist mit $W^1$ gleich oder von $W^1$ verschieden;

R ist ein Wasserstoffatom oder ein Alkyl von 1 bis 6 C-Atomen, ein Alkoxy von 1 bis 5 C-Atomen, ein Halogenatom oder eine Carboxy- oder Sulfogruppe, bevorzugt ein Wasserstoffatom;

E ist ein Wasserstoffatom oder eine Sulfo- oder Carboxygruppe oder eine Gruppe der allgemeinen Formel $—SO_2—Y$ mit Y der oben angegebenen Bedeutung oder eine gegebenfalls substituierte Sulfonamidgruppe;

$X^1$ ist ein Wasserstoffatom oder ein Halogenatom; eine Cycloalkylgruppe von 5 bis 8 C-Atomen, eine Aralkyloxygruppe, eine Alkoxygruppe von 1 bis 4 C-Atomen, eine Aryloxygruppe, eine Alkylgruppe von 1 bis 4 C-Atomen, eine Arylgruppe, eine Aralkylgruppe, eine Cyangruppe, eine Carboxygruppe, eine Carbalkoxygruppe von 2 bis 5 C-Atomen, eine Arylaminogruppe, eine Carbamoylgruppe, eine N-Alkyl-carbamoyl-Gruppe oder N,N-Dialkyl-carbamoyl-Gruppe mit Alkylresten von jeweils 1 bis 4 C-Atomen, eine N-Aryl-carbamoyl-Gruppe, eine Alkanoylaminogruppe von 2 bis 5 C-Atomen oder eine Aroylaminogruppe, wobei die Arylreste in diesen genannten Substituenten bevorzugt Phenylreste sind, die noch durch 1 oder 2 Substituenten aus der Gruppe Halogen, Nitro, Alkyl von 1 bis 4 C-Atomen, Alkoxy von 1 bis 4 C-Atomen, Carboxy und Sulfo substituiert sein können, wobei $X^1$ bevorzugt ein Brom- oder Chloratom ist;

$X^2$ ist mit $X^1$ gleich oder von $X^1$ verschieden und hat eine der für $X^1$ angegebenen Bedeutungen;

die Gruppe E steht bevorzugt in ortho-Stellung zur Gruppe $—B—W^1—Q^1—(SO_2—Y)_n$ bzw. $—B—W^2—Q^2—(SO_2—Y)_n$ gebunden;

von den Carboxy-, Sulfo- und Sulfatogruppen, die im Molekül (1) enthalten sein können, enthält das Molekül (1) zwingend mindestens eine, dadurch gekennzeichnet, daß man eine Verbindung der allgemeinen Formel (3)

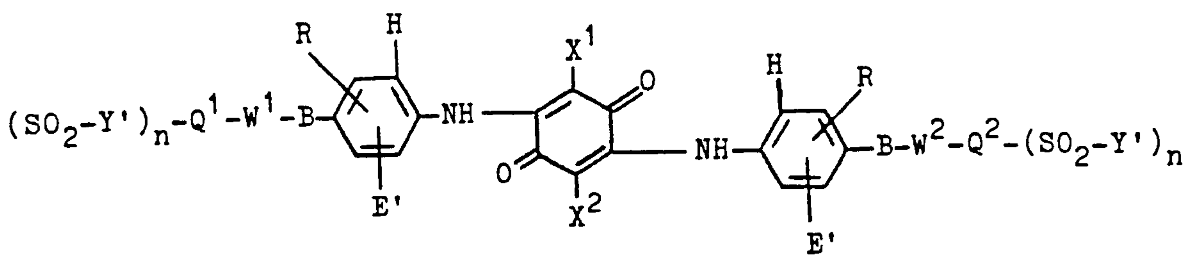

(3)

(in welcher Y' die Vinylgruppe, die β-Hydroxyethyl-Gruppe oder eine Ethylgruppe, die in β-Stellung einen durch ein Alkali eliminierbaren Substituenten enthält, und n, R, B, $Q^1$, $Q^2$, $W^1$, $W^2$, $X^1$ und $X^2$ die oben genannten Bedeutungen haben und E' eine der für E oben genannten Bedeutungen hat oder eine β-Hydroxyethylsulfonylgruppe ist und wobei substituierte Alkylgruppen in diesen Resten auch hydroxysubstituierte Alkylgruppen sein können, die Gruppen E' bevorzugt in ortho-Stellung zur Gruppe $—B—W^2—Q^2—(SO_2—Y')_n$ bzw. $(SO_2—Y')_n—Q^1—W^1—B—$ gebunden sind und die Benzolkerne in einer der ortho-Stellungen zur angegebenen Aminogruppe —NH— nicht substituiert sein dürfen) in saurem Medium und vorzugsweise in Gegenwart eines Oxidationsmittels zum Triphendioxazin cyclisiert, wobei man gegebenenfalls vor oder gleichzeitig mit der Cyclisierung oder erst nach der Cyclisierungsreaktion gegebenenfalls vorhandene Hydroxyalkylgruppen, mittels eines Sulfatisierungs- oder Phosphatierungsmittels, in die entsprechenden β-Sulfatoalkyl- bzw. β-Phosphatoalkyl-Gruppen verestert und gegebenenfalls gleichzeitig Sulfogruppen in Arylreste einführt.

2. Verfahren nach Anspruch 1 oder 2, dadurch gekennzeichnet, daß E eine Sulfogruppe ist.

3. Verfahren nach Anspruch 1 oder 2, dadurch gekennzeichnet, daß $W^1$ bzw. $W^2$ eine 1,3-Phenylen-, 1,4-Phenylen- oder β-(p-Phenylen)-ethylen-Gruppe oder eine 2-(4'-Sulfophenyl)-1,3-propylen- oder eine 2-Sulfato-1,3-propylen-Gruppe oder eine Gruppe der Formel $—CH_2—CH_2—O—CH_2—CH_2—$, $—CH_2—CH_2—NH—CH_2—CH_2—$ oder $—CH_2—CH_2—N(CH_3)—CH_2—CH_2—$ ist.

4. Verfahren nach Anspruch 1 oder 2, dadurch gekennzeichnet, daß $W^1$ eine Gruppe der Formel

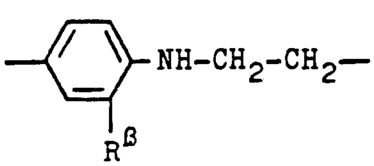

oder

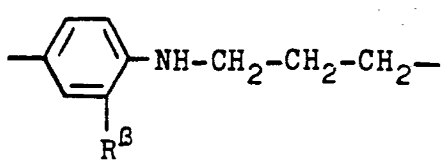

und $W^2$ eine Gruppe der Formel

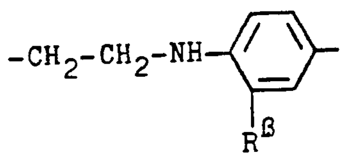 oder 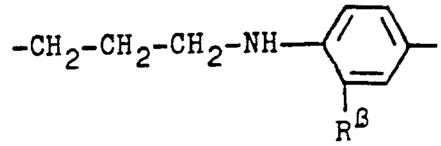

in welchen $R^{\beta}$ ein Wasserstoffatom oder eine Sulfogruppe ist, bedeuten.

5. Verfahren nach Anspruch 1 oder 2, dadurch gekennzeichnet, daß $W^1$ bzw. $W^2$ eine 1,2-Ethylen-, 1,3-Propylen-, 1,4-Butylen- oder eine Isopropylen-Gruppe ist.

6. Verfahren nach mindestens einem der Ansprüche 1, 2, 4, 5, 6 und 7, dadurch gekennzeichnet, daß E eine β-Sulfatoethylsulfonyl-, N,N-Di-(β-Sulfatoethyl)sulfamoyl-, N-(β-Sulfatoethyl)-sulfamoyl-, N-(β-Sulfoethyl)-sulfamoyl- oder N-Methyl-N-(β-sulfoethyl)-sulfamoyl-Gruppe ist.

7. Verfahren nach mindestens einem der Ansprüche 1 bis 6, dadurch gekennzeichnet, daß $X^1$ und $X^2$ beide für ein Chloratom stehen.

8. Verfahren nach Anspruch 1, dadurch gekennzeichnet, daß die erhaltene Verbindung der Formel (1) eine Verbindung entsprechend der allgemeinen Formel (1a)

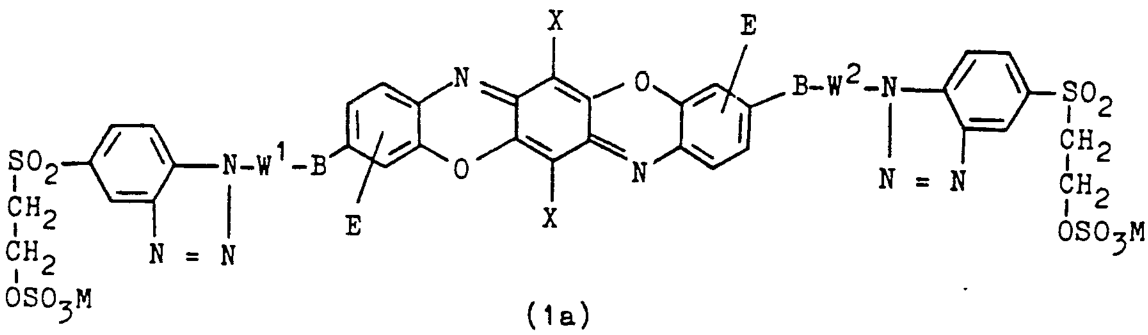

(1a)

ist, in welcher

X für ein Brom- oder ein Chloratom steht,

M ein Wasserstoffatom oder ein Alkalimetall ist,

E eine Sulfo- oder Carboxygruppe bedeutet und

$W^1$ und $W^2$ beide eine 1,2-Ethylen- oder 1,3-Propylen-Gruppe bedeuten.

9. Verfahren nach Anspruch 8, dadurch gekennzeichnet, daß E eine Sulfogruppe und X ein Chloratom ist.

10. Verfahren nach Anspruch 1, dadurch gekennzeichnet, daß die erhaltene Verbindung der Formel (1) eine Verbindung entsprechend der allgemeinen Formel (1b)

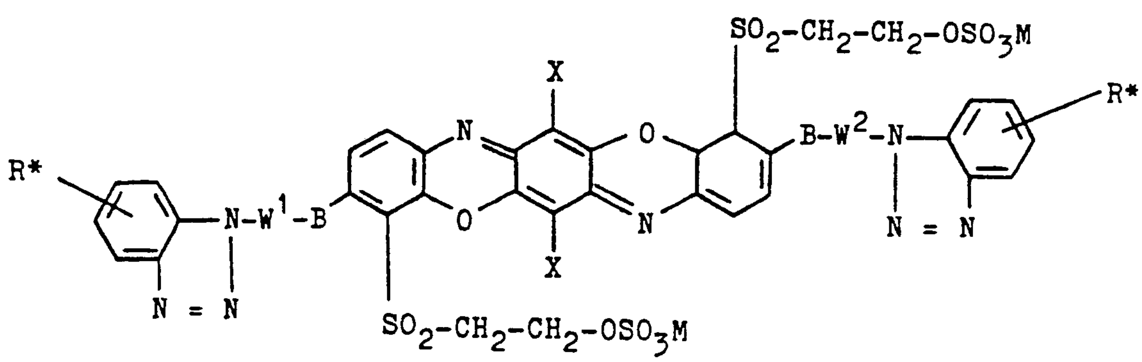

(1b)

ist, in welcher

X für ein Bromatom oder ein Chloratom steht,

M ein Wasserstoffatom oder ein Alkalimetall ist,

B die Gruppe —NH— bedeutet,

$W^1$ und $W^2$ beide für die 1,2-Ethylen- oder 1,3-Propylen- oder eine Isopropylen-Gruppe stehen oder $W^1$ eine Gruppe der Formel

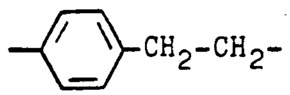 oder 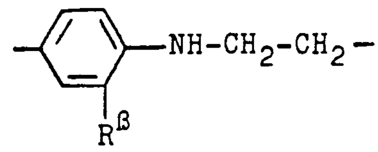

oder 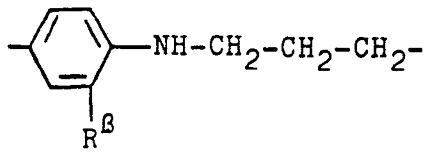

und $W^2$ eine Gruppe der Formel

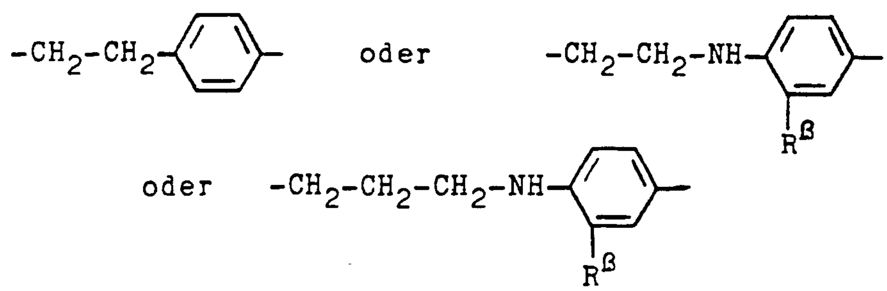

oder

oder

in welchen $R^ß$ für ein Wasserstoffatom oder eine Sulfogruppe steht, und

$R^*$ jeweils eine Sulfogruppe bedeutet.

11. Verfahren nach Anspruch 1, dadurch gekennzeichnet, daß die erhaltene Verbindung der Formel (1) eine Verbindung entsprechend der allgemeinen Formel (1c)

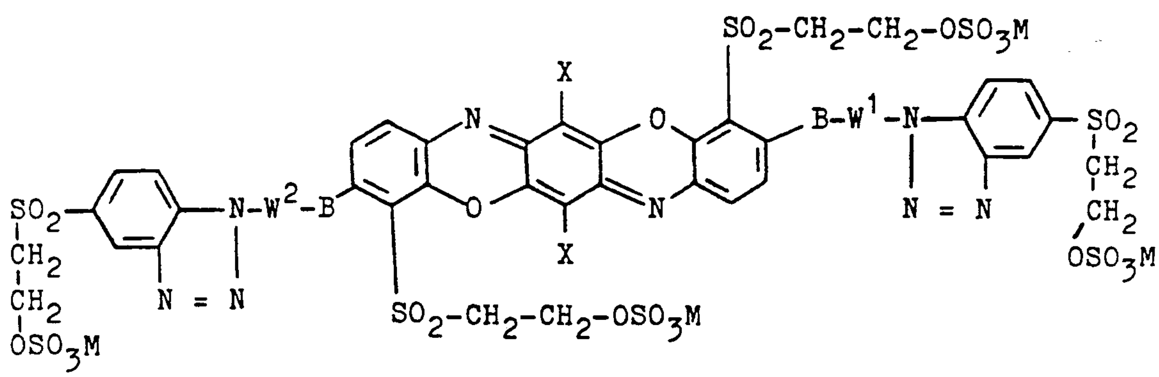

(1c)

ist, in welcher

X für ein Brom- oder ein Chloratom steht,

M ein Wasserstoffatom oder ein Alkalimetall ist,

B die Gruppe —NH— bedeutet und

$W^1$ und $W^2$ beide einen bivalenten, sulfogruppenhaltigen oder sulfatogruppenhaltigen Rest bedeuten.

12. Verfahren nach Anspruch 10 oder 11 oder beiden, dadurch gekennzeichnet, daß beide X für ein Chloratom stehen.

13. Verwendung einer in Anspruch 1 genannten und definierten Verbindung der allgemeinen Formel (1) oder einer nach Anspruch 1 hergestellten Verbindung der allgemeinen Formel (1) zum Färben (einschließlich Bedrucken) von hydroxy- und/oder carbonamidgruppenhaltigem Material, insbesondere Fasermaterial.

14. Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder in das Material einbringt und ihn mittels Wärme und/oder mittels eines säurebindenden Mittels fixiert, dadurch gekennzeichnet, daß man als Farbstoff eine Verbindung der in Anspruch 1 genannten und definierten allgemeinen Formel (1) einsetzt.

15. Verfahren zur Herstellung einer Verbindung der allgemeinen Formel (4)

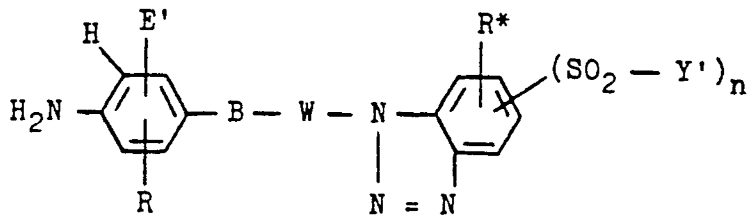

(4)

in welcher Y' die Vinylgruppe, die β-Hydroxyethyl-Gruppe oder eine Ethylgruppe, die in β-Stellung einen durch ein Alkali eliminierbaren Substituenten enthält, bedeutet, R, B, R* und n die in Anspruch 1 genannten Bedeutungen haben, W ein bivalenter, gegebenenfalls substituierter aliphatischer, gegebenenfalls durch Alkyl substituierter ($C_5$—$C_{10}$)-cycloaliphatischer, gegebenenfalls durch Alkyl substituierter aliphatisch-($C_5$—$C_8$)-cycloaliphatischer, gegebenenfalls substituierter araliphatischer oder gegebenenfalls substituierter aromatisch-carbocyclischer Rest ist, wobei die aliphatischen Reste in W durch Heterogruppen, bevorzugt 1 oder 2 Heterogruppen, unterbrochen sein können, die aus den Gruppen —O—, —S—, —$SO_2$—, —CO—, 1,4-Piperidino, —NH— und —N($R^o$)—, worin $R^o$ eine Alkylgruppe von 1 bis 6 C-Atomen, die substituiert sein kann, oder eine Alkanoylgruppe von 2 bis 5 C-Atomen ist, ausgewählt sind, und/oder wobei aliphatische und Arylreste durch eine solche Heterogruppe miteinander verbunden sein können, sowie E' ein Wasserstoffatom, eine Sulfo- oder Carboxygruppe oder eine Gruppe der oben definierten Formel —$SO_2$—Y' oder eine gegebenenfalls substituierte Sulfonamidgruppe ist, wobei die Gruppe E' bevorzugt in ortho-Stellung zum Rest B gebunden steht, dadurch gekennzeichnet, daß man eine Verbindung der allgemeinen Formel (6)

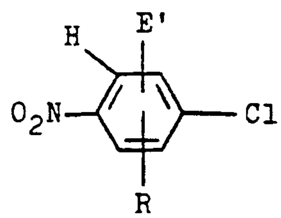

(6)

in welcher R und E' die oben angegebenen Bedeutungen haben, mit einer Aminoverbindung der allgemeinen Formel (7)

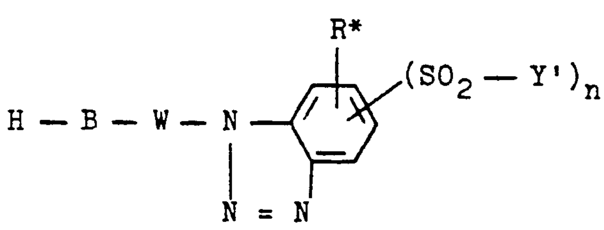

(7)

in welcher B, R*, W, Y' und n die oben genannten Bedeutungen haben, unter Zusatz eines basischen, säurebindenden Mittels bei einer Temperatur zwischen 70 und 120°C umsetzt und in der erhaltenen Nitroverbindung die Nitrogruppe zur Aminogruppe reduziert.

16. Verfahren zur Herstellung einer Verbindung der allgemeinen Formel (8)

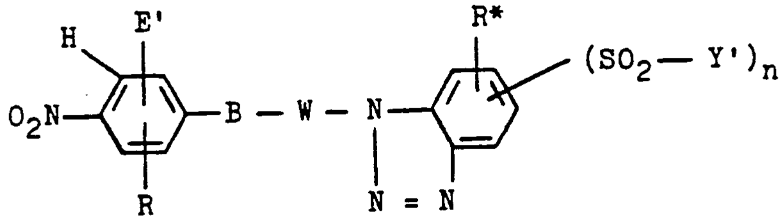

(8)

in welcher Y' die Vinylgruppe, die β-Hydroxyethyl-Gruppe oder eine Ethylgruppe, die in β-Stellung einen durch ein Alkali eliminierten Substituenten enthält, bedeutet, R, B, R* und n die in Anspruch 1 genannten Bedeutungen haben, W ein bivalenter, gegebenenfalls substituierter aliphatischer, gegebenenfalls durch Alkyl substituierter ($C_5$—$C_{10}$)-cycloaliphatischer, gegebenenfalls durch Alkyl substituierter

aliphatisch—($C_5$—$C_8$)-cycloaliphatischer, gegebenenfalls substituierter araliphatischer oder gegebenenfalls substituierter aromatisch-carbocyclischer Rest ist, wobei die aliphatischen Reste in W durch Heterogruppen, bevorzugt 1 oder 2 Heterogruppen, unterbrochen sein können, die aus den Gruppen —O—, —S—, —$SO_2$—, —CO—, 1,4-Piperidino, —NH— und —N(R°)—, worin R° eine Alkylgruppe von 1 bis 6 C-Atomen, die substituiert sein kann, oder eine Alkanoylgruppe von 2 bis 5 C-Atomen ist, ausgewählt sind, und/oder wobei aliphatische und Arylreste durch eine solche Heterogruppe miteinander verbunden sein können, sowie E' ein Wasserstoffatom, eine Sulfo- oder Carboxygruppe oder eine Gruppe der oben definierten Formel —$SO_2$—Y' oder eine gegebenenfalls substituierte Sulfonamidgruppe ist, wobei die Gruppe E' bevorzugt in ortho-Stellung zum Rest B gebunden steht, dadurch gekennzeichnet, daß man eine Verbindung der allgemeinen Formel (6)

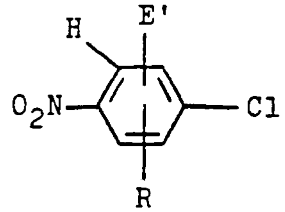

(6)

in welcher R und E' die oben angegebenen Bedeutungen haben, mit einer Aminoverbindung der allgemeinen Formel (7)

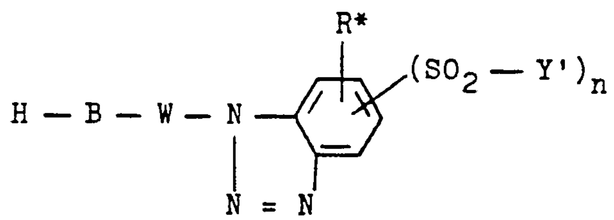

(7)

in welcher B, R*, W, Y' und n die oben genannten Bedeutungen haben, unter Zusatz eines basischen, säurebindenden Mittels bei einer Temperatur zwischen 70 und 120°C umsetzt.

17. Verwendung einer Verbindung der allgemeinen Formel (4) von Anspruch 15 oder einer Verbindung der allgemeinen Formel (8) von Anspruch 16 zur Synthese von Farbstoffen, insbesondere von Triphendioxazin-Verbindungen der allgemeinen Formel (1) gemäß Anspruch 1.

**Revendications pour les Etats contractants: FR BE GB IT LI CH**

1. Composé triphénodioxazinique soluble dans l'eau qui répond à la formule générale 1:

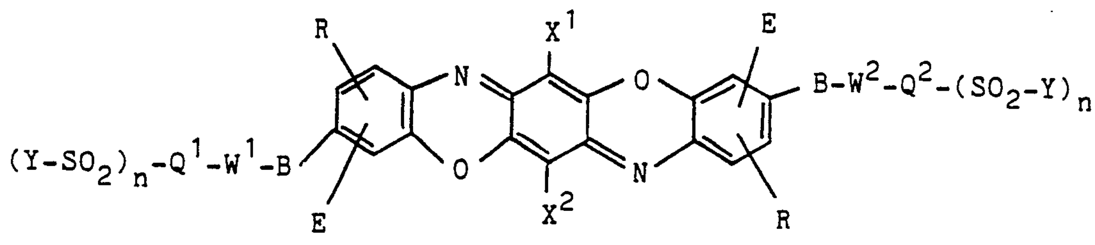

(1)

dans laquelle

n désigne un nombre égal à 0 ou à 1, avec les conditions que, lorsque n est égal à 0, le radical —$SO_2$—Y represénte un atome d'hydrogène et que n ne puisse être nul que lorsque E représente un radical —$SO_2$—Y dont le symbole Y désigne un radical sulfato-2 éthyle,

Y représente un radical vinyle ou représente un radical éthyle portant, à sa position 2, un substituant éliminable sous l'action d'un composé alcalin,

$Q^1$ représente un radical benzotriazolique répondant à la formule générale 2a représentée ci-dessous,

$Q^2$ représente un radical benzotriazolique répondant à la formule générale 2b représentée ci-dessous:

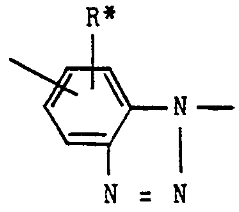

(2a)

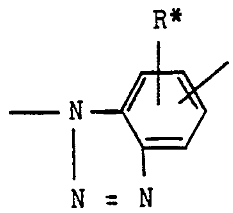

(2b)

dans lesquelles

R* représente un atome d'hydrogène, un alkyle contenant de 1 à 4 atomes de carbone, un alcoxy contenant de 1 à 4 atomes de carbone, un atome d'halogène ou un radical carboxy ou sulfo, et

la liaison libre partant du radical benzénique assure la liaison avec le groupement $(Y—SO_2)_n—$,

B représente un atome d'oxygène ou de soufre, un radical amino de formule —N(R')— dont le symbole R' représente un alkyle en $C_1—C_6$, éventuellement substitué, ou, mieux, le radical —NH—,

$W^1$ représente un radical bivalent qui est un radical aliphatique éventuellement substitué, un radical cycloaliphatique en $C_5—C_{10}$ éventuellement porteur d'un alkyle, un radical aliphatique $—(C_5—C_8)$-cycloaliphatique éventuellement porteur d'un alkyle, un radical araliphatique éventuellement substitué ou un radical aromatique-carbocyclique éventuellement substitué, les radicaux aliphatiques dans $W^1$ pouvant être interrompus par des hétéroradicaux pris dans l'ensemble constitué par les radicaux —O—, —S—, $—SO_2—$, —CO—, pipéridino-1,4, —NH— et —N(R°)—, le symbole R° désignant un alkyle en $C_1—C_6$ éventuellement substitué ou un alcanoyle en $C_2—C_5$, et/ou des radicaux aliphatiques et aryles pouvant être liés entre eux par un tel hétéroradical,

$W^2$ a l'une des significations qui ont été indiquées pour $W^1$, et est identique à $W^1$ ou différent, de $W^1$,

R représente un atome d'hydrogène, un alkyle contenant de 1 à 6 atomes de carbone, un alcoxy contenant de 1 à 5 atomes de carbone, un atome d'halogène ou un radical carboxy ou sulfo, de préférence un atome d'hydrogène,

E représente un atome d'hydrogène, un radical sulfo ou carboxy, un radical de formule générale $—SO_2—Y$ dans lequel Y a la signification qui lui a été donné ci-dessus, ou un radical sulfamoyle éventuellement substitué,

$X^1$ représente un atome d'hydrogène, un atome d'halogène, un cycloalkyle contenant de 5 à 8 atomes de carbone, un aralkyloxy, un alcoxy contenant de 1 à 4 atomes de carbone, un aryloxy, un alkyle contenant de 1 à 4 atomes de carbone, un aryle, un aralkyle, un cyano, un carboxy, un alcoxycarbonyle contenant de 2 à 5 atomes de carbone, un arylamino, un carbamoyle, un N-alkylcarbamoyle ou un N,N-dialkylcarbamoyle dont l'alkyle ou chacun des alkyles contient de 1 à 4 atomes de carbone, un N-aryl-carbamoyle, un alcanoylamino contenant de 2 à 5 atomes de carbone ou un aroylamino, les radicaux aryles contenus dans les substituants cités étant de préférence des radicaux phényles éventuellement porteurs d'un ou de deux substituants pris dans l'ensemble constitué par les halogènes, le nitro, les alkyles en $C_1—C_4$, les alcoxy en $C_1—C_4$, le carboxy et le sulfo, le symbole $X^1$ représentant de préférence un atome de brome ou de chlore,

$X^2$ est identique à $X^1$ ou différent de $X^1$ et a une des significations qui ont été données pour $X^1$,

le radical E est de préférence en position ortho par rapport au radical $—B—W^1—Q^1—(SO_2—Y)_n$ ou au radical $—B—W^2—Q^2—(SO_2—Y)_n$,

la molécule (1) contenant obligatoirement au moins un des radicaux carboxy, sulfo et sulfato qui peuvent être présents dans cette molécule.

2. Composé selon la revendication 1, caractérisé en ce que E représente un radical sulfo.

3. Composé selon l'une des revendications 1 et 2, caractérisé en ce que $W^1$ ou $W^2$ représente un radical phénylène-1,3, phénylène-1,4, β-(p-phénylène)-éthylène, (sulfo-4 phényl)-2 propylène-1,3 ou sulfato-2 propylène-1,3 ou un radical répondant à l'une des formules:

$$—CH_2—CH_2—O—CH_2—CH_2—,$$

$$—CH_2—CH_2—NH—CH_2—CH_2— \text{ et}$$

$$—CH_2—CH_2—N(CH_3)—CH_2—CH_2—.$$

4. Composé selon l'une des revendications 1 et 2, caractérisé en ce que $W^1$ représente un radical de formule:

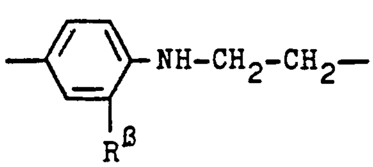

ou

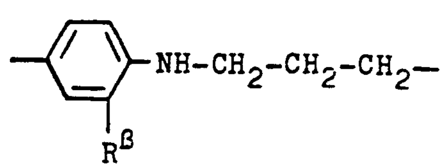

et $W^2$ un radical de formule

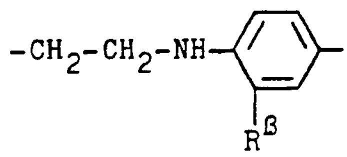

ou

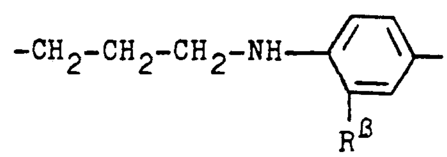

dans lesquelles $R^{\beta}$ représente un atome d'hydrogène ou un radical sulfo.

5. Composé selon l'une des revendications 1 et 2, caractérisé en ce que $W^1$ ou $W^2$ représente un radical éthylène-1,2, propylène-1,3, butylène-1,4 ou isopropylène.

6. Composé selon au moins une des revendications 1, 2, 4, 5 et 7, caractérisé en ce que E représente un radical sulfato-2 éthylsulfonyle, N,N-bis-(sulfato-2 éthyl)-sulfamoyle, N-(sulfato-2 éthyl)-sulfamoyle, N-(sulfo-2 éthyl)-sulfamoyle ou N-méthyl-N-(sulfo-2 éthyl)-sulfamoyle.

7. Composé selon au moins une des revendications 1 à 6 caractérisé en ce que $X^1$ et $X^2$ représentent chacun un atome de chlore.

8. Composé selon la revendication 1 qui répond à la formule générale la:

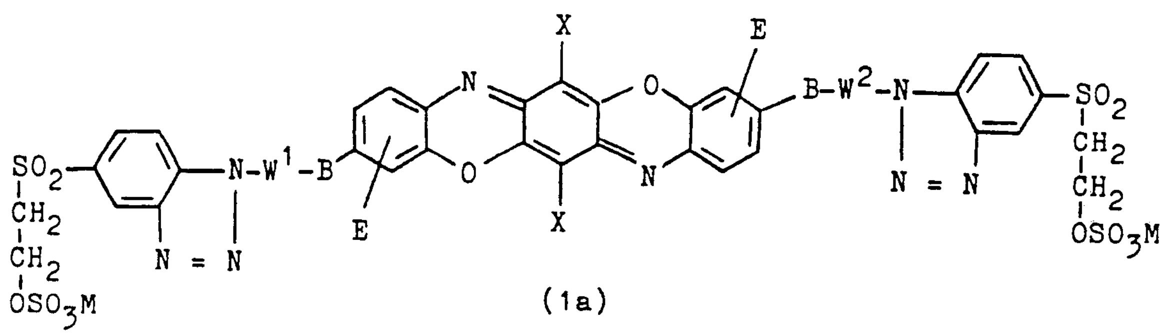

(1a)

dans laquelle

X représente un atome de brome ou de chlore,

M représente un atome d'hydrogène ou un métal alcalin,

E représente un radical sulfo ou carboxy et

$W^1$ et $W^2$ représentent chacun un radical éthylène-1,2 ou propylène-1,3.

9. Composé selon la revendication 8 caractérisé en ce que E représente un radical sulfo et X un atome de chlore.

10. Composé selon la revendication 1 qui répond à la formule générale 1b:

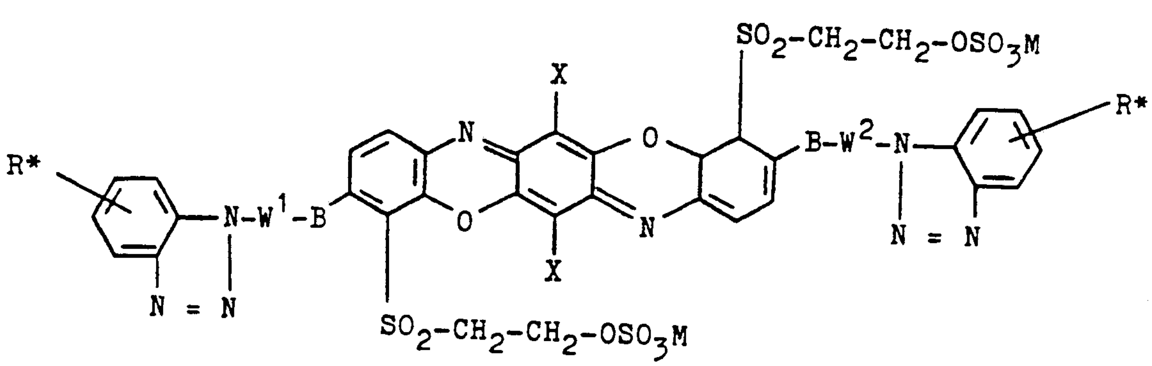

(1b)

dans laquelle

X représente un atome de brome ou de chlore,

M représente un atome d'hydrogène ou un métal alcalin,

B représente un radical —NH—,

$W^1$ et $W^2$ représentent chacun un radical éthylène-1,2, propylène-1,3 ou isopropylène, ou $W^1$ représente un radical répondant à l'une des formules suivantes:

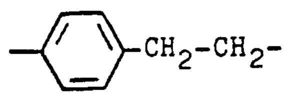

oder

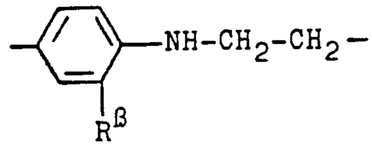

et

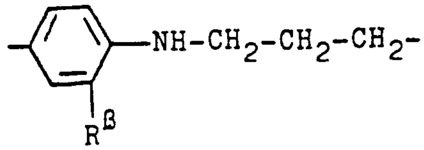

et $W^2$ un radical répondant à l'une des formules suivantes:

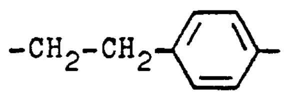

oder

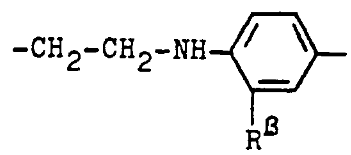

et

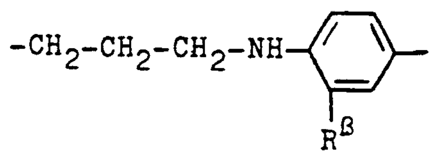

dans lesquelles $R^{\beta}$ représente un atome d'hydrogène ou un radical sulfo et les R* représentent chacun un radical sulfo.

11. Composé selon la revendication 1 qui répond à la formule générale 1c:

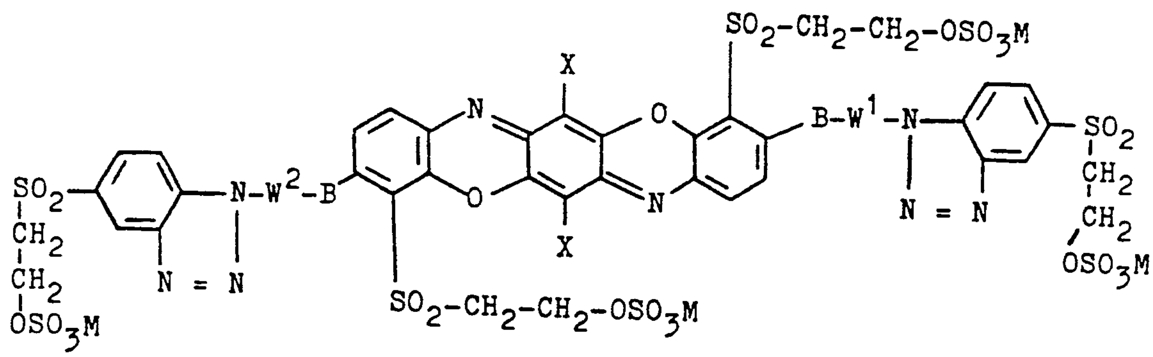

(1c)

dans laquelle

X représente un atome de brome ou de chlore,

M représente un atome d'hydrogène ou un métal alcalin,

B représente un radical —NH— et

$W^1$ et $W^2$ représentent chacun un radical bivalent qui contient un radical sulfo ou un radical sulfato.

12. Composé selon l'une des revendications 10 et 11 ou selon les deux, caractérisé en ce que les deux symboles X représentent un atome de chlore.

13. Procédé pour préparer les composés qui ont été mentionnées et définis à la revendication 1, procédé caractérisé en ce que l'on cyclise un composé répondant à la formule générale 3:

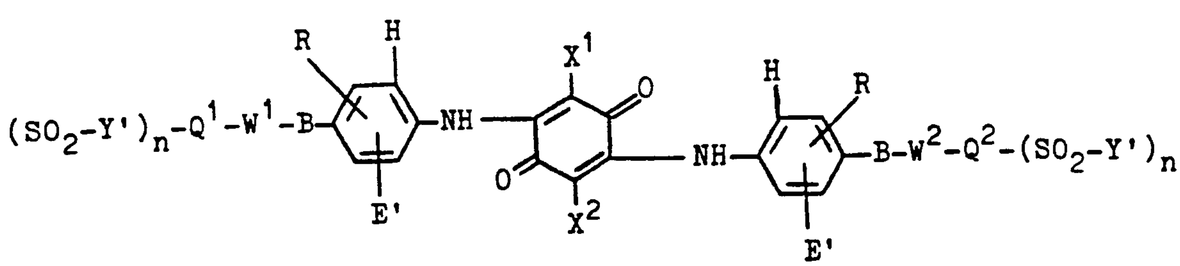

(3)

(dans laquelle Y' représente un radical vinyle, un radical hydroxy-2 éthyle ou un radical éthyle portant, en sa position 2, un substituant éliminable sous l'action d'un composé alcalin, n, R, B, $Q^1$, $Q^2$, $W^1$, $W^2$, $X^1$ et $X^2$ ont les significations qui leur ont été données à la revendication 1, et E' a l'une des significations qui ont été données pour E à la revendication 1 ou représente un radical hydroxy-2 éthylsulfonyle, et dans laquelle les radicaux alkyles substitués présents dans ces radicaux peuvent également être des radicaux alkyles hydroxylés, les radicaux E' sont de préférence en position ortho par rapport au radical $—B—W^2—Q^2—(SO_2—Y')_n$ ou $(Y'—SO_2)_n—Q^1—W^1—B—$, et les noyaux benzéniques ne peuvent pas être substitués à l'une des positions ortho par rapport au radical amino —NH— représenté), en milieu acide et, de préférence, en présence d'un agent d'oxydation, cyclisation qui conduit au composé triphénodioxazinique, et éventuellement, avant ou pendant la cyclisation, ou seulement après la réaction de cyclisation, on transforme d'éventuels radicaux hydroxyalkyles, par estérification au moyen d'un agent de sulfatation ou de phosphatation, en les radicaux sulfato-2 alkyles ou phosphato-2 alkyles correspondants, et, le cas échéant, on introduit en même temps des radicaux sulfo dans des radicaux aryles.

14. Application d'un composé de formule générale 1 mentionné et défini à la revendication 1, à la teinture (y compris à l'impression) d'une matière contenant des radicaux hydroxy et/ou carbamoyles, plus particulièrement d'une matière fibreuse de ce genre.

15. Procédé pour teindre (y compris imprimer) une matière contenant des radicaux hydroxy et/ou carbamoyles, plus particulièrement une matière fibreuse de ce genre, par application ou introduction d'un colorant sur ou dans la matière, et fixage de celui-ci par la chaleur et/ou au moyen d'un accepteur d'acides, procédé caractérisée en ce que l'on utilise, comme colorant, un composé répondant à la formule générale 1 qui a été représentée et définie à la revendication 1.

16. Composé répondant à la formule générale 4:

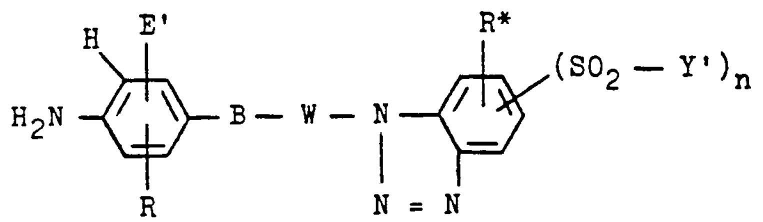

(4)

dans laquelle

Y' représente un radical vinyle, un radical hydroxy-2 éthyle ou un radical éthyle qui porte, à sa position 2, un substituant éliminable au moyen d'un composé alcalin,

R, B, R* et n ont les significations qui leur ont été données à la revendication 1,

W représente un radical bivalent qui est un radical aliphatique éventuellement substitué, un radical cycloaliphatique en $C_5—C_{10}$ éventuellement porteur d'un alkyle, un radical aliphatique $—(C_5—C_8)$-cycloaliphatique éventuellement porteur d'un alkyle, un radical araliphatique éventuellement substitué, ou un radical aromatique-carbocyclique éventuellement substitué, les radicaux aliphatiques contenus dans W pouvant être interrompus par des hétéroradicaux, de préférence par un ou deux hétéroradicaux, pris dans l'ensemble constitué par les radicaux —O—, —S—, $—SO_2—$, —CO—, pipéridino-1,4, —NH— et —N(R°)—, le symbole R° désignant un alkyle en $C_1—C_6$ éventuellement substitué ou un alcanoyle en $C_2—C_5$, et/ou des radicaux aliphatiques et aryles pouvant être unis entre eux par un tel hétéroradical, et

E' représente un atome d'hydrogène, un radical sulfo ou carboxy, un radical répondant à la formule $—SO_2—Y'$ définie ci-dessus ou un radical sulfamoyle éventuellement substitué, le radical E' étant de préférence en position ortho par rapport au radical B.

17. Composé répondant à la formule générale 8:

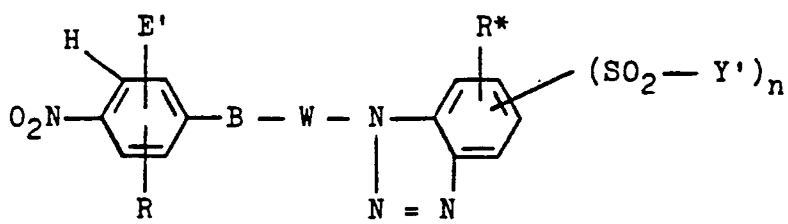

(8)

dans laquelle

Y' représente un radical vinyle, un radical hydroxy-2 éthyle ou un radical éthyle qui porte, à sa position 2, un substituant éliminable au moyen d'un composé alcalin,

R, B, R* et n ont les significations qui leur ont été données à la revendication 1,

W représente un radical bivalent qui est un radical aliphatique éventuellement substitué, un radical cycloaliphatique en $C_5$—$C_{10}$ éventuellement porteur d'un alkyle, un radical aliphatique —($C_5$—$C_8$)-cycloaliphatique éventuellement porteur d'un alkyle, un radical araliphatique éventuellement substitué, ou un radical aromatique-carbocyclique éventuellement substitué, les radicaux aliphatiques contenus dans W pouvant être interrompus par des hétéroradicaux, de préférence par un ou deux hétéroradicaux, pris dans l'ensemble constitué par les radicaux —O—, —S—, —$SO_2$—, —CO—, pipéridino-1,4, —NH— et —N(R°)—, le symbole R° désignant un alkyle en $C_1$—$C_6$ éventuellement substitué ou un alcanoyle en $C_2$—$C_5$, et/ou des radicaux aliphatiques et aryles pouvant être unis entre eux par un tel hétéroradical, et

E' représente un atome d'hydrogène, un radical sulfo ou carboxy, un radical répondant à la formule —$SO_2$—Y' définie ci-dessus ou un radical sulfamoyle éventuellement substitué, le radical E' étant de préférence en position ortho par rapport au radical B.

18. Procédé pour préparer un composé de formule générale 4 tel que défini à la revendication 16, ou un composé de formule générale 8 tel que défini à la revendication 17, procédé caractérisé en ce que l'on fait réagir un composé répondant à la formule générale 6:

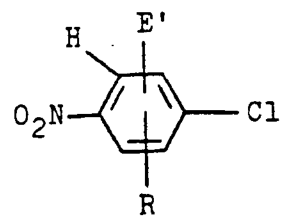

(6)

dans laquelle R et E' ont les significations qui leur ont été données à la revendication 16, avec un composé aminé répondant à la formule générale (7):

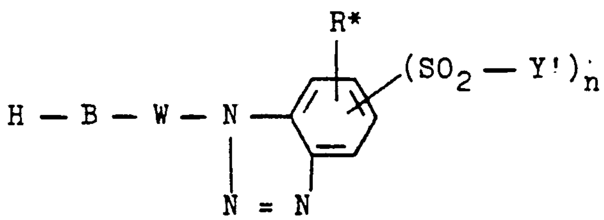

(7)

dans laquelle B, R*, W, Y' et n ont les significations qui leur ont été données à la revendication 16, en présence d'un composé basique jouant le rôle d'accepteur d'acides, à une température comprise entre 70 et 120°C, et, dans le composé nitré de formule générale 8 que l'on obtient ainsi, on réduit éventuellement le radical nitro en un radical amino.

19. Application d'un composé de formule générale 4 selon la revendication 16, ou d'un composé de formule générale 8 selon la revendication 17, à la synthèse de colorants, plus particulièrement de composés triphénodioxaziniques de formule générale 1 selon la revendication 1.

**Revendications pour l'Etat contractant: ES**

1. Procédé pour préparer un composé triphénodioxazinique hydrosoluble répondant à la formule générale 1:

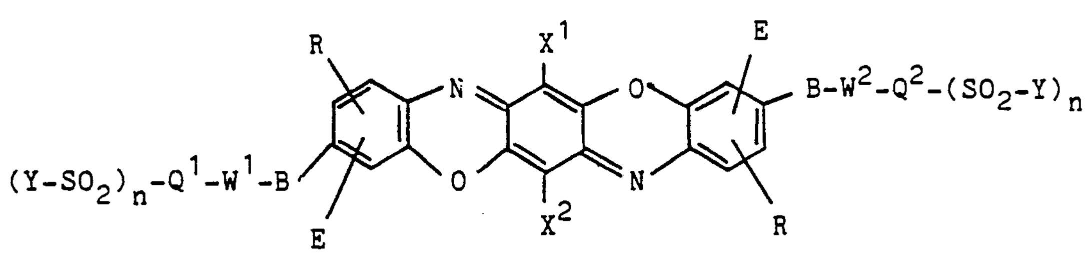

(1)

dans laquelle

n désigne un nombre égal à 0 ou à 1, avec les conditions que, lorsque n est égal à 0, le radical —$SO_2$—Y représente un atome d'hydrogène et que n ne puisse être nul que lorsque E représente un radical —$SO_2$—Y

dont le symbole Y désigne un radical sulfato-2 éthyle,

Y représente un radical vinyle ou représente un radical éthyle portant, à sa position 2, un substituant éliminable sous l'action d'un composé alcalin,

$Q^1$ représente un radical benzotriazolique répondant à la formule générale 2a représentée ci-dessous,

$Q^2$ représente un radical benzotriazolique répondant à la formule générale 2b représentée ci-dessous:

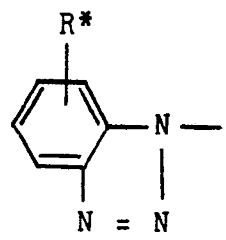
(2a)

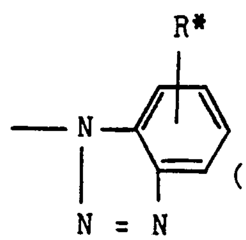
(2b)

dans lesquelles

R* représente un atome d'hydrogène, un alkyle contenant de 1 à 4 atomes de carbone, un alcoxy contenant de 1 à 4 atomes de carbone, un atome d'halogène ou un radical carboxy ou sulfo, et

la liaison libre partant du radical benzénique assure la liaison avec le groupement $(Y—SO_2)_n—$,

B représente un atome d'oxygène ou de soufre, un radical amino de formule —N(R')— dont le symbole R' représente un alkyle en $C_1—C_6$, éventuellement substitué, ou, mieux, le radical —NH—,

$W^1$ représente un radical bivalent qui est un radical aliphatique éventuellement substitué, un radical cycloaliphatique en $C_5—C_{10}$ éventuellement porteur d'un alkyle, un radical aliphatique $—(C_5—C_8)$-cycloaliphatique éventuellement porteur d'un alkyle, un radical araliphatique éventuellement substitué ou un radical aromatique-carbocyclique éventuellement substitué, les radicaux aliphatiques dans $W^1$ pouvant être interrompus par des hétéro-groupes pris dans l'ensemble constitué par les radicaux —O—, —S—, $—SO_2—$, —CO—, pipéridino-1,4, —NH— et —N(R°)—, le symbole R° désignant un alkyle en $C_1—C_6$ éventuellement substitué ou un alcanoyle en $C_2—C_5$, et/ou des radicaux aliphatiques et aryles pouvant être liés entre eux par un tel hétéro-groupe,

$W^2$ a l'une des significations qui ont été indiquées pour $W^1$, et est identique à $W^1$ ou différent de $W^1$,

R représente un atome d'hydrogène, un alkyle contenant de 1 à 6 atomes de carbone, un alcoxy contenant de 1 à 5 atomes de carbone, un atome d'halogène ou un radical carboxy ou sulfo, de préference un atome d'hydrogène,

E représente un atome d'hydrogène, un radical sulfo ou carboxy, un radical de formule générale $—SO_2—Y$ dans lequel Y a la signification qui lui a été donné ci-dessus, ou un radical sulfamoyle éventuellement substitué,

$X^1$ représente un atome d'hydrogène, un atome d'halogène, un cycloalkyle contenant de 5 à 8 atomes de carbone, un aralkyloxy, un alcoxy contenant de 1 à 4 atomes de carbone, un aryloxy, un alkyle contenant de 1 à 4 atomes de carbone, un aryle, un aralkyle, un cyano, un carboxy, un alcoxycarbonyle contenant de 2 à 5 atomes de carbone, un arylamino, un carbamoyle, un N-alkylcarbamoyle ou un N,N-dialkylcarbamoyle dont l'alkyle ou chacun des alkyles contient de 1 à 4 atomes de carbone, un N-aryl-carbamoyle, un alcanoylamino contenant de 2 à 5 atomes de carbone ou un aroylamino, les radicaux aryles contenus dans les substituants cités étant de préférence des radicaux phényles éventuellement porteurs d'un ou de deux substituants pris dans l'ensemble constitué par les halogènes, le nitro, les alkyles en $C_1—C_4$, les alcoxy en $C_1—C_4$, le carboxy et le sulfo, le symbole $X^1$ représentant de préference un atome de brome ou de chlore,

$X^2$ est identique à $X^1$ ou différent de $X^1$ et a une des significations qui ont été données pour $X^1$,

le radical E est de préférence en position ortho par rapport au radical $—B—W^1—Q^1—(SO_2—Y)_n$ ou au radical $—B—W^2—Q^2—(SO_2—Y)_n$,

la molécule (1) contenant obligatoirement au moins un des radicaux carboxy, sulfo et sulfato qui peuvent être présents dans cette molécule, procédé caractérisé en ce que l'on cyclise un composé répondant à la formule générale 3:

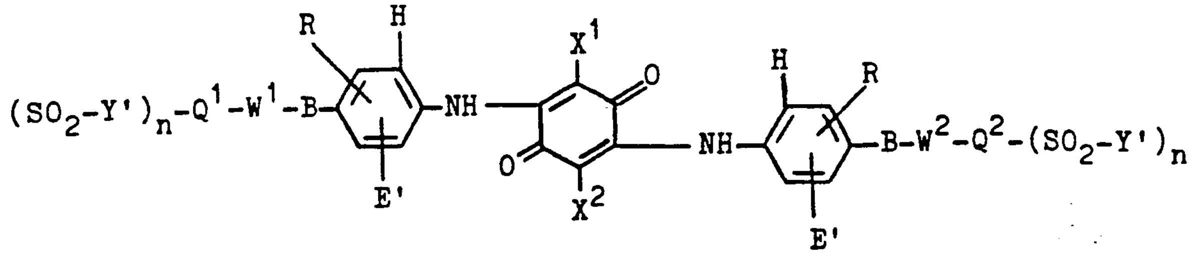

(3)

(dans laquelle Y' représente un radical vinyle, un radical hydroxy-2 éthyle ou un radical éthyle portant, en sa position 2, un substituant éliminable sous l'action d'un composé alcalin, n, R, B, $Q^1$, $Q^2$, $W^1$, $W^2$, $X^1$ et $X^2$ ont les significations qui leur ont été données ci-dessus et E' a l'une des significations qui ont été données ci-dessus pour E ou représente un radical hydroxy-2 éthylsulfonyle, et dans laquelle les radicaux alkyles substitués présents dans ces radicaux peuvent également être des radicaux alkyles hydroxylés, les radicaux E' sont de préférence en position ortho par rapport au radical $—B—W^2—Q^2—(SO_2—Y')_n$ ou $(Y'—SO_2)_n—Q^1—W^1—B—$, et les noyaux benzéniques ne peuvent pas être substitués à l'une des positions ortho par rapport au radical amino —NH— représenté), en milieu acide et, de préférence, en présence d'un agent d'oxydation, cyclisation qui conduit au composé triphénodioxazinique, et éventuellement, avec ou pendant la cyclisation, ou seulement après la réaction de cyclisation, on transforme d'éventuels radicaux hydroxyalkyles, par estérification au moyen d'un agent de sulfatation ou de phosphatation, en les radicaux sulfato-2 alkyles ou phosphato-2 alkyles correspondants, et, le cas échéant, on introduit en même temps des radicaux sulfo dans des radicaux aryles.

2. Procédé selon l'une des revendications 1 et 2, caractérisé en ce que E représente un radical sulfo.

3. Procédé selon l'une des revendications 1 et 2, caractérisé en ce que $W^1$ ou $W^2$ représente un radical phénylène-1,3, phénylène-1,4, β-(p-phénylène)-éthylène, (sulfo-4 phényl)-2 propylène-1,3 ou sulfato-2 propylène-1,3 ou un radical répondant à l'une des formules:

$$—CH_2—CH_2—O—CH_2—CH_2—,$$

$$—CH_2—CH_2—NH—CH_2—CH_2— \text{ et}$$

$$—CH_2—CH_2—N(CH_3)—CH_2—CH_2—.$$

4. Procédé selon l'une des revendications 1 et 2, caractérisé en ce que $W^1$ représente un radical de formule:

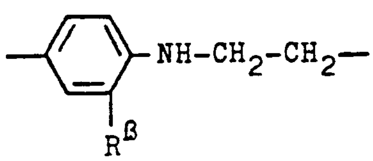

ou

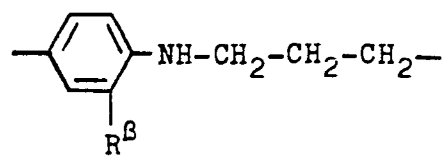

et $W^2$ un radical de formule

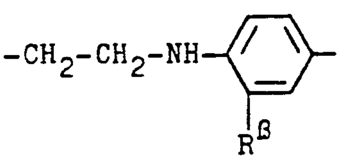

ou

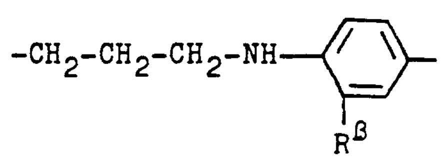

dans lesquelles $R^β$ représente un atome d'hydrogène ou un radical sulfo.

5. Procédé selon l'une des revendications 1 et 2, caractérisé en ce que $W^1$ ou $W^2$ représente un radical éthylène-1,2, propylène-1,3, butylène-1,4 ou isopropylène.

6. Procédé selon au moins une des revendications 1, 2, 4, 5 et 7, caractérisé en ce que E représente un radical sulfato-2 éthylsulfonyle, N,N-bis-(sulfato-2 éthyl)-sulfamoyle, N-(sulfato-2 éthyl)-sulfamoyle, N-(sulfo-2 éthyl)-sulfamoyle ou N-méthyl-N-(sulfo-2 éthyl)-sulfamoyle.

7. Procédé selon au moins une des revendications 1 à 6, caractérisé en ce que $X^1$ et $X^2$ représentent chacun un atome de chlore.

8. Procédé selon la revendication 1, caractérisé en ce que le composé de formule 1 obtenu est un composé répond à la formule générale Ia:

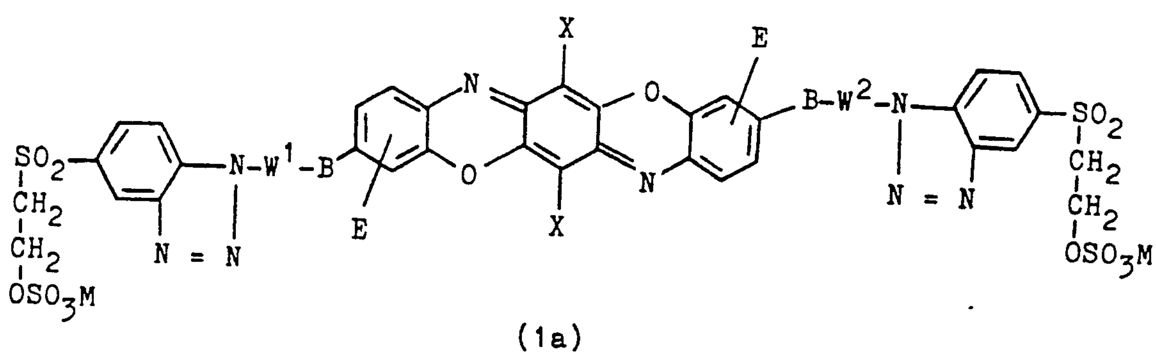

(1a)

dans laquelle

X représente un atome de brome ou de chlore,

M représente un atome d'hydrogène ou un métal alcalin,

E représente un radical sulfo ou carboxy et

$W^1$ et $W^2$ représentent chacun un radical éthylène-1,2 ou propylène-1,3.

9. Procédé selon la revendication 8 caractérisé en ce que E représente un radical sulfo et X un atome de chlore.

10. Procédé selon la revendication 1 caractérisé en ce que le composé de formule 1 obtenu est un composé répondant à la formule générale 1b:

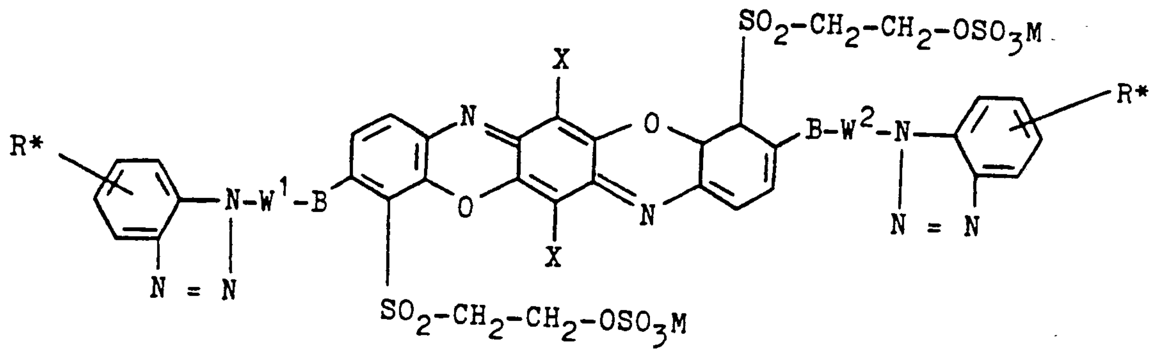

(1b)

dans laquelle

X représente un atome de brome ou de chlore,

M représente un atome d'hydrogène ou un métal alcalin,

B représente un radical —NH—,

$W^1$ et $W^2$ représentent chacun un radical éthylène-1,2, propylène-1,3 ou isopropylène, ou $W^1$ représente un radical répondant à l'une des formules suivantes:

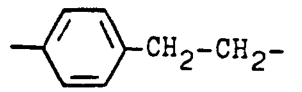 et 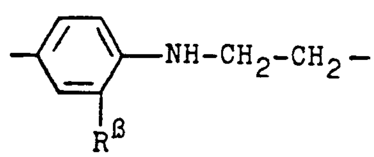

et 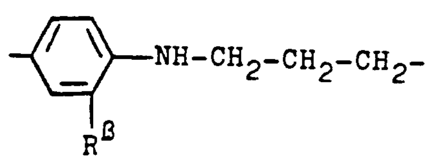

et $W^2$ un radical répondant à l'une des formules suivantes:

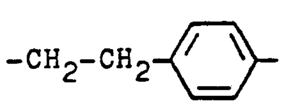 et 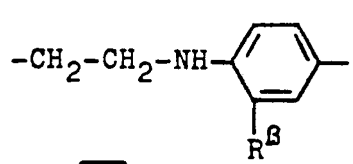

et 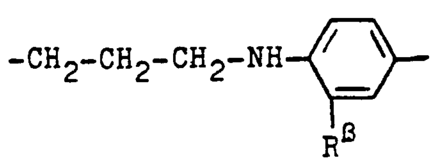

dans lesquelles $R^\beta$ représente un atome d'hydrogène ou un radical sulfo et les R* représentent chacun un radical sulfo.

11. Procédé selon la revendication 1 caractérisé en ce que le composé de formule 1 obtenu est un composé répondant à la formule générale 1c:

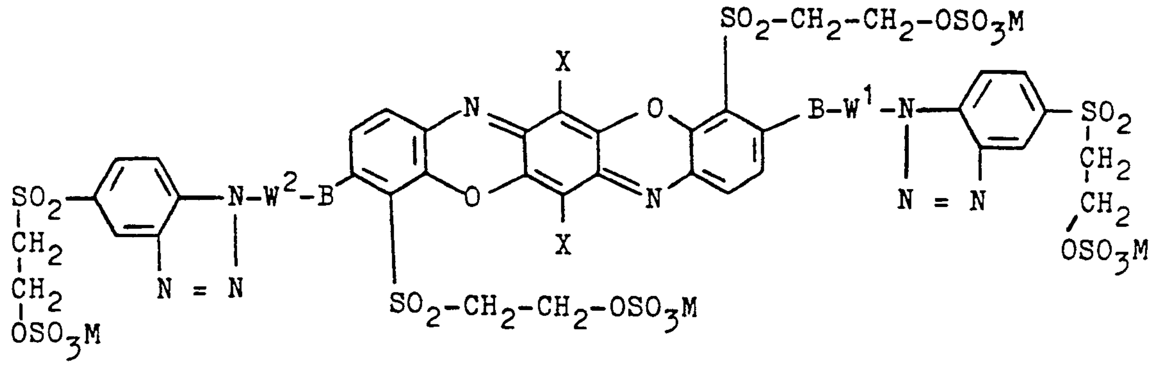

(1c)

dans laquelle

X représente un atome de brome ou de chlore,

M représente un atome d'hydrogène ou un métal alcalin,

B représente un radical —NH— et

$W^1$ et $W^2$ représentent chacun un radical bivalent qui contient un radical sulfo ou un radical sulfato.

12. Procédé selon l'une des revendications 10 et 11 ou selon les deux, caractérisé en ce que les deux symboles X représentent chacun un atome de chlore.

13. Application d'un composé de la formule générale 1 mentionné et défini à la revendication 1, ou d'un composé de formule générale 1 qui a été préparé selon la revendication 1, à la teinture (y compris à l'impression) d'une matière contenant des radicaux hydroxy et/ou carbamoyles, plus particulièrement d'une matière fibreuse de ce genre.

14. Procédé pour teindre (y compris imprimer) une matière contenant des radicaux hydroxy et/ou carbamoyles, plus particulièrement une matière fibreuse de ce genre, par application ou introduction d'un colorant sur ou dans la matière, et fixage de celui-ci par la chaleur et/ou au moyen d'un accepteur d'acides, procédé caractérisé en ce que l'on utilise, comme colorant, un composé répondant à la formule générale 1 qui a été représentée et définie à la revendication 1.

15. Procédé pour préparer un composé répondant à la formule générale 4:

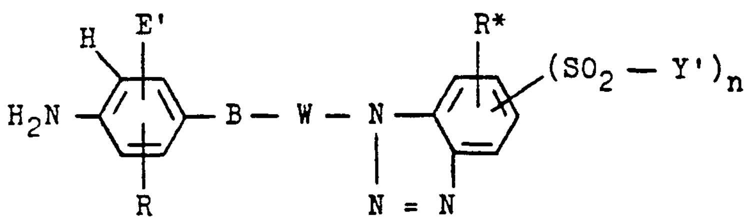

(4)

dans laquelle

Y' représente un radical vinyle, un radical hydroxy-2 éthyle ou un radical éthyle qui porte, à sa position 2, un substituant éliminable au moyen d'un composé alcalin,

R, B, R* et n ont les significations qui leur ont été données à la revendication 1,

W représente un radical bivalent qui est un radical aliphatique éventuellement substitué, un radical cycloaliphatique en $C_5$—$C_{10}$ éventuellement porteur d'un alkyle, un radical aliphatique —($C_5$—$C_8$)-cycloaliphatique éventuellement porteur d'un alkyle, un radical araliphatique éventuellement substitué, ou un radical aromatique-carbocyclique éventuellement substitué, les radicaux aliphatiques contenus dans W pouvant être interrompus par des hétéroradicaux, de préférence par un ou deux hétéroradicaux, pris dans l'ensemble constitué par les radicaux —O—, —S—, —$SO_2$—, —CO—, pipéridino-1,4, —NH— et —N($R^o$)—, le symbole $R^o$ désignant un alkyle en $C_1$—$C_6$ éventuellement substitué ou un alcanoyle en $C_2$—$C_5$, et/ou des radicaux aliphatiques et aryles pouvant être unis entre eux par un tel hétéro-radical, et

E' représente un atome d'hydrogène, un radical sulfo ou carboxy, un radical répondant à la formule —$SO_2$—Y' définie ci-dessus ou un radical sulfamoyle éventuellement substitué, le radical E' étant de préférence en position ortho par rapport au radical B, procédé caractérisé en ce que l'on fait réagir un composé répondant à la formule générale 6:

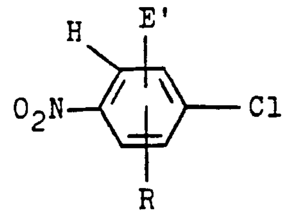

(6)

dans laquelle R et E' ont les significations qui leur ont été données ci-dessus, avec un composé aminé répondant à la formule générale (7):

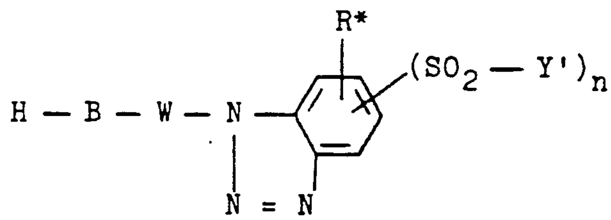

(7)

dans laquelle B, R*, W, Y' et n ont les significations qui leur ont été données plus haut, en présence d'un composé basique agissant comme accepteur d'acides, à une température comprise entre 70 et 120°C, et, dans le composé nitré obtenu, on réduit le radical nitro pour le convertir en radical amino.

16. Procédé de préparation d'un composé répondant à la formule générale 8:

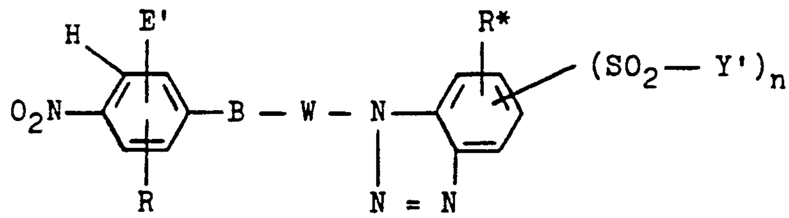

(8)

dans laquelle

Y' représente un radical vinyle, un radical hydroxy-2 éthyle ou un radical éthyle qui porte, à sa position 2, un substituant éliminable au moyen d'un composé alcalin,

R, B, R* et n ont les significations qui leur ont été données à la revendication 1,

W représente un radical bivalent qui est un radical aliphatique éventuellement substitué, un radical cycloaliphatique en $C_5$—$C_{10}$ éventuellement porteur d'un alkyle, un radical aliphatique —($C_5$—$C_8$)-cycloaliphatique éventuellement porteur d'un alkyle, un radical araliphatique éventuellement substitué, ou un radical aromatique-carbocyclique éventuellement substitué, les radicaux aliphatiques contenus dans W pouvant être interrompus par des hétéroradicaux, de préférence par un ou deux hétéroradicaux, pris dans l'ensemble constitué par les radicaux —O—, —S—, —$SO_2$—, —CO—, pipéridino-1,4, —NH— et —N(R°)—, le symbole R° désignant un alkyle en $C_1$—$C_6$ éventuellement substitué ou un alcanoyle en $C_2$—$C_5$, et/ou des radicaux aliphatiques et aryles pouvant être unis entre eux par un tel hétéroradical, et

E' représente un atome d'hydrogène, un radical sulfo ou carboxy, un radical répondant à la formule —$SO_2$—Y' définie ci-dessus ou un radical sulfamoyle éventuellement substitué, le radical E' étant de préférence en position ortho par rapport au radical B, procédé caractérisé en ce que l'on fait réagir un composé répondant à la formule générale 6:

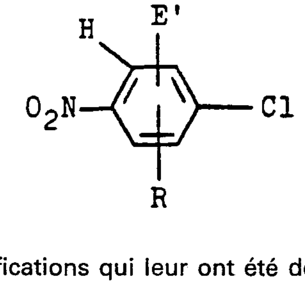

(6)

dans laquelle R et E' ont les significations qui leur ont été données ci-dessus, avec un composé aminé répondant à la formule générale 7:

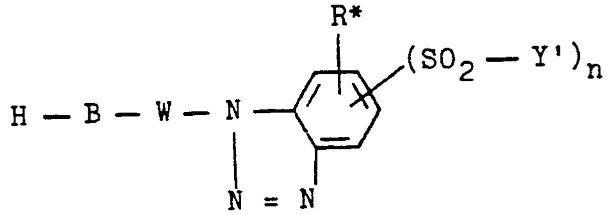

(7)

dans laquelle B, R*, W, Y' et n ont les significations qui leur ont été données ci-dessus, en présence d'un composé basique agissant comme accepteur d'acides, à une température comprise entre 70 et 120°C.

17. Application d'un composé de formule générale 4 selon la revendication 15, ou d'un composé de formule générale 8 selon la revendication 16, à la synthèse de colorants, plus particulièrement de composés triphénodioxaziniques de formule générale 1 selon la revendication 1.

**Claims for the Contracting States: BE CH DE FR GB IT LI**

1. A water-soluble triphendioxazine compound corresponding to the general formula (1)

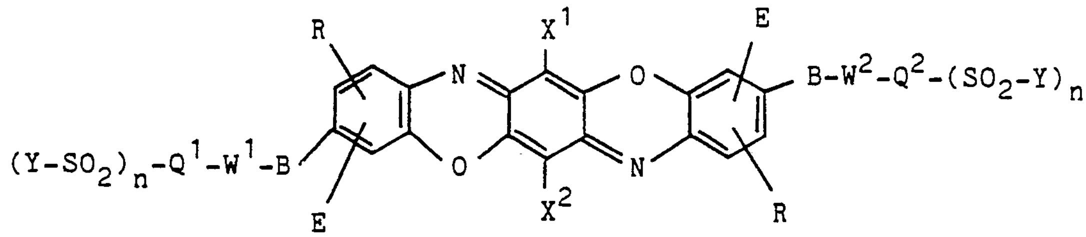

(1)

in which:

n is the number 0 or 1, the group $—SO_2—Y$ representing a hydrogen atom if n = 0, and n only being zero if E is a group $—SO_2—Y$ in which Y is β-sulfatoethyl;

Y is the vinyl group or an ethyl group containing, in the β-position, a substituent which can be eliminated by means of an alkali, and is preferably β-sulfatoethyl;

$Q^1$ is a benzotriazole radical of the general formula (2a) indicated below and

$Q^2$ is a benzotriazole radical of the general formula (2b) indicated below

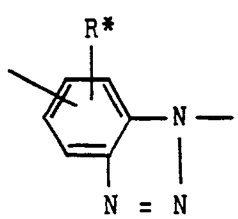

(2a)

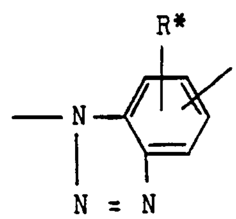

(2b)

in which

R* denotes a hydrogen atom, an alkyl group having 1 to 4 carbon atoms, an alkoxy group having 1 to 4 carbon atoms, a halogen atom or a carboxyl or sulfo group and the free bond on the benzene radical characterizes the bond leading to the grouping $(Y—SO_2)_n—$,

B is an oxygen or sulfur atom or an amino group of the formula —N(R')— in which R' is an alkyl group which has 1 to 6 carbon atoms and can be substituted, or is preferably the group —NH—;

$W^1$ is a divalent, optionally substituted aliphatic radical, $(C_5—C_{10})$-cycloaliphatic radical which is optionally substituted by alkyl, aliphatic-$(C_5—C_8)$-cycloaliphatic radical which is optionally substituted by alkyl, optionally substituted araliphatic radical or optionally substituted aromatic-carbocyclic radical, it being possible for the aliphatic radicals in $W^1$ to be interrupted by hetero groups selected from the groups —O—, —S—, $—SO_2—$, —CO—, 1,4-piperidino, —NH— and —N(R°)—, wherein R° is an alkyl group which has 1 to 6 carbon atoms and can be substituted or is an alkanoyl group having 2 to 5 carbon atoms, and/or it being possible for aliphatic and aryl radicals to be attached to one another through such a hetero group;

$W^2$ has a meaning which is indicated for $W^1$ and is identical with $W^1$ or different from $W^1$;

R is a hydrogen atom or an alkyl group having 1 to 6 carbon atoms, an alkoxy group having 1 to 5

carbon atoms, a halogen atom or a carboxy or sulfo group, preferably a hydrogen atom;

E is a hydrogen atom or a sulfo or carboxyl group or a group of general formula $—SO_2—Y$ in which Y has the meaning indicated above or is an optionally substituted sulfonamide group;

$X^1$ is a hydrogen atom or a halogen atom, a cycloalkyl group having 5 to 8 carbon atoms, an aralkyloxy group, an alkoxy group having 1 to 4 carbon atoms, an aryloxy group, an alkyl group having 1 to 4 carbon atoms, an aryl group, an aralkyl group, a cyano group, a carboxyl group, a carboalkoxy group having 2 to 5 carbon atoms, an arylamino group, a carbamoyl group, an N-alkylcarbamoyl group or N,N-dialkylcarbamoyl group in which the alkyl radicals each have 1 to 4 carbon atoms, an N-arylcarbamoyl group, an alkanoylamino group having 2 to 5 carbon atoms or an aroylamino group, the aryl radicals in these named substituents being preferably phenyl radicals which can also be substituted by 1 or 2 substituents belonging to the group comprising halogen, nitro, alkyl having 1 to 4 carbon atoms, alkoxy having 1 to 4 carbon atoms, carboxyl and sulfo, $X^1$ preferably being a bromine or chlorine atom;

$X^2$ is identical with $X^1$ or different from $X^1$ and has one of the meanings indicated for $X^1$;

the group E is preferably attached in the ortho-position to the group $—B—W^1—Q^1—(SO_2—Y)_n$ or $—B—W^2—Q^2—(SO_2—Y)_n$; and

the molecule (1) must contain at least one of the carboxyl, sulfo and sulfato groups which can be present in the molecule (1).

2. A compound as claimed in claim 1, wherein E is a sulfo group.

3. A compound as claimed in claim 1 or 2, wherein $W^1$ and/or $W^2$ is a 1,3-phenylene, 1,4-phenylene or β-(p-phenylene)-ethylene group or a 2-(4'-sulfophenyl)-1,3-propylene or 2-sulfato-1,3-propylene group or a group of the formula $—CH_2—CH_2—O—CH_2—CH_2—$, $—CH_2—CH_2—NH—CH_2—CH_2—$ or $—CH_2—CH_2—N(CH_3)—CH_2—CH_2—$.

4. A compound as claimed in claim 1 or 2, wherein $W^1$ denotes a group of formula

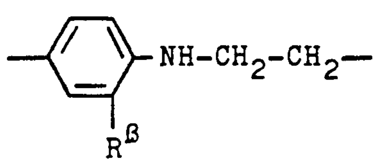

or

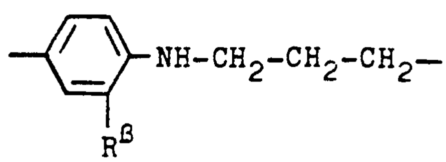

and $W^2$ is a group of formula

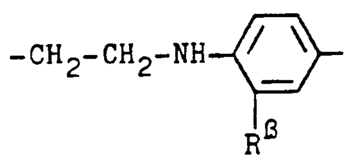

or

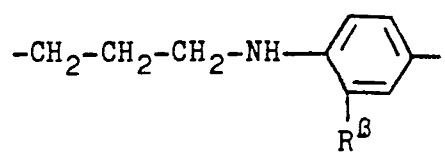

in which $R^β$ is a hydrogen atom or a sulfo group.

5. A compound as claimed in claim 1 or 2, wherein $W^1$ and/or $W^2$ is a 1,2-ethylene, 1,3-propylene, 1,4-butylene or isopropylene group.

6. A compound as claimed in at least one of claims 1, 2 or 4 or 5, wherein E is a β-sulfatoethylsulfonyl, N,N-di(β-sulfatoethyl)-sulfamoyl, N-(β-sulfatoethyl)-sulfamoyl, N-(β-sulfoethyl)-sulfamoyl or N-methyl-N-(β-sulfoethyl)-sulfamoyl group.

7. A compound as claimed in at least one of claims 1 to 6, wherein $X^1$ and $X^2$ both represent a chlorine atom.

8. A compound as claimed in claim 1, corresponding to the general formula (1a)

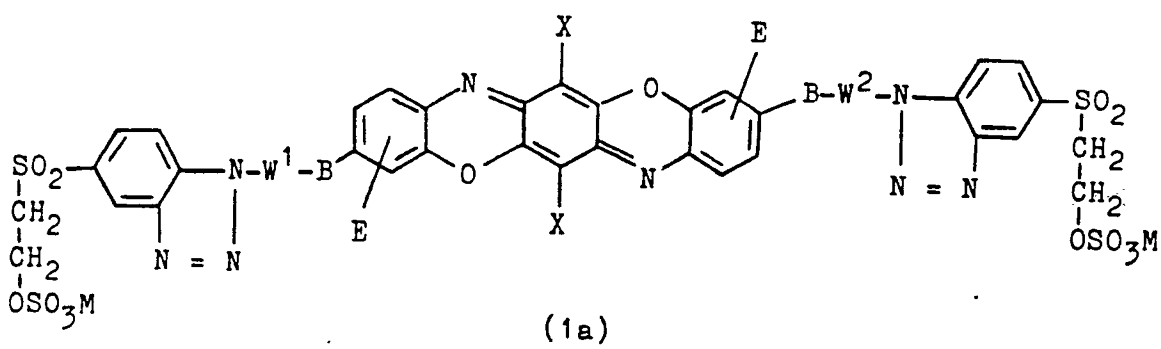

(1a)

in which

X represents a bromine or chlorine atom,

M is a hydrogen atom or an alkali metal,

E denotes a sulfo or carboxyl group and
$W^1$ and $W^2$ both denote a 1,2-ethylene or 1,3-propylene group.
9. A compound as claimed in claim 8, wherein E is a sulfo group and X is a chlorine atom.
10. A compound as claimed in claim 1, corresponding to the general formula (1b)

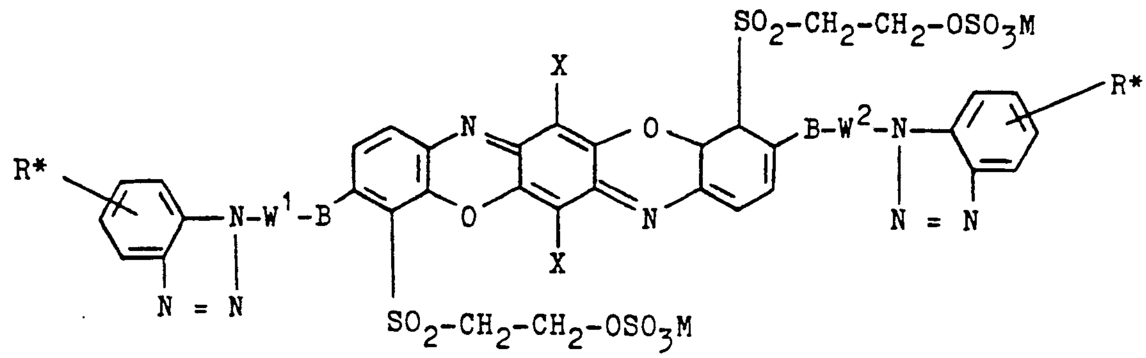

(1b)

in which
X represents a bromine atom or a chlorine atom,
M is a hydrogen atom or an alkali metal,
B denotes the group —NH— and
$W^1$ and $W^2$ both represent the 1,2-ethylene or 1,3-propylene group or an isopropylene group or $W^1$ denotes a group of formula

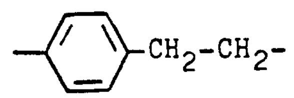 or 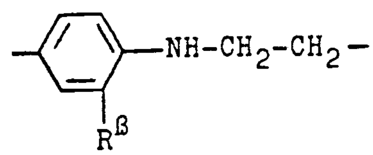

or 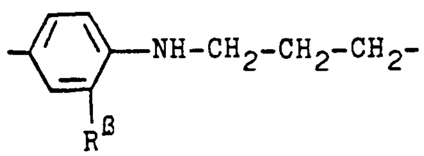

and $W^2$ denotes a group of formula

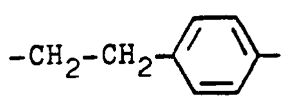 or 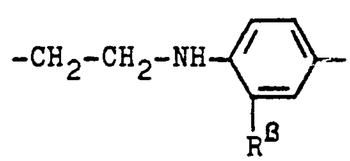

or 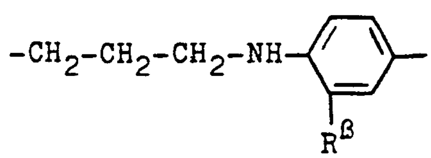

in which $R^ß$ represents a hydrogen atom or a sulfo group, and each R* denotes a sulfo group.

11. A compound as claimed in claim 1, corresponding to the general formula (1c)

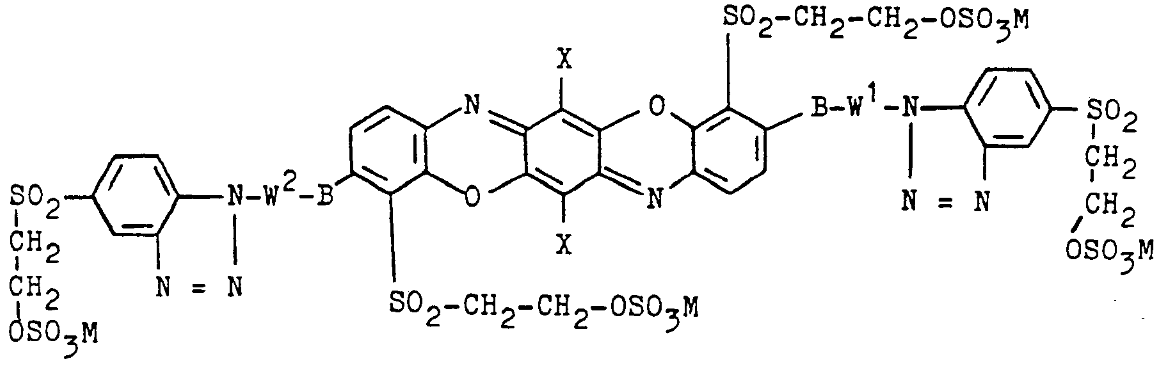

(1c)

in which

X represents a bromine or chlorine atom,

M is a hydrogen atom or an alkali metal,

B denotes the group —NH— and

$W^1$ and $W^2$ both denote a divalent radical which contains sulfo groups or sulfato groups.

12. A compound as claimed in claim 10 or 11 or both, wherein both Xs represent a chlorine atom.

13. A process for the preparation of the compounds mentioned and defined in claim 1, which comprises cyclizing a compound of the general formula (3)

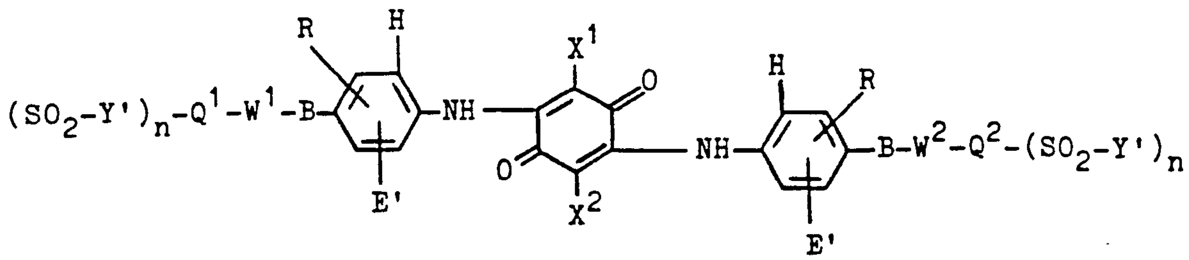

(3)

(in which Y′ is the vinyl group, the β-hydroxyethyl group or an ethyl group containing, in the β-position, a substituent which can be eliminated by means of an alkali, and n, R, B, $Q^1$, $Q^2$, $W^1$, $W^2$, $X^1$ and $X^2$ have the meanings mentioned in claim 1 and E′ has one of the meanings mentioned in E in claim 1 or is a β-hydroxyethylsulfonyl group, and substituted alkyl groups in these radicals can also be hydroxy-substituted alkyl groups, the groups E′ are preferably attached in the ortho-position relative to the group $—B—W^2—Q^2—(SO_2—Y')_n$ or $(SO_2—Y')_n—Q^1—W^1—B—$ respectively, and the benzene nuclei must not be substituted in one of the ortho-positions relative to the amino group —NH— indicated) in an acid medium and, preferably, in the presence of an oxidizing agent to give the triphendioxazine, any hydroxyalkyl groups which may be present being esterified, if appropriate before or at the same time as the cyclization or not until after the cyclization reaction, by means of a sulfating or phosphating agent to give the corresponding β-sulfatoalkyl or β-phosphatoalkyl groups, respectively, and, if appropriate, sulfo groups being introduced into aryl radicals at the same time.

14. The use of a compound of the general formula (1) which is mentioned and defined in claim 1, for dyeing (including printing) material containing hydroxyl and/or carboxamide groups, in particular fiber material.

15. A process for dyeing (including printing) material containing hydroxyl and/or carboxamide groups, in particular fiber material, in which a dyestuff is applied to the material or is introduced into the material and is fixed by means of heat and/or by means of an acid-binding agent, which comprises employing, as the dyestuff, a compound of the general formula (1) which is mentioned and defined in claim 1.

16. A compound of the general formula (4)

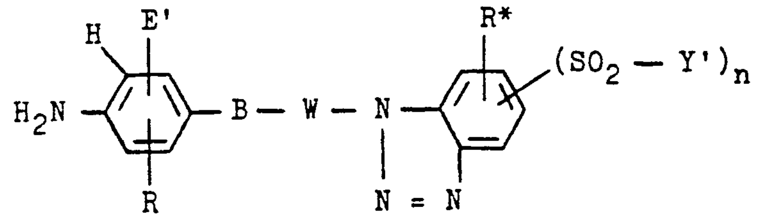

(4)

in which Y' denotes the vinyl group, the β-hydroxyethyl group or an ethyl group containing, in the β-position, a substituent which can be eliminated by means of an alkali, R, B, R* and n have the meanings mentioned in claim 1, W is a divalent, optionally substituted aliphatic radical, ($C_5$—$C_{10}$)-cycloaliphatic radical which is optionally substituted by alkyl, aliphatic-($C_5$—$C_8$)-cycloaliphatic radical which is optionally substituted by alkyl, optionally substituted araliphatic radical or optionally substituted aromatic-carbocyclic radical, it being possible for the aliphatic radicals in W to be interrupted by hetero groups, preferably 1 or 2 hetero groups, selected from the groups comprising —O—, —S—, —$SO_2$—, —CO—, 1,4-piperidino, —NH— and —N($R^c$)—, wherein $R^c$ is an alkyl group and has 1 to 6 carbon atoms and can be substituted or is an alkanoyl group having 2 to 5 carbon atoms, and/or for aliphatic and aryl radicals to be attached to one another through such a hetero group, and E' is a hydrogen atom, a sulfo or carboxyl group or a group of the formula —$SO_2$—Y' defined above or an optionally substituted sulfonamide group, it being preferable for the group E' to be attached in the ortho-position relative to the radical B.

17. A compound of the general formula (8)

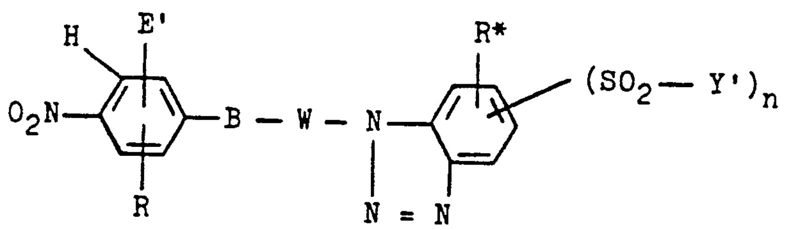

(8)

in which Y' denotes the vinyl group, the β-hydroxyethyl group or an ethyl group containing, in the β-position, a substituent which can be eliminated by means of an alkali, R, B, R* and n have the meanings mentioned in claim 1, W is a divalent, optionally substituted aliphatic radical, ($C_5$—$C_{10}$)-cycloaliphatic radical which is optionally substituted by alkyl, aliphatic-($C_5$—$C_8$)-cycloaliphatic radical which is optionally substituted by alkyl, optionally substituted araliphatic radical or optionally substituted aromatic-carbocyclic radical, it being possible for the aliphatic radicals in W to be interrupted by hetero groups, preferably 1 or 2 hetero groups, selected from the groups comprising —O—, —S—, —$SO_2$—, —CO—, 1,4-piperidino, —NH— and —N($R^o$)—, wherein $R^o$ is an alkyl group and has 1 to 6 carbon atoms and can be substituted or is an alkanoyl group having 2 to 5 carbon atoms, and/or for aliphatic and aryl radicals to be attached to one another through such a hetero group, and E' is a hydrogen atom, a sulfo or carboxyl group or a group of the formula —$SO_2$—Y' defined above or an optionally substituted sulfonamide group, it being preferable for the group E' to be attached in the ortho-position relative to the radical B.

18. A process for the preparation of a compound, indicated and defined in claim 16, of the general formula (4) or of a compound, indicated and defined in claim 17, of the general formula (8), which comprises reacting a compound of the general formula (6)

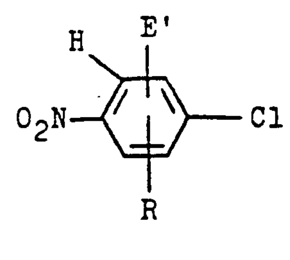

(6)

in which R and E' have the meanings indicated in claim 16, with an amino compound of the general formula (7)

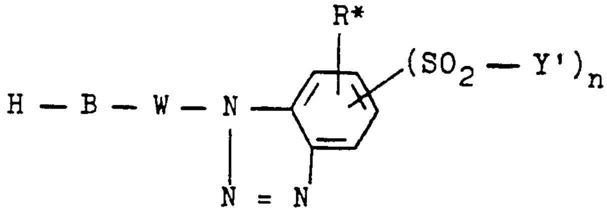

(7)

in which B, R*, W, Y' and n have the meanings indicated in claim 16, at a temperature between 70 and 120°C and with the addition of a basic, acid-binding agent, and, if appropriate, reducing the nitro group in the nitro compound of the general formula (8) thus obtained to give the amino group.

19. The use of a compound of general formula (4) from claim 16 or a compound of the general formula (8) from claim 17 for the synthesis of dyestuffs, in particular triphendioxazine compounds of the general formula (1) as claimed in claim 1.

**Claims for the Contracting State: ES**

1. A process for the preparation of a water-soluble triphendioxazine compound corresponding to the general formula (1)

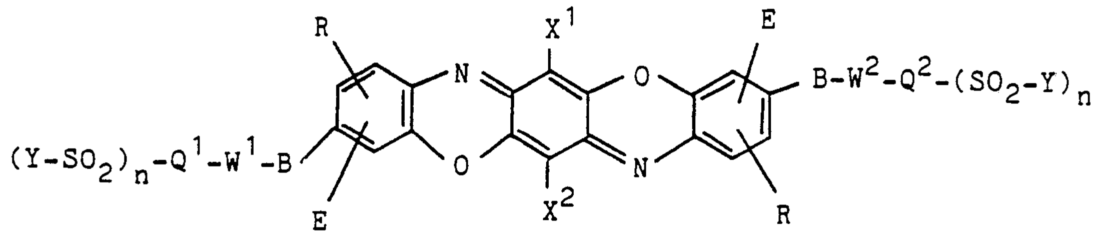

(1)

in which:

n is the number 0 or 1, the group $—SO_2—Y$ representing a hydrogen atom if n = 0, and n only being zero if E is a group $—SO_2—Y$ in which Y is β-sulfatoethyl;

Y is the vinyl group or an ethyl group containing, in the β-position, a substituent which can be eliminated by means of an alkali, and is preferably a β-sulfatoethyl group;

$Q^1$ is a benzotriazole radical of the general formula (2a) indicated below and

$Q^2$ is a benzotriazole radical of the general formula (2b) indicated below

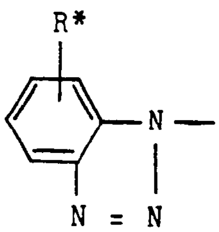

(2a)

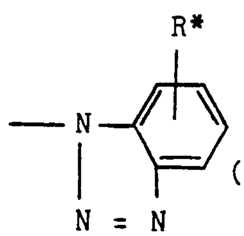

(2b)

in which

R* denotes a hydrogen atom, an alkyl group having 1 to 4 carbon atoms, an alkoxy group having 1 to 4 carbon atoms, a halogen atom or a carboxyl or sulfo group and the free bond on the benzene radical characterizes the bond leading to the grouping $(Y—SO_2)_n—$,

B is an oxygen or sulfur atom or an amino group of the formula —N(R')— in which R' is an alkyl group which has 1 to 6 carbon atoms and can be substituted, or is preferably the group —NH—;

$W^1$ is a divalent, optionally substituted aliphatic radical, $(C_5—C_{10})$-cycloaliphatic radical which is optionally substituted by alkyl, aliphatic-$(C_5—C_8)$-cycloaliphatic radical which is optionally substituted by alkyl, optionally substituted araliphatic radical or optionally substituted aromatic-carbocyclic radical, it being possible for the aliphatic radicals in $W^1$ to be interrupted by hetero groups selected from the groups —O—, —S—, $—SO_2—$, —CO—, 1,4-piperidino, —NH— and $—(R°)—$, wherein R° is an alkyl group which has 1 to 6 carbon atoms and can be substituted or is an alkanoyl group having 2 to 5 carbon atoms, and/or it being possible for aliphatic and aryl radicals to be attached to one another through such a hetero group;

$W^2$ has a meaning which is indicated for $W^1$ and is identical with $W^1$ or different from $W^1$;

R is a hydrogen atom or an alkyl group having 1 to 6 carbon atoms, an alkoxy group having 1 to 5 carbon atoms, a halogen atom or a carboxy or sulfo group, preferably a hydrogen atom;

E is a hydrogen atom or a sulfo or carboxyl group or a group of general formula $—SO_2—Y$ in which Y has the meaning indicated above or is an optionally substituted sulfonamide group;

$X^1$ is a hydrogen atom or a halogen atom, a cycloalkyl group having 5 to 8 carbon atoms, an aralkyloxy group, an alkoxy group having 1 to 4 carbon atoms, an aryloxy group, an alkyl group having 1 to 4 carbon atoms, an aryl group, an aralkyl group, a cyano group, a carboxyl group, a carboalkoxy group having 2 to 5 carbon atoms, an arylamino group, a carbamoyl group, an N-alkylcarbamoyl group or N,N-dialkylcarbamoyl group in which the alkyl radicals each have 1 to 4 carbon atoms, an N-arylcarbamoyl group, an alkanoylamino group having 2 to 5 carbon atoms or an aroylamino group, the aryl radicals in these named substituents being preferably phenyl radicals which can also be substituted by 1 or 2 substituents belonging to the group comprising halogen, nitro, alkyl having 1 to 4 carbon atoms, alkoxy having 1 to 4 carbon atoms, carboxyl and sulfo, $X^1$ preferably being a bromine or chlorine atom;

$X^2$ is identical with $X^1$ or different from $X^1$ and has one of the meanings indicated for $X^1$;

the group E is preferably attached in the ortho-position to the group $—B—W^1—Q^1—(SO_2—Y)_n$ or $—B—W^2—Q^2—(SO_2—Y)_n$; and

the molecule (1) must contain at least one of the carboxyl, sulfo and sulfato groups which can be present in the molecule (1),

which comprises cyclizing a compound of the general formula (3)

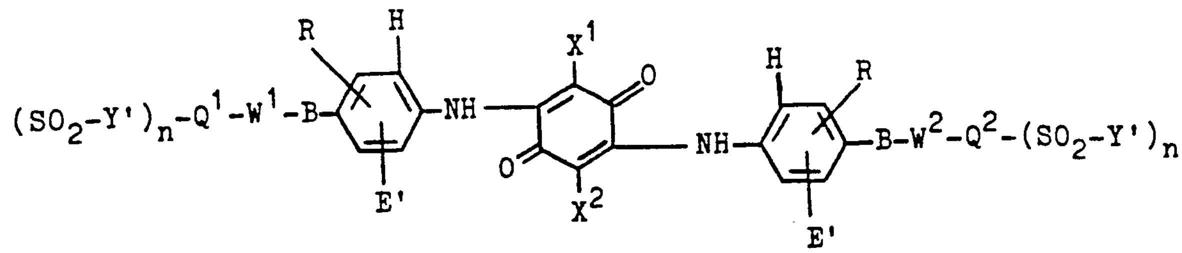

(3)

(in which Y' is the vinyl group, the β-hydroxyethyl group or an ethyl group containing, in the β-position, a substituent which can be eliminated by means of an alkali, and n, R, B, $Q^1$, $Q^2$, $W^1$, $W^2$, $X^1$ and $X^2$ have the meanings mentioned above and E' has one of the meanings mentioned for E above or is a β-hydroxyethylsulfonyl group, and substituted alkyl groups in these radicals can also be hydroxy-substituted alkyl groups, the groups E' are preferably attached in the ortho-position relative to the group $—B—W^2—Q^2—(SO_2—Y')_n$ or $(SO_2—Y')_n—Q^1—W^1—B—$ respectively, and the benzene nuclei must not be substituted in one of the ortho-positions relative to the amino group —NH— indicated) in an acid medium and, preferably, in the presence of an oxidizing agent to give the triphendioxazine, any hydroxyalkyl groups which may be present being esterified, if appropriate before or at the same time as the cyclization or not until after the cyclization reaction, by means of a sulfating or phosphating agent to give the corresponding β-sulfatoalkyl or β-phosphatoalkyl groups, respectively, and, if appropriate, sulfo groups being introduced into aryl radicals at the same time.

2. The process as claimed in claim 1 or 2, wherein E is a sulfo group.

3. A compound as claimed in claim 1 or 2, wherein $W^1$ and/or $W^2$ is a 1,3-phenylene, 1,4-phenylene or β-(p-phenylene)-ethylene group or a 2-(4'-sulfophenyl)-1,3-propylene or 2-sulfato-1,3-propylene group or a group of the formula $—CH_2—CH_2—O—CH_2—CH_2—$, $—CH_2—CH_2—NH—CH_2—CH_2—$ or $—CH_2—CH_2—N(CH_3)—CH_2—CH_2—$.

4. The process as claimed in claim 1 or 2, wherein $W^1$ denotes a group of the formula

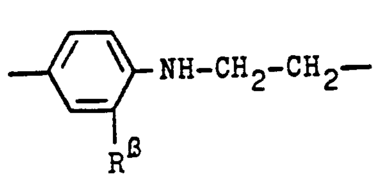

or

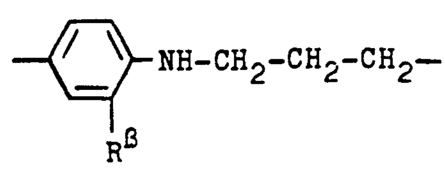

and $W^2$ denotes a group of the formula

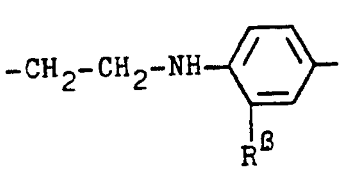

or

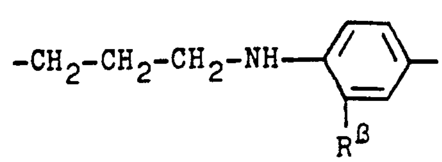

in which $R^{\beta}$ is a hydrogen atom or a sulfo group.

5. The process as claimed in claim 1 or 2, wherein $W^1$ and/or $W^2$ is a 1,2-ethylene, 1,3-propylene, 1,4-butylene or isopropylene group.

6. The process as claimed in at least one of claims 1, 2, 4 or 5, wherein E is a β-sulfatoethylsulfonyl, N,N-di(β-sulfatoethyl)-sulfamoyl, N-(β-sulfatoethyl)-sulfamoyl, N-(β-sulfoethyl)-sulfamoyl or N-methyl-N-(β-sulfoethyl)-sulfamoyl group.

7. The process as claimed in at least one of claims 1 to 6, wherein $X^1$ and $X^2$ both represent a chlorine atom.

8. The process as claimed in claim 1, wherein the resulting compound of the formula (1) is a compound corresponding to the general formula (1a)

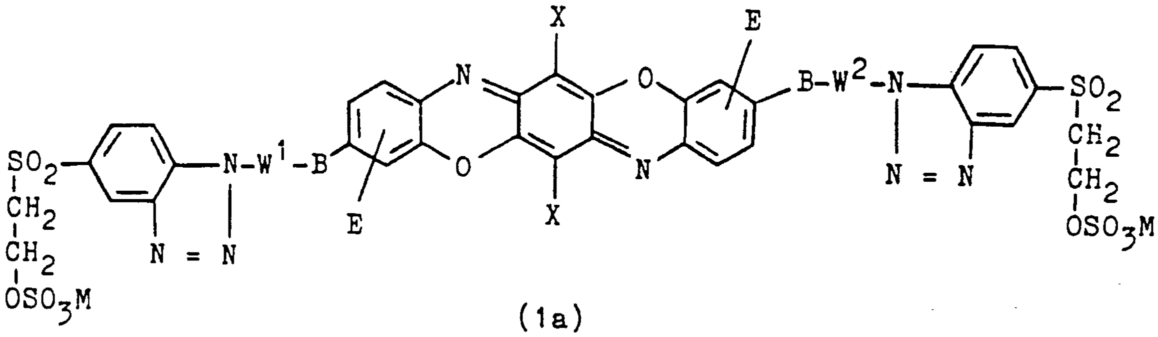

(1a)

in which

X represents a bromine or a chlorine atom,

M is a hydrogen atom or an alkali metal,

E denotes a sulfo or carboxyl group and

$W^1$ and $W^2$ both denote a 1,2-ethylene or 1,3-propylene group.

9. The process as claimed in claim 8, wherein E is a sulfo group and X is a chlorine atom.

10. The process as claimed in claim 1, wherein the resulting compound of the formula (1) is a compound corresponding to the general formula (1b) in which

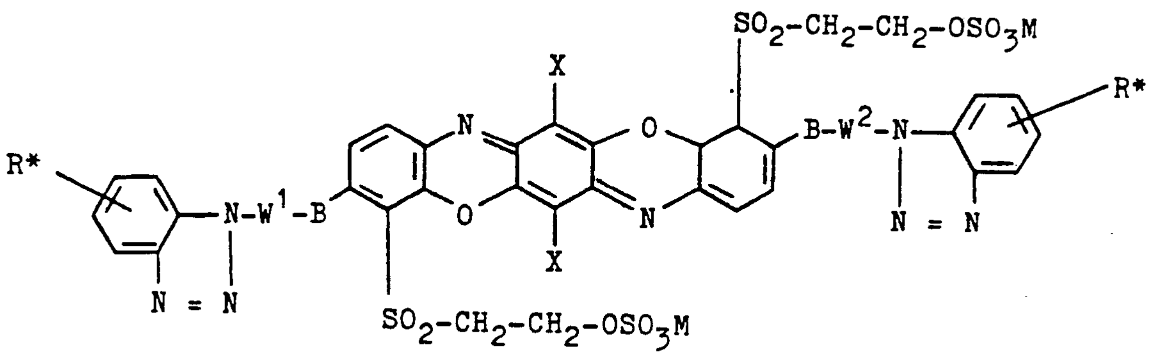

(1b)

in which

X represents a bromine atom or a chlorine atom,

M is a hydrogen atom or an alkali metal,

B denotes the group —NH—,

$W^1$ and $W^2$ both represent the 1,2-ethylene or 1,3-propylene group or an isopropylene group, or $W^1$ denotes a group of the formula

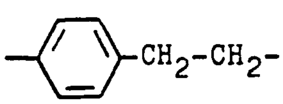

or

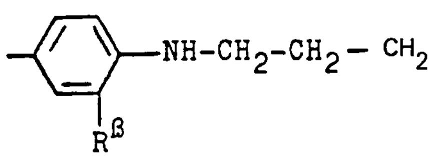

or 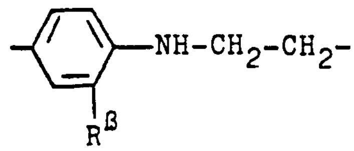

and $W^2$ denotes a group of the formula

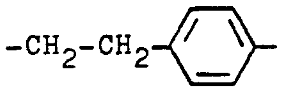

or

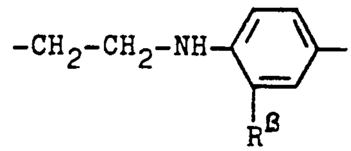

or

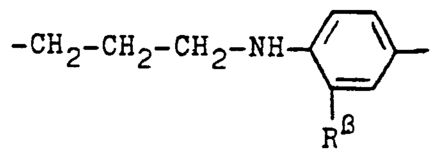

in which $R^ß$ represents a hydrogen atom or a sulfo group, and each R* denotes a sulfo group.

11. The process as claimed in claim 1, wherein the resulting compound of the formula (1) is a compound corresponding to the general formula (1c)

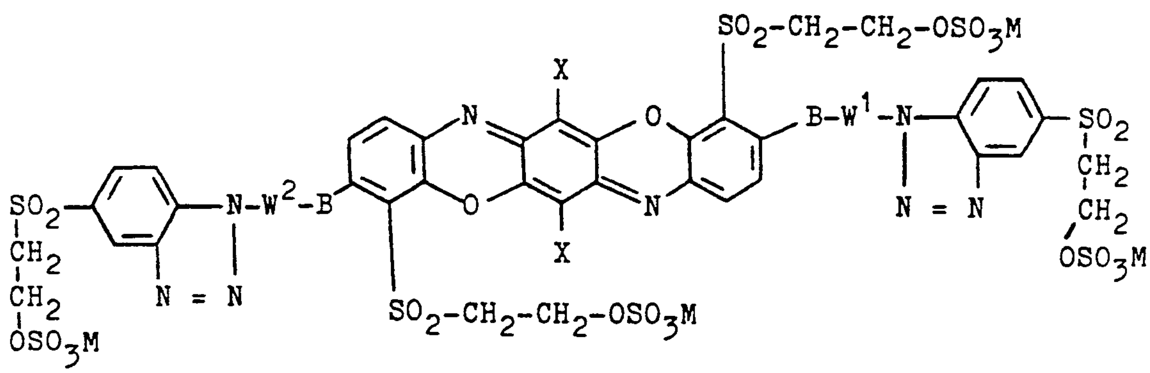

(1c)

in which

X represents a bromine atom or a chlorine atom,

M is a hydrogen atom or an alkali metal,

B denotes the group —NH— and

$W^1$ and $W^2$ both denote a divalent radical which contains sulfo groups or sulfato groups.

12. The process as claimed in claim 10 or 11 or both, wherein both of the Xs represent a chlorine atom.

13. The use of a compound of general formula (1) which is mentioned and defined in claim 1, or of a compound of the general formula (1) which has been prepared as claimed in claim 1, for dyeing (including printing) material containing hydroxyl and/or carboxamide groups, in particular fiber material.

14. A process for dyeing (including printing) material containing hydroxyl and/or carboxamide groups, in particular fiber material, in which a dyestuff is applied to the material or is introduced into the material and is fixed by means of heat and/or by means of an acid-binding agent, which comprises employing, as the dyestuff, a compound of the general formula (1) which is mentioned and defined in claim 1.

15. A process for the preparation of a compound of the general formula (4)

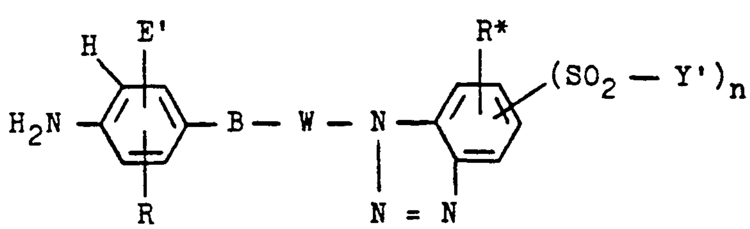 (4)

in which Y' denotes the vinyl group, the β-hydroxyethyl group or an ethyl group containing, in the β-position, a substituent which can be eliminated by means of an alkali, R, B, R* and n have the meanings mentioned in claim 1, W is a divalent, optionally substituted aliphatic radical, ($C_5$—$C_{10}$)-cycloaliphatic radical which is optionally substituted by alkyl, aliphatic-($C_5$—$C_8$)-cycloaliphatic radical which is optionally substituted by alkyl, optionally substituted araliphatic radical or optionally substituted aromatic-carbocyclic radical, it being possible for the aliphatic radicals in W to be interrupted by hetero groups, preferably 1 or 2 hetero groups, selected from the groups comprising —O—, —S—, —$SO_2$—, —CO—, 1,4-piperidino, —NH— and —N(R°)—, wherein R° is an alkyl group and has 1 to 6 carbon atoms and can be substituted or is an alkanoyl group having 2 to 5 carbon atoms, and/or for aliphatic and aryl radicals to be attached to one another through such a hetero group, and E' is a hydrogen atom, a sulfo or carboxyl group or a group of the formula —$SO_2$—Y' defined above or an optionally substituted sulfonamide group, it being preferable for the group E' to be attached in the ortho-position relative to the radical B, which comprises reacting a compound of the general formula (6)

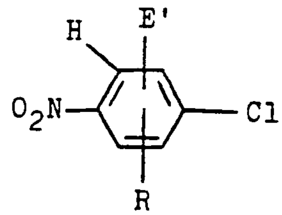

(6)

in which R and E' have the meanings indicated above, with an amino compound of the general formula (7)

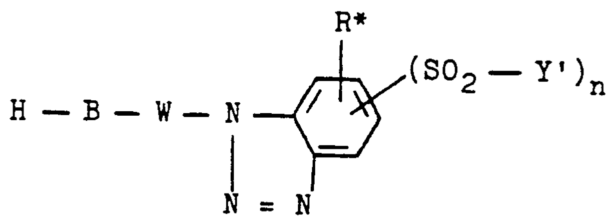

(7)

in which B, R*, W, Y' and n have the meanings mentioned above, at a temperature between 70 and 120°C and with the addition of a basic, acid-binding agent, and reducing the nitro group in the resulting nitro compound give the amino group.

16. A process for the preparation of acompound of the general formula (8)

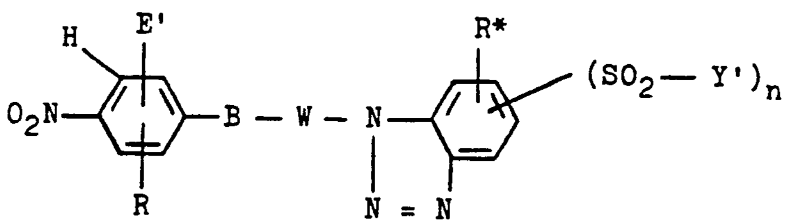

(8)

in which Y' denotes the vinyl group, the β-hydroxyethyl group or an ethyl group containing, in the β-position, a substituent which can be eliminated by means of an alkali, R, B, R* and n have the meanings mentioned in claim 1, W is a divalent, optionally substituted aliphatic radical, ($C_5$—$C_{10}$)-cycloaliphatic radical which is optionally substituted by alkyl, aliphatic-($C_5$—$C_8$)-cycloaliphatic radical which is optionally substituted by alkyl, optionally substituted araliphatic radical or optionally substituted aromatic-carbocyclic radical, it being possible for the aliphatic radicals in W to be interrupted by hetero groups, preferably 1 or 2 hetero groups, selected from the groups comprising —O—, —S—, —$SO_2$—, —CO—, 1,4-piperidino, —NH— and —N(R°)—, wherein R° is an alkyl group and has 1 to 6 carbon atoms and can be substituted or is an alkanoyl group having 2 to 5 carbon atoms, and/or for aliphatic and aryl radicals to be attached to one another through such a hetero group, and E' is a hydrogen atom, a sulfo or carboxyl group or a group of the formula —$SO_2$—Y' defined above or an optionally substituted sulfonamide group, it being preferable for the group E' to be attached in the ortho-position relative to the radical B, which comprises reacting a compound of the general formula (6)

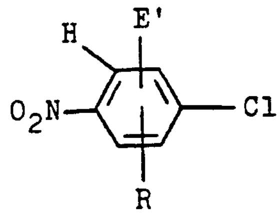

(6)

in which R and E' have the meanings indicated above, with an amino compound of the general formula (7)

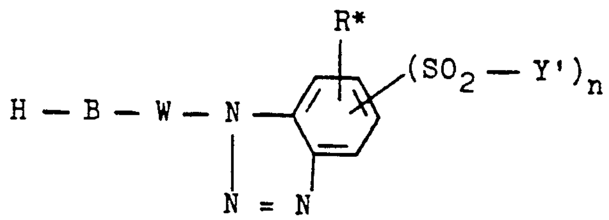

(7)

in which B, R*, W, Y' and n have the meanings mentioned above, at a temperature between 70 and 120°C and with the addition of a basic, acid-binding agent.

17. The use of a compound of the general formula (4) from claim 15 or a compound of the general formula (8) from claim 16 for the synthesis of dyestuffs, in particular triphendioxazine compounds of the general formula (1) as claimed in claim 1.